# FASCICULE DE BREVET EUROPEEN

(11) **EP 2 281 822 B1**
(45) Date de publication et mention de la délivrance du brevet: **16.01.2013**
(21) Numéro de dépôt: 10290436.4
(22) Date de dépôt: 03.08.2010
(51) Int. Cl.: C07D 401/12, C07D 401/14, C07D 403/12, C07D 403/14, C07D 413/14, C07D 417/14, A61K 31/404, A61P 35/00

(54) **Nouveaux dérivés dihydroindolones, leur procédé de préparation et les compositions pharmaceutiques qui les contiennent**
Neue Dihydroindolon-Derivate, ihr Herstellungsverfahren und die pharmazeutischen Zusammensetzungen, die sie enthalten
New dihydroindolone derivatives, method of preparing same and pharmaceutical compositions containing them

(30) Priorité: 04.08.2009 FR 0903839
(43) Date de publication de la demande: 09.02.2011
(73) Titulaire: Les Laboratoires Servier, 92284 Suresnes Cedex (FR)
(72) Inventeur: Ortuno, Jean-Claude, 78390 Bois d'Arcy (FR); Cordi, Alexis, 92150 Suresnes (FR); Lacoste, Jean-Michel, décédé (FR); Fejes, Imre, 1012 Budapest (HU); Burbridge, Michael, 78480 Verneuil sur Seine (FR); Hickman, John, 75017 Paris (FR); Pierre, Alain, 78580 Les Alluets Le Roi (FR)

(56) Documents cités:
- WO-A2-01/60814

## Description

La présente invention concerne de nouveaux dérivés dihydroindolones, leur procédé de préparation et les compositions pharmaceutiques qui les contiennent.

Les composés de la présente invention sont nouveaux et présentent des caractéristiques pharmacologiques très intéressantes dans le domaine de la cancérologie.

Les besoins de la thérapeutique anticancéreuse exigent le développement constant de nouveaux agents antitumoraux, dans le but d'obtenir à la fois des médicaments plus actifs et mieux tolérés.

Un des problèmes majeurs en cancérologie provient de la faculté qu'ont les cellules cancéreuses à migrer depuis la tumeur primaire formée vers d'autres cibles. La migration cellulaire est un processus physiologique crucial pour le développement embryonnaire et le maintien de l'homéostasie tissulaire. La migration fait intervenir des changements morphologiques importants, et implique la répression de voies de signalisation intracellulaire.

Des dérégulations de la migration cellulaire sont fortement impliquées dans la pathologie cancéreuse, et plus particulièrement dans le processus de formation des métastases (Hanahan D. et al., 2000, Cell, 100, 57-70).

Il est ainsi particulièrement important de pouvoir contrôler ce processus qui conduit de façon inéluctable au cancer généralisé, et à la mort certaine du patient.

L'art antérieur WO01/60814 décrit des dérivés dihydroindolones utiles en tant qu'inhibiteurs de kinases et leur utilisation dans des pathologies impliquant ces kinases, comme le cancer par exemple.

Les composés de la présente invention, outre le fait qu'ils soient nouveaux, présentent une activité *in vitro* surprenante d'inhibition de la migration des cellules cancéreuses, et donc de la progression tumorale.

Ainsi, les composés de la présente invention possèdent des propriétés qui les rendent particulièrement utiles pour le traitement des cancers et notamment des tumeurs solides métastatiques.

La présente invention concerne plus particulièrement les composés de formule (I) : 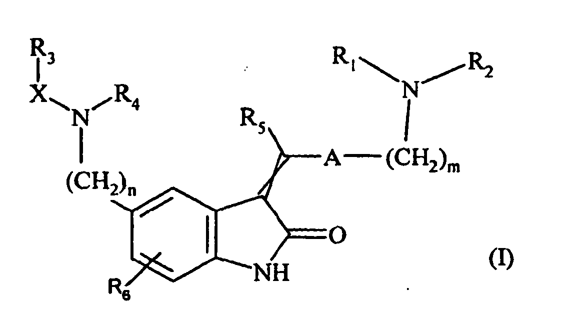 dans laquelle :
◆ m représente 1 ou 2,
◆ n représente 1 ou 2,
◆ A représente un groupement pyrrolyle non substitué ou substitué par 1 à 3 groupements alkyle (C₁-C₆) linéaire ou ramifié,
◆ X représente le groupement C(O), S(O) ou SO₂
◆ R₁ et R₂, identiques ou différents, représentent chacun un groupement alkyle (C₁-C₆) linéaire ou ramifié,
   ou R₁ et R₂ forment ensemble avec l'atome d'azote qui les porte un groupement hétérocyclique,
◆ R₃ et R₄ forment ensemble avec les atomes qui les portent un groupement hétérocyclique,
◆ R₅ représente un atome d'hydrogène ou un groupement alkyle (C₁-C₆) linéaire ou ramifié,
◆ R₆ représente un atome d'hydrogène ou un atome d'halogène,
   étant entendu que :
   - par "groupement hétérocyclique", on entend un groupement mono ou bicyclique pouvant contenir de 5 à 8 sommets, pouvant contenir de un à trois hétéroatomes choisis parmi azote, oxygène et soufre, et pouvant contenir une ou plusieurs insaturations, le groupement hétérocyclique ainsi défini pouvant être non substitué ou substitué par un ou plusieurs groupements choisis parmi alkyle (C₁-C₆) linéaire ou ramifié, alkényle (C₁-C₆) linéaire ou ramifié, oxo, hydroxy, alkoxy (C₁-C₆) linéaire ou ramifié, aryle, arylalkyle, et arylalkényle,

   - par "aryle", on entend le groupement phényle non substitué ou substitué par un ou plusieurs groupements choisis parmi les atomes d'halogène et le groupement alkyle (C₁-C₆) linéaire ou ramifié,
   - la notation × signifie que la double liaison est de configuration Z ou E,
   leurs isomères optiques et géométriques, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

La valeur préférée pour n et m est 1.

R₁ et R₂ représentent avantageusement un groupement alkyle comme par exemple le groupement éthyle, ou forment ensemble avec l'atome d'azote qui les porte un groupement monocyclique à 5 ou 6 chaînons comme par exemple les groupements pipéridinyle, morpholinyle, pyrrolidinyle, pipérazinyle, ou méthylpipérazinyle, ou un groupement bicyclique comportant de 6 à 8 chaînons comme par exemple les groupements octahydrocyclopentapyrrolyle ou azabicyclo[3.1.0]hexanyle. De façon préférée, R₁ et R₂ forment ensemble avec l'atome d'azote qui les porte le groupement morpholinyle.

Avantageusement, R₃ et R₄ forment ensemble avec le groupement X et l'atome d'azote qui les portent un groupement monocyclique à 5 ou 6 chaînons comme par exemple les groupements pyrrolidinonyle, pyrrolidinedionyle, oxazolidinonyle, oxazolidinedionyle, diméthyloxazolidinedionyle, thiazolidinonyle, thiazolidinedionyle, diméthylthiazolidinedionyle, imidazolidinonyle, imidazolidinedionyle, thiadiazinonyle ou dihydrothiadiazinonyle, ou un groupement bicyclique à 6 chaînons comme par exemple les groupements azabicyclo[3.1.0]hexanonyle ou azabicyclo[3.1.0]hexanedionyle. De façon préférée R₃ et R₄ forment ensemble avec le groupement X et l'atome d'azote qui les portent les groupements 2,4-dioxo-1,3-thiazolidin-3-yle, 2,4-dioxo-1,3-oxazolidin-3-yle, 2-oxo-1,3-thiazolidin-3-yle, 2-oxo-1,3-oxazolidin-3-yle, 4-hydroxy-2-oxo-1,3-thiazolidin-3-yle, et 2,4-dioxo-3-azabicyclo[3.1.0]hex-3-yle.

R₅ représente avantageusement un atome d'hydrogène ou les groupements méthyle et éthyle.

X représente préférentiellement le groupement C(O).

Préférentiellement, A est le groupement pyrrolyle non substitué et plus particulièrement le groupement 1*H*-pyrrol-2,4-yle.

L'invention concerne de façon préférée les composés de formule (I) pour lesquels R₁ et R₂ forment ensemble avec l'atome d'azote qui les porte le groupement morpholinyle, m et n représentent la valeur 1, R₅ et R₆ représentent un atome d'hydrogène et A représente le groupement 1*H*-pyrrol-2,4-yle.

La double liaison exocyclique des composés de formule (I) est de configuration Z ou E, et plus préférentiellement de configuration Z.

Encore plus particulièrement, l'invention concerne les composés de formule (I) qui sont:
● le 3-[(3- {[4-(4-morpholinylméthyl)-1*H*-pyrrol-2-yl]méthylène}-2-oxo-2,3-dihydro-1*H*-indol-5-yl)méthyl]-1,3-thiazolidine-2,4-dione,
● le 3-[3-(4-morpholin-4-ylméthyl-1*H*-pyrrol-2-ylméthylène)-2-oxo-2,3-dihydro-1*H-*indol-5-ylméthyl]-oxazolidine-2,4-dione,
● le 3-(4-morpholin-4-ylméthyl-1*H*-pyrrol-2-ylméthylène)-5-(2-oxo-oxazolidin-3-yl méthyl)-1,3-dihydro-indol-2-one,

● le 5-(4-hydroxy-2-oxo-thiazolidin-3-ylméthyl)-3-(4-morpholin-4-ylméthyl-1*H* pyrrol-2-ylméthylène)-1,3-dihydro-indol-2-one,
● le (1R,5S)-3-[3-(4-morpholin-4-ylméthyl-1*H*-pyrrol-2-ylméthylène)-2-oxo-2,3-dihydro-1*H*-indol-5-ylméthyl]-3-aza-bicyclo[3.1.0]hexane-2,4-dione,
● le 3-(4-morpholin-4-ylméthyl-1*H*-pyrrol-2-ylméthylène)-5-(2-oxo-thiazolidin-3-ylméthyl)-1,3-dihydro-indol-2-one,
● le 3-{3-[1-(4-morpholin-4-ylméthyl-1*H*-pyrrol-2-yl)-propylidène]-2-oxo-2,3-dihydro-1*H*-indol-5-ylméthyl}-thiazolidine-2,4-dione,
● le 3-{3-[1-(4-morpholin-4-ylméthyl-1*H*-pyrrol-2-yl)-éthylidène]-2-oxo-2,3-dihydro-1*H*-indol-5-ylméthyl}-thiazolidine-2,4-dione.

Les isomères optiques et géométriques, ainsi que les sels d'addition à un acide ou à une base pharmaceutiquement acceptable des composés préférés de l'invention font partie intégrante de l'invention.

L'invention s'étend également au procédé de préparation des composés de formule (I) caractérisé en ce que l'on utilise comme produit de départ le composé de formule (II): 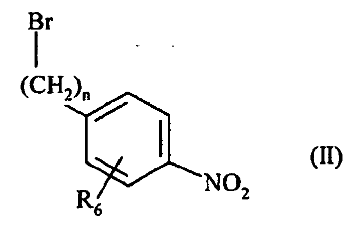 dans laquelle n et R₆ sont tels que défini dans la formule (I),
sur lequel on condense, en présence d'une base, le composé de formule (III) : 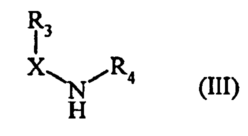 dans laquelle X, R₃ et R₄ sont tels que définis dans la formule (I), pour conduire au composé de formule (IV) : 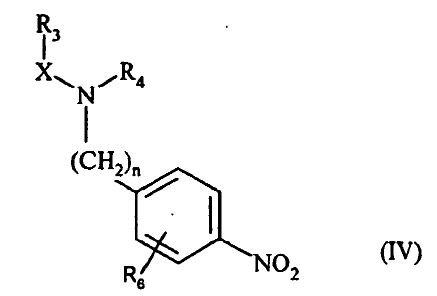 dans laquelle n, X, R₃, R₄ et R₆ sont tels que définis précédemment,
que l'on soumet à une hydrogénation chimique ou catalytique, pour conduire au composé de formule (V) : 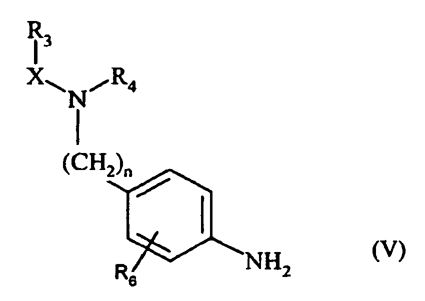 dans laquelle n, X, R₃, R₄ et R₆ sont tels que définis précédemment,
que l'on soumet à l'action de tBuOCl en présence de (méthylsulfanyl)acétate d'éthyle, suivi de l'action successive de triéthylamine et d'acide chlorhydrique pour conduire au composé de formule (VI) : 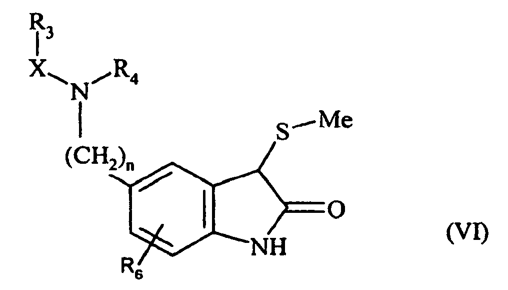 dans laquelle n, X, R₃, R₄ et R₆ sont tels que définis précédemment,
que l'on soumet à l'action de Zinc en poudre pour conduire au composé de formule (VII) : 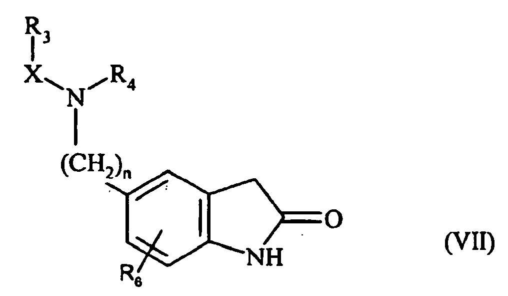 dans laquelle n, X, R₃, R₄ et R₆ sont tels que définis précédemment,
sur lequel on condense en présence de pipéridine le composé de formule (VIII) : 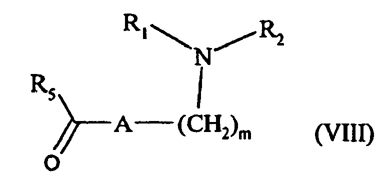 dans laquelle m, A, R₁, R₂, et R₅ sont tels que définis dans la formule (I),
pour conduire après traitement acide au composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

Les composés de formules (II), (III) et (VIII) sont soit commerciaux, soit accessibles à l'homme du métier par des réactions chimiques classiques et décrites dans la littérature.

Une variante avantageuse concerne le procédé de préparation des composés de formule (I) caractérisé en ce que l'on utilise comme produit de départ le composé de formule (IX): 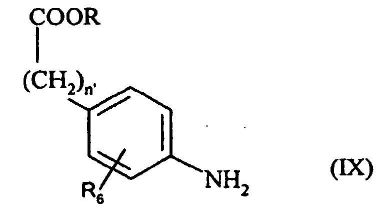 dans laquelle n' représente 0 ou 1, et R représente un groupement alkyle (C₁-C₆)linéaire ou ramifié, et R₆ est tel que défini dans la formule (I),
que l'on soumet à l'action de tBuOCl en présence de (méthylsulfanyl)acétate d'éthyle, suivi de l'action successive de triéthylamine et d'acide chlorhydrique, pour conduire au composé de formule (X): 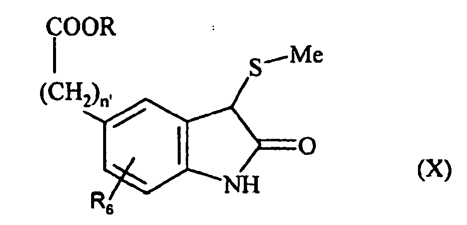 dans laquelle n', R et R₆ sont tels que définis précédemment,
que l'on soumet à l'action de Zinc en poudre pour conduire au composé de formule (XI): 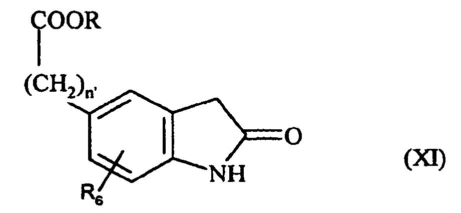 dans laquelle n', R et R₆ sont tels que définis précédemment,
que l'on place dans un milieu réducteur pour conduire au composé de formule (XII): 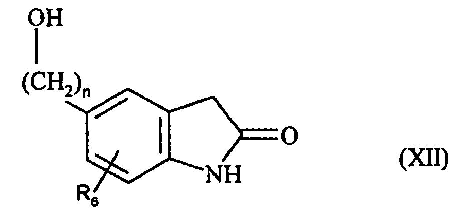 dans laquelle n et R₆ sont tels que définis précédemment,
sur lequel on condense en présence de pipéridine le composé de formule (VIII) : 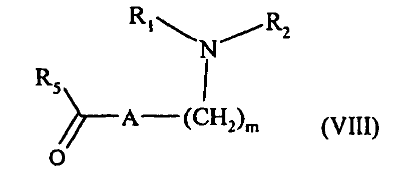 dans laquelle m, A, R₁, R₂, et R₅ sont tels que définis dans la formule (I), pour conduire après traitement acide au composé de formule (XIII) : 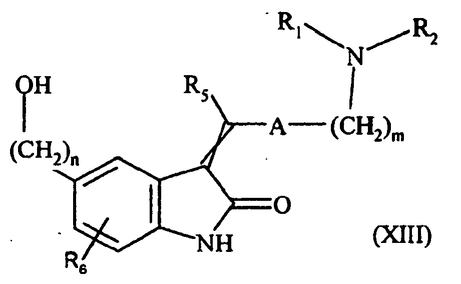 dans laquelle m, n, A, R₁, R₂, R₅ et R₆ sont tels que définis précédemment,
sur lequel on condense directement en présence de triphénylphosphine et d'azodicarboxylate d'éthyle par exemple le composé de formule (III) : 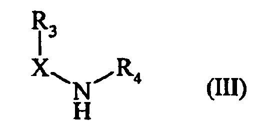 dans laquelle X, R₃ et R₄ sont tels que définis dans la formule (I),
pour conduire au composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

Les composés de formule (III), (VIII) et (IX) sont soit commerciaux, soit accessibles à l'homme du métier par des réactions chimiques classiques et décrites dans la littérature.

L'étude pharmacologique des dérivés de l'invention a montré qu'ils possédaient la capacité d'inhiber la migration des cellules cancéreuses. Cette propriété présente donc un intérêt thérapeutique majeur dans le traitement des cancers, plus particulièrement dans le traitement des cancers métastatiques.

Parmi les traitements des cancers envisagés on peut citer, sans s'y limiter, les cancers du colon, du sein, du foie, des reins, du cerveau, de l'oesophage, les mélanomes, les myélomes, les cancers de l'ovaire, les cancers du poumon non à petites cellules, les cancers du poumon à petites cellules, les cancers de la prostate et du pancréas, les sarcomes.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Par ailleurs, la présente invention concerne également l'association d'un composé de formule (I) avec un agent anticancéreux choisi parmi les agents génotoxiques, les poisons mitotiques, les anti-métabolites, les inhibiteurs du protéasome, ou les inhibiteurs de kinase, ainsi que l'utilisation de ce type d'association pour la fabrication de médicaments utiles dans le traitement du cancer.

Les composés de l'invention peuvent également être utilisés en association avec une radiothérapie dans le traitement du cancer.

Les Préparations et Exemples suivants illustrent l'invention et ne la limitent en aucune façon.

Les points de fusion sont mesurés dans un appareil Capillaire.

### Préparation 1 : 4-Morpholin-4-ylméthyl-1H-pyrrole-2-carbaldéhyde

A une solution de morpholine (0,38 mole) dans l'acide acétique (120 ml) à 0°C est ajoutée une solution de 1*H*-pyrrole-2-carbaldéhyde (0,32 mole) dans l'acide acétique (100 ml). Puis du formaldéhyde (37% aq., 26 ml) est ajouté goutte à goutte. La réaction est agitée pendant 65 heures à température ambiante. Le milieu est concentré (environ 50 ml) et refroidi dans un bain de glace. Le pH de la solution est alcalinisé (pH=12) avec une solution aqueuse de soude à 20%. Le produit est extrait avec du dichlorométhane (DCM) (3x250 ml). Les phases organiques sont combinées. La phase organique obtenue est lavée avec une solution aqueuse saturée de NaCl, séchée sur sulfate de sodium, filtrée et évaporée à sec. Le résidu obtenu (huile) est purifié sur gel de silice (SiO₂, gradient DCM / MeOH) pour conduire au produit du titre.
*Spectrométrie de masse* (ES +, m/z) : 195,1124 (M+H)⁺

### Préparation 2 : 1-(4-Morpholin-4-ylméthyl-1H-pyrrol-2-yl)-éthanone

A une solution de morpholine (22 mmoles) dans l'acide acétique (60 ml) à 0°C sont ajoutés de la 1-(1*H*-pyrrol-2-yl)-éthanone (20 mmoles) et du formaldéhyde (20 mmoles) à 37% dans l'eau. Le milieu réactionnel est agité une nuit à température ambiante. A 0°C la solution est amenée à pH alcalin (10-12) avec une solution aqueuse à 20% de NaOH puis extraite au DCM. La phase organique est lavée à l'eau, avec une solution aqueuse saturée de NaCl, séchée sur sulfate de sodium, filtrée et évaporée à sec. Purification par flash chromatographie sur gel de silice (SiO₂; Gradient DCM / MeOH) pour conduire au produit du titre.
*Spectrométrie de masse* (ES +, m/z): 209,1289 (M+H)⁺

### Préparation 3 : 4-[(1R,5S)-3-Aza-bicyclo[3.1.0]hex-3-ylméthyl]-3,5-diméthyl-1H pyrrole-2-carbaldéhyde

A une solution de (1R,5S)-3-aza-bicyclo[3.1.0]hexane (10 mmoles) dans l'acide acétique (10 ml) sont ajoutés 10 mmoles de 3,5-diméthyl-1H-pyrrole-2-carbaldéhyde et du formaldéhyde (11 mmoles) à 37% dans l'eau. Le milieu réactionnel est agité une nuit à température ambiante puis évaporé à sec. Purification par flash chromatographie sur gel de silice (SiO₂; heptane / AcOEt) puis trituration dans l'heptane pour conduire au produit du titre.
*Spectrométrie de masse* (ES +, m/z) : 219,1503 (M+H)⁺

### Préparation 4 : 4-((1R,5S)-3-Aza-bicyclo[3.1.0]hex-3-ylméthyl)-1H-pyrrole-2-carbaldéhyde

On procède comme dans la Préparation 1 en remplaçant la morpholine par le (1*R*,5*S*)-3-azabicyclo[3.1.0]hexane.
*Spectrométrie de masse* (ES +, m/z) : 191,1179 (M+H)⁺

### Préparation 5 : 1-(4-Morpholin-4-ylméthyl-1H-pyrrol-2-yl)-propan-1-one

A une solution de morpholine (89,3 mmoles) dans l'acide acétique (50 ml) à 0°C sont ajoutés la 1-(1*H*-pyrrol-2-yl)-propan-1-one (81,2 mmoles) et du formaldéhyde (89,3 mmoles) à 37% dans l'eau. Le milieu réactionnel est agité une nuit à température ambiante. Après concentration du milieu la solution est amenée à pH alcalin (10-12) avec une solution aqueuse à 20% de NaOH à 0°C puis extraite au DCM. La phase organique est lavée à l'eau, avec une solution aqueuse saturée de NaCl, séchée sur sulfate de sodium, filtrée et évaporée à sec et purifiée par flash chromatographie sur gel de silice (SiO₂ ; Gradient AcOEt 100% à AcOEt/MeOH 9/1) pour conduire au produit du titre.
*Spectrométrie de masse* (ES +, m/z) : 223,1444 (M+H)⁺

### Préparation 6 : 1-Méthyl-4-morpholin-4-ylméthyl-1H-pyrrole-2-carbaldéhyde et 1-méthyl-5-morpholin-4-ylméthyl-1H-pyrrole-2-carbaldéhyde

A une solution de morpholine (130 mmoles) dans l'acide acétique (60 ml) à 0°C sont ajoutés le 1-méthyl-1*H*-pyrrole-2-carbaldéhyde (110 mmoles) et du formaldéhyde (110 mmoles) à 37% dans l'eau. Le milieu réactionnel est agité 16 heures à 50°C. Après évaporation à sec environ 100 ml d'une solution aqeuse à 25% de soude est ajoutée. Après extraction au DCM la phase organique est lavée à l'eau, avec une solution aqueuse saturée de NaCl, séchée sur sulfate de sodium, filtrée et évaporée à sec. Le résidu est purifié par flash chromatographie sur gel de silice (SiO_{2;} Gradient AcOEt / MeOH) pour conduire aux produits du titre le 1-méthyl-4-morpholin-4-ylméthyl-1*H*-pyrrole-2-carbaldéhyde et le 1-méthyl-5-morpholin-4-ylméthyl-1*H*-pyrrole-2-carbaldéhyde dans un rapport de 1/5.
**1-Méthyl-4-morpholin-4-ylméthyl-1*H*-pyrrole- 2-carbaldéhyde**
*Spectrométrie de masse* (ES +, m/z) : 209,1282 (M+H)⁺
**1-Méthyl-5-morpholin-4-ylméthyl-1*H*-pyrrole-2-carbaldéhyde**
*Spectrométrie de masse* (ES +, m/z) : 209,1286 (M+H)⁺

### Préparation 7 : 5-Morpholin-4-ylméthyl-1H-pyrrole-2-carbaldéhyde

### Stade A : 4-(1H-Pyrrol-2-ylméthyl)-morpholine

A une solution de morpholine (22 mmoles) dans l'acide acétique (10 ml) est ajouté du pyrrole (20 mmoles) goutte à goutte à 0°C. Puis du formaldéhyde (37% aq. 20 mmoles) est ajouté goutte à goutte. La réaction est agitée pendant 2 heures à température ambiante. La solution est refroidie à 0°C et est amenée à pH alcalin (10-11) avec une solution aqueuse de NaOH à 20%. Après extraction au DCM les phases organiques sont lavées à l'eau, avec une solution aqueuse saturée en NaCl, séchées sur sulfate de sodium, filtrées et évaporées à sec pour conduire au produit du titre sous la forme d'un solide blanc qui est engagé directement dans l'étape suivante.

### Stade B : 5-Morpholin-4-ylméthyl-1H-pyrrole-2-carbaldéhyde

A une solution de DMF (18,04 mmoles) dans le DCM (10 ml) est ajouté POCl₃ goutte à goutte à 0°C. Après 5 minutes d'agitation une solution du composé obtenu dans le Stade A (9,02 mmoles) est ajoutée goutte à goutte. Après 3 heures d'agitation à 0°C le milieu réactionnel est porté à 40°C pendant 30 minutes. A température ambiante est ajoutée une solution aqueuse d'acétate de potassium (32 mmoles) puis le milieu réactionnel est agité à 40°C pendant 30 minutes. A température ambiante la solution est amenée à pH alcalin avec une solution de NaOH 2M puis extraite au DCM. La phase organique est lavée à l'eau, avec une solution de NaCl saturée, séchées sur sulfate de sodium, filtrée et évaporée à sec. Le résidu obtenu est purifié sur gel de silice (SiO₂; Gradient DCM / AcOEt) pour conduire au produit du titre.
*Spectrométrie de masse* (ES +, m/z) : 195,1150 (M+H)⁺

### Préparation 8 : 3-Morpholin-4-ylméthyl-1H-pyrrole-2-carbaldéhyde

### Stade A : 4-(1-Triisopropylsilanyl-1H-pyrrol-3-ylméthyl)-morpholine

A une solution de morpholine (98,5 mmoles) dans l'acide acétique (100ml) à 0°C sont ajoutés du 1-triisopropylsilanyl-1*H*-pyrrole (89,5 mmoles) et du formaldéhyde (89,5 mmoles) à 37% dans l'eau. Le milieu réactionnel est agité pendant une nuit à température ambiante. La solution est amenée à pH alcalin (10-12) avec une solution aqueuse à 20% de NaOH puis extraite au DCM. La phase organique est lavée à l'eau, avec une solution aqueuse saturée en NaCl, séchées sur sulfate de sodium, filtrée et évaporée à sec. Le résidu obtenu est purifié par flash chromatographie sur gel de silice (SiO₂ ; Gradient DCM / AcOEt) pour conduire au produit du titre qui est directement engagé dans l'étape suivante.

### Stade B : 4-(1H-Pyrrol-3-ylméthyl)-morpholine

A une solution du composé obtenu dans le Stade A (75 mmoles) dans le THF (100 ml) est ajouté goutte à goutte à 0°C du Bu₄NF 1,0 M dans le THF (75,0 mmole). Après agitation à 0°C pendant une heure la solution est jetée dans la glace puis extraite au DCM. Les phases organiques sont combinées puis lavées avec une solution aqueuse saturée de NaCl, séchées sur sulfate de sodium filtrées et évaporées à sec pour conduire au produit du titre qui est directement engagé dans l'étape suivante.

### Stade C: 3-Morpholin-4-ylméthyl-1H-pyrrole-2-carbaldéhyde

A une solution de DMF (66 mmoles) dans le DCM est ajouté goutte à goutte du POCl₃ à 0°C. Après 5 minutes d'agitation, une solution du composé obtenu dans le Stade B (33 mmoles) dans le DCM (60 ml) est ajoutée goutte à goutte. Après 4 heures d'agitation à 0°C et 30 minutes au reflux une solution aqueuse d'acétate de sodium (110 mmoles) est ajoutée. Après 30 minutes supplémentaires au reflux une solution aqueuse 2 M de soude est ajoutée à température ambiante jusqu'à pH alcalin (9-10). La solution est agitée 2 heures à température ambiante puis 300 ml de DCM sont ajoutés. Après extraction au DCM la phase organique est lavée à l'eau, avec une solution aqueuse saturée de NaCl, séchée sur sulfate de sodium, filtrée et évaporée à sec. Le résidu obtenu est purifié par flash chromatographie sur gel de silice (SiO₂; Gradient DCM / AcOEt) pour conduire au produit du titre.
*Spectrométrie de masse* (ES +, m/z) : 195,1134 (M+H)⁺

### Préparation 9 : 5-Diéthylaminométhyl-1H-pyrrole-2-carbaldéhyde

On procède comme dans la Préparation 7 en remplaçant la morpholine par la diéthylamine.
*Spectrométrie de masse* (ES +, m/z) : 181,1340 (M+H)⁺

### Préparation 10 : 4-(4-Méthyt-pipérazin-1-ylméthyt)-1H-pyrrole-2-carbaldéhyde

On procède comme dans la Préparation 1 en remplaçant la morpholine par la 1-méthyl-pipérazine.
*Spectrométrie de masse* (ES +, m/z) : 208,1436 (M+H)⁺

### Préparation 11 : 4-Pyrrolidin-1-ylméthyl-1H-pyrrole-2-carbaldéhyde

On procède comme dans la Préparation 1 en remplaçant la morpholine par la pyrrolidine.
*Spectrométrie de masse* (ES +, m/z) : 179,1183 (M+H)⁺

### Préparation 12 : 4-((3aR,6aS)-Hexahydro-cyclopenta[c]pyrrol-2-(1H)-yl méthyl)-1H-pyrrole-2-carbaldéhyde

On procède comme dans la Préparation 1 en remplaçant la morpholine par le (3aR,6aS)-hexahydro-cyclopenta[c]pyrrole.
*Spectrométrie de masse* (ES +, m/z) : 219,1481 (M+H)⁺

### Préparation 13 : 4-Pipéridin-1-ylméthyl-1H-pyrrole-2-carbaldéhyde

On procède comme dans la Préparation 1 en remplaçant la morpholine par le (3aR,6aS)-hexahydro-cyclopenta[c]pyrrole.
*Spectrométrie de masse* (ES +, m/z) : 193,1349 (M+H)⁺

### Préparation 14 : 4-Diéthylaminométhyl-1H-pyrrole-2-carbaldéhyde

On procède comme dans la Préparation 1 en remplaçant la morpholine par la diéthyl-amine.
*Spectrométrie de masse* (ES +, m/z) : 181,1342 (M+H)⁺

### Préparation 15 : 4-((3aR,6aS)-hexahydro-cyclopenta[c]pyrrol-2(1H)-ylméthyl)-3,5-diméthyl-1H-pyrrole-2-carbaldéhyde

On procède comme dans la Préparation 3 en remplaçant la (1*R*,5*S*)-3-azabicyclo[3.1.0]hexane par le (3aR,6aS)-hexahydro-cyclopenta[c]pyrrole.
*Spectrométrie de masse* (ES +, m/z) : 247,1785 (M+H)⁺

### Préparation 16 : 5-morpholin-4-ylméthyl-1H-pyrrole-3-carbaldéhyde

On procède comme dans la Préparation 1 en remplaçant le 1*H*-pyrrole-2-carbaldéhyde par le 1*H*-pyrrole-3-carbaldéhyde.
*Spectrométrie de masse* (ES +, m/z) : 195,1133 (M+H)⁺

### Préparation 17 : 5-((1R,5S)-3-aza-bicyclo[3.1.0]hex-3-ylméthyl)-1H-pyrrole-3-carbaldéhyde

On procède comme dans la Préparation 1 en remplaçant le 1*H*-pyrrole-2-carbaldéhyde par le 1*H*-pyrrole-3-carbaldéhyde et en remplaçant la morpholine par la (1*R*,5*S*)*-*3-azabicyclo[3.1.0]hexane.
*Spectrométrie de masse* (ES +, m/z) : 191,1175 (M+H)⁺

### Préparation 18: 4-[2-(Pyrrolidin-1-yl)éthyl]-1H-pyrrole-2-carbaldéhyde

### Stade A: 2,2,2-Trichloro-1-(1H-pyrrol-2-yl)éthanone

A une solution de chlorure de trichloroacétyl (160,7 mmoles) dans l'éther éthylique (26 ml) est ajouté une solution de pyrrole (149 mmoles) dans l'éther éthylique (83 ml) goutte à goutte sur une période de 3 heures. Après une heure d'agitation supplémentaire une solution de carbonate de potassium (94 mmoles) dans l'eau (39 ml) est ajoutée goutte à goutte. Les phases sont séparées et la phase organique est séchée sur sulfate de sodium et filtrée. A cette phase organique, du charbon actif est ajouté. Après 5 minutes d'agitation la phase organique est filtrée et évaporée à sec pour conduire au produit du titre.

### Stade B : 5-(Trichloroacétyl)-1H-pyrrole-3-carbaldéhyde

A une solution du composé obtenu dans le Stade A (87 mmoles) et de AlCl₃ dans un mélange DCM / Nitrométhane (80 ml / 80 ml) est ajouté goutte à goutte du dichlorométhoxy-méthane (130,5 mmoles) à -30°C. Après deux heures d'agitation le milieu réactionnel est jeté dans 800 ml d'eau glacée. La phase aqueuse est extraite à l'AcOEt, puis les phases organiques sont combinées. La phase organique est lavée avec une solution aqueuse saturée de NaCl, séchée sur sulfate de sodium, filtrée et évaporée à sec pour conduire au produit du titre (cristaux bruns) qui est utilisé directement dans l'étape suivante.

### Stade C : 4-Formyl-1H-pyrrole-2-carboxylate d'éthyle

A une solution du composé obtenu dans le Stade B (83,7 mmoles) dans l'éthanol (200 ml) est ajouté goutte à goutte et en 20 minutes une solution d'éthylate de sodium (13,4 mmoles) dans l'éthanol (45 ml) à température ambiante. Après 3 heures d'agitation le milieu est évaporé à sec puis repris dans l'éther éthylique (200 ml). A cette phase organique est ajoutée une solution aqueuse de HCl 2N (150 ml) et ce mélange est agité puis les phases sont séparées et la phase aqueuse est extraite à l'éther éthylique. La phase organique est lavée avec une solution aqueuse saturée de NaHCO₃, avec une solution aqueuse saturée de NaCl, séchée sur sulfate de sodium, filtrée et évaporée à sec pour conduire au produit du titre (cristaux bruns) qui est utilisé directement dans l'étape suivante.

### Stade D : 4-[(Diméthylamino)méthyl]-1H-pyrrole-2-carboxylate d'éthyle

A une solution du composé obtenu dans le Stade C et de diméthylamine (1,1 eq.) est ajouté par portions du NaBH(OAc)₃ (952 mg). Le milieu est agité pendant une nuit à température ambiante. Après évaporation à sec le résidu est repris dans le CH₂Cl₂ et de l'eau. La phase aqueuse est extraite au CH₂Cl₂. Les phases organiques sont rassemblées puis extraites avec une solution aqueuse de HCl IN. La phase aqueuse est alcalinisée puis extraite au CH₂Cl₂. La phase organique est séchée sur sulfate de magnésium, filtrée et évaporée à sec pour conduire au produit du titre qui est utilisé directement dans l'étape suivante.

### Stade E: Iodure de [5-(éthoxycarbonyl)-1H-pyrrol-3-yl]-N,N,N-triméthylméthanaminium

A une solution du produit obtenu dans le stade D (0,3 g) dans un mélange tétrahydrofurane (2,2 ml) CH₂Cl₂ (2,2 ml) est ajouté de l'iodure de méthyle (0,350 ml). Apparition d'un précipité. Le milieu réactionnel est agité une heure à température ambiante puis laissé à 4°C pendant une nuit. Le milieu réactionnel est évaporé à sec et le résidu obtenu est directement utilisé dans le stade suivant.

### Stade F : 4-(Cyanométhyl)-1H-pyrrole-2-carboxylate d'éthyle

Une solution dans l'éthanol du produit obtenu dans le stade E et de cyanure de sodium (0,225 g) est portée au reflux pendant 72 heures. Après évaporation à sec, le résidu est repris dans un mélange CH₂Cl₂ / eau. La phase aqueuse est extraite trois fois au CH₂Cl₂. Les phases organiques sont rassemblées. La phase organique est séchée sur sulfate de magnésium, filtrée et évaporée à sec pour conduire au produit du titre qui est utilisé directement dans l'étape suivante.

### Stade G : 4-(2-Aminoéthyl)-1H-pyrrole-2-carboxylate d'éthyle

A une solution du produit obtenu dans le stade F (7,45 g) dans le MeOH (1,5 L) est ajouté 223 ml d'une solution de NH₃ 1,4 N dans le MeOH et du PtO₂ (5,2 g). Le milieu réactionnel est mis sous atmosphère d'hydrogène puis agité pendant 20 heures à température ambiante. Après filtration et évaporation à sec, le résidu obtenu est purifié sur gel de silice (SiO2 : Gradient CH₂Cl₂ / MeOH. Adjuvant NH₃ 1,4N dans le MeOH) pour conduire au produit du titre qui est utilisé directement dans le stade suivant.

### Stade H : 4-[2-(Pyrrolidin-1-yl)éthyl]-1H-pyrrole-2-carboxylate d'éthyle

A une solution du produit obtenu dans le stade G (1,3 g) dans le DMF (134 ml) est ajouté de la 2,6-lutidine (4,15 ml) et le 1,4-dibromo-butane (4,68 ml). Le milieu réactionnel est agité à 80°C pendant 12 heures et une nuit à température ambiante. Le milieu réactionnel est dilué dans 600 ml d'acétate d'éthyle. La phase organique est lavée avec une solution aqueuse saturée de NaHCO₃, à l'eau et avec une solution aqueuse saturée de NaCl, séchée sur sulfate de magnésium, filtrée et évaporée à sec. Le résidu obtenu est purifié sur gel de silice (SiO₂ : Gradient CH₂Cl₂ / MeOH. Adjuvant NH₃ 1,4N dans le MeOH) pour conduire au produit du titre qui est utilisé directement dans le stade suivant.

### Stade I : {4-[2-(Pyrrolidin-1-yl)éthyl]-1H-pyrrol-2-yl}méthanol

A une solution du produit obtenu dans le stade H (0,2 g) dans le THF (10 ml) à 0°C est ajouté par portions de l'hydrure de lithium et d'aluminium. Après 3 heures d'agitation à température ambiante le milieu réactionnel est hydrolysé à 0°C avec une solution de NaOH aq. et de l'eau. Après filtration le filtrat est évaporé à sec et le résidu obtenu purifié sur gel de silice (SiO₂: Gradient CH₂Cl₂ / MeOH. Adjuvant NH₃ 1,4N dans le MeOH) pour conduire au produit du titre qui est utilisé directement dans le stade suivant.

### Stade J : 4-[2-(Pyrrolidin-1-yl)éthyl]-1H-pyrrole-2-carbaldéhyde

A une solution du produit obtenu dans le stade I (0,09 g) dans le THF (3,8 ml) est ajouté du MnO₂ activé (0,315 g). Après 20 heures d'agitation à température ambiante, le milieu est filtré et le filtrat évaporé à sec pour conduire au produit du titre qui est directement utilisé dans l'étape suivante.

### Préparation 19 : 4-[2-(Morpholin-4-yl)éthyl]-1H-pyrrole-2-carbaldéhyde

Le produit du titre est obtenu comme décrit dans la préparation 18 en remplaçant dans le stade H le 1,4-dibromo-butane par le 1-bromo-2-(2-bromoéthoxy)éthane.

### Préparation 20: 5-[(3aR,6aS)-Hexahydrocyclopenta[c]pyrrol-2(1H)-ylméthyl]-1H-pyrrole-2-carbaldéhyde

On procède comme dans la préparation 1 en remplaçant la morpholine par le (3aR,6aS)-héxahydro-cyclopenta[c]pyrrole.
*Spectrométrie de masse* (ES +, m/z) : 219.1494 (M+H)

### Préparation 21: 4-[(4-Méthoxypipéridin-1-yl)méthyl]-1H-pyrrole-2-carbaldéhyde

On procède comme dans la préparation 1 en remplaçant la morpholine par la 4-méthoxypipéridine.

### Préparation 22: 4-(Morpholin-4-ylméthyl)-1H-pyrrole-3-carbaldéhyde

### Stade A : 4-(Morpholin-4-ylcarbonyl)-1H-pyrrole-3-carboxylate de méthyle

A une solution de 4-(méthoxycarbonyl)-1*H*-pyrrole-3-carboxylic acid (2,35g) dans le DMF (49 ml) est ajouté de la diisopropyléthylamine (2,11 ml) et du HATU (4,9 ml). Après 15 minutes d'agitation à 40°C de la morpholine (1,35 ml) est ajoutée. Après 9 heures d'agitation à 60°C et à température ambiante pendant un week-end, on dilue avec du CH₂Cl₂ et une solution de soude IN. La phase aqueuse est extraite 3 fois au CH₂Cl₂ et les phases organiques sont rassemblées. La phase organique est séchée sur sulfate de magnésium, filtrée et évaporée à sec pour conduire au produit du titre qui est utilisé directement dans l'étape suivante.

### Stade B : [4-(Morpholin-4-ylméthyl)-1H-pyrrol-3-yl]méthanol

Le produit du titre est obtenu en utilisant le protocole décrit dans le stade I de la préparation 18 et en partant du produit obtenu dans le Stade A.

### Stade C : 4-Morpholin-4-ylméthyl-1H-pyrrole-3-carbaldéhyde

Le produit du titre est obtenu en utilisant le protocole décrit dans le stade J de la préparation 18 et en partant du produit obtenu dans le Stade B.

En procédant comme dans la Préparation 1 en partant des réactifs appropriés on obtient la Préparation suivante :

### Préparation 23 : 4-[(2-methyl-4-morpholinyl)methyl]-1H-pyrrole-2-carbaldehyde

### Exemple 1 : 3-[(3-{[4-(4-Morpholinylméthyl)-1H-pyrrol-2-yl]méthylène}-2-oxo-2,3-dihydro-1H-indol-5-yl)méthyl]-1,3-thiazolidine-2,4-dione, chlorhydrate

### Stade A : 3-Méthylsulfanyl-2-oxo-2,3-dihydro-1H-indole-5-carboxylate d'éthyle

A une solution de 4-amino-benzoate d'éthyle (40,0 g) dans le DCM (900 ml) à -60°C est ajouté goutte à goutte le (méthylsulfanyl)acétate d'éthyle (34,5 ml) sous atmosphère d'azote. Puis 29,0 g de tBuOCl sont ajoutés goutte à goutte au milieu réactionnel en 25 minutes en gardant la température entre -60°C et -55°C. Après une heure d'agitation à - 50°C, de la triéthylamine (15,4m1) est ajoutée goutte à goutte. Le milieu réactionnel est amené à 0°C puis une solution aqueuse de HCl 3M (485 ml) est ajoutée goutte à goutte. Après 12 heures d'agitation à température ambiante les phases sont séparées et la phase organique est lavée successivement par une solution de HCl aq. 2M, de l'eau puis par une solution aqueuse de NaCl sat. La phase organique est séchée sur sulfate de sodium, filtrée et évaporée à sec. Le résidu obtenu est repris dans l'éther. Les cristaux obtenus sont filtrés, lavés à l'éther et séchés sous vide pour conduire au produit du titre, engagé directement dans l'étape suivante.

### Stade B : 2-Oxo-2,3-dibydro-1H-indole-5-carboxylate d'éthyle

A une solution du composé obtenu au Stade A (0,1448 mol) dans l'acide acétique glacial (700 ml) est ajouté du zinc en poudre (0,8690 mol). Le mélange biphasique est agité à 40°C pendant 8 heures. Le milieu réactionnel est filtré puis le filtrat est évaporé à sec. Le résidu est agité dans l'eau pendant une nuit, filtré et le précipité obtenu lavé à l'eau. Le produit est repris dans le toluène puis évaporé à sec puis repris dans l'éther, filtré et lavé à l'éther puis séché sous vide à 40°C sous P₂O₅ pour conduire au produit du titre qui est engagé directement dans l'étape suivante.

### Stade C : 5-Hydroxyméthyl-1,3-dihydro-indol-2-one

A une solution/suspension du composé obtenu au Stade B (66,8 mmol) dans le THF (850 ml) à -65°C est ajouté goutte à goutte une solution 1M de DIBAL-H (546 mmol) en une heure et sous atmosphère inerte. Le Milieu réactionnel et amené à -30°C en 1h30. 22 ml d'eau sont ajoutés goutte à goutte, puis 22 ml d'une solution aqueuse à 15% de soude à 0°C puis 55 ml d'eau. Du MgSO₄ (300 g) est ajouté au milieu réactionnel et l'agitation est maintenue pendant 30 minutes. La suspension est filtrée sur célite et lavée au THF. Le filtrat est évaporé à sec pour donner un solide pateux de couleur brune. Le résidu est trituré dans le méthanol, et la poudre obtenue est filtrée, séchée sous vide pour conduire au produit du titre qui est engagé directement dans l'étape suivante.

### Stade D : 5-Hydroxyméthyl-3-(4-morpholin-4-ylméthyl-1H-pyrrol-2-ylméthylène)-1,3-dihydro-indol-2-one

Une solution du composé obtenu au Stade C (18 mmol), et du composé obtenu dans la Préparation 1 (19 mmol) et de pipéridine (18 mmol) dans l'éthanol est portée au reflux. Après 40 minutes le milieu réactionnel est refroidi dans un bain de glace. Le précipité formé est filtré, lavé à l'éthanol froid. Le produit obtenu et repris dans le toluène, porté au reflux pendant 5 minutes puis évaporé à sec et séché sous vide pour conduire au produit du titre. Mélange d'isomères Z / E (98/2)
*Spectrométrie de masse* (ES +, m/z) : 340,1657 (M+H)⁺ et 340,1668 (M+H)⁺

### Stade E : 3-[(3-{[4-(4-Morpholinylméthyl)-1H-pyrrol-2-yl]méthylène}-2-oxo-2,3-dihydro-1H-indol-5-yl)méthyl]-1,3-thiazolidine-2,4-dione, chlorhydrate

15,4 mmol de thiazolidine-2,4-dione et de la triphénylphosphine greffée sur résine (3 mmol/g, 30,8 mmol) sont agités sous atmosphère inerte pendant 10 minutes à température ambiante dans le THF anhydre. Le milieu est refroidi par un bain de glace et du *tert-butyl* azodicarboxylate (15,4 mmol) est ajouté. Après 10 minutes d'agitation à 0°C est ajouté le composé obtenu dans le Stade D (10,2 mmol). Après 1h30 d'agitation à 0°C le milieu est filtré et la résine lavée au THF. Le filtrat est évaporé à sec puis chromatographié sur silice. Le produit obtenu est repris dans un mélange DCM / Méthanol (4/1) (150ml) puis une solution de HCl 1,25M dans l'éthanol (10 ml) est ajoutée. La solution est concentrée à environ 15 ml puis refroidie dans un bain de glace.
Les cristaux formés sont filtrés sous azote et lavés à l'éthanol froid et séchés sous vide à 40°C pour conduire au produit du titre. Mélange d'isomères Z / E (99/1) *Spectrométrie de masse* (ES +, m/z) : 439,1439 (M+H)⁺ et 439,1431 (M+H)⁺
*Point de fusion : 211 °C*
*Microanalyse élémentaire:*

| | *C* | *H* | *N* | *S* | *Cl⁻* |
|---|---|---|---|---|---|
| *% théorique* | *55, 63* | *4,88* | *11,80* | *6,75* | *7,46* |
| *% expérimental* | *55,36* | *4,73* | *11,78* | *6,72* | *7,84* |

### Exemple 1a : 3-[(3-{[4-(4-Morpholinylméthyl)-1H-pyrrol-2-yl]méthylène}-2-oxo-2,3-dihydro-1H-indol-5-yl)méthyl]-1,3-thiazolidine-2,4-dione, chlorhydrate

Le composé du titre est obtenu par purification sur gel de silice (SiO₂; Gradient CH₂Cl₂ / MeOH) du composé obtenu dans le stade E exemple 1 avant salification, puis transformé en chlorhydrate tel que décrit dans le stade E exemple 1 pour conduire au produit du titre.
Mélange d'isomères Z / E (1,5 / 98,5)
*Spectrométrie de masse* (ES +, m/z) : 439,1422 (M+H)⁺ et 439,1448 (M+H)⁺
*Microanalyse élémentaire :*

| | *C* | *H* | *N* | *S* | *Cl⁻* |
|---|---|---|---|---|---|
| *% théorique* | *55, 63* | *4,88* | *11,80* | *6,75* | *7,46* |
| *% expérimental* | *56, 06* | *4,81* | *11,84* | *6,99* | *7,48* |

### Exemple 1b : 3-[(3-{[4-(4-Morpholinylméthyt)-1H-pyrrol-2-yl]méthylène}-2-oxo-2,3-dihydro-1H-indol-5-yl)méthyl]-1,3-thiazolidine-2,4-dione

15,4 mmol de thiazolidine-2,4-dione et de la triphénylphosphine greffée sur résine (3 mmol/g, 30,8 mmol) sont agités sous atmosphère inerte pendant 10 minutes à température ambiante dans le THF anhydre. Le milieu est refroidi par un bain de glace et du tert-butyl azodicarboxylate (15,4 mmol) est ajouté. Après 10 minutes d'agitation à 0°C est ajouté le composé obtenu dans le Stade D de l'Exemple 1 (10,2 mmol). Après 1h30 d'agitation à 0°C le milieu est filtré et la résine lavée au THF. Le filtrat est évaporé à sec puis chromatographié sur silice.
*Spectrométrie de masse* (ES +, m/z) : 439,1435 (M+H)⁺

### Exemple 2 : 3-{3-[1-(4-Morpholin-4-ylméthyl-1H-pyrrol-2-yl)-éthylidène]-2-oxo-2,3-dihydro-1H-indol-5-ylméthyl}-thiazolidine-2,4-dione, chlorhydrate

### Stade A : 5-Hydroxyméthyl-3-[1-(4-morpholin-4-ylméthyl-1H-pyrrol-2-yl)-éthylidènel-1,3-dihydro-indol-2-one

Une solution du composé obtenu dans le Stade C de l'Exemple 1 (2,5 mmoles), du composé obtenu dans la Préparation 2 (2,9 mmoles) et d'acétate d'ammonium (5,2 mmoles) dans l'éthanol (4 ml) est portée à 120°C sous micro-ondes pendant 20 minutes. Le précipité obtenu est filtré lavé à l'éthanol et l'éther éthylique puis séché sous vide pour conduire au produit du titre. Mélange d'isomères Z / E (99/1).
*Spectrométrie de masse* (ES +, m/z) : 354,1799 (M+H)⁺ et 354,1793 (M+H)⁺

### Stade B : 3-{3-[1-(4-Morpholin-4-ylméthyl-1H-pyrrol-2-yl)-éthylidène]-2-oxo-2,3-dihydro-1H-indol-5-ylméthyl}-thiazolidine-2,4-dione,chlorhydrate

Le produit du titre est obtenu en suivant le protocole décrit dans le stade E de l'Exemple 1 par condensation du composé obtenu dans le Stade A (1,3 mmole) et de la thiazolidine-2,4-dione (1,7 mmole). Le produit obtenu après traitement est purifié sur gel de silice (SiO₂; Gradient AcOEt / MeOH) puis transformé en chlorhydrate tel que décrit dans le Stade E exemple 1 pour conduire au produit du titre. Mélange d'isomères Z / E (99/1)
*Spectrométrie de masse* (ES +, m/z) : 453,1586 (M+H)⁺ et 453,1600 (M+H)⁺
*Point de fusion : 225 °C*
*Microanalyse élémentaire :*

| | *C* | *H* | *N* | *S* | *Cl⁻* |
|---|---|---|---|---|---|
| *% théorique* | *56,49* | *5,15* | *11,46* | *6.56* | *7, 25* |
| *% expérimental* | *55,90* | *5, 00* | *11, 06* | *6, 25* | *8, 29* |

### Exemple 3 : 3-[3-(4-Morpholin-4-ylméthyl-1H-pyrrol-2-ylméthylène)-2-oxo-2,3-dihydro-1H-indol-5-ylméthyl]-oxazolidine-2,4-dione, chlorhydrate

### Stade A : Oxazolidine-2,4-dione

A une solution de NaOMe (1,05 mmole) dans le MeOH (21 ml) sont ajoutés du 2-hydroxyacétamide (1 mmole) et du diéthyle carbonate (1,15 mmoles) puis le milieu réactionnel est porté au reflux pendant 1,5 heure. Le milieu réactionnel est évaporé à sec puis repris dans l'eau. La phase aqueuse est extraite au diéthyle éther. La phase aqueuse est acidifiée (pH 2) puis évaporée à sec. Le résidu est trituré dans AcOEt et filtré. Le produit obtenu est purifié sur gel de silice (c-Hexane / AcOEt; 1/1) pour conduire au produit du titre sous la forme d'une poudre blanche.

### Stade B : 3-[3-(4-Morpholin-4-ylméthyl-1H-pyrrol-2-ylméthylène)-2-oxo-2,3-dihydro-1H-indol-5-ylméthyl]-oxazolidine-2,4-dione, chlorhydrate

Le produit du titre est obtenu suivant le protocole décrit dans le Stade E de l'Exemple 1 par condensation du composé obtenu dans le Stade D de l'Exemple 1 (4,8 mmol) et le composé obtenu dans le Stade A (7,31 mmol). Le produit obtenu après filtration du milieu réactionnel est purifié sur gel de silice (SiO₂; Gradient AcOEt / MeOH ) puis transformé en chlorhydrate tel que décrit dans le Stade E de l'Exemple 1 pour conduire au produit du titre.
Mélange d'isomères Z / E (97 / 3)
*Spectrométrie de masse* (ES +, m/z) : 423,1670 (M+H)⁺ et 423,1697 (M+H)⁺
*Point de fusion : 216 °C*
*Microanalyse élémentaire :*

| | *C* | *H* | *N* | *Cl⁻* |
|---|---|---|---|---|
| *% théorique* | *57,58* | *5,05* | *12, 21* | *7,73* |
| *% expérimental* | *57,55* | *4,80* | *12, 21* | *7,96* |

### Exemple 4 : 3-{3-[1-(4-Morpholin-4-ylméthyl-1H-pyrrol-2-yl)-éthylidène]-2-oxo-2,3-dihydro-1H-indol-5-ylméthyl}-oxazolidine-2,4-dione, chlorhydrate

Le produit du titre est obtenu suivant le protocole décrit dans le Stade E de l'Exemple 1 par condensation du produit obtenu dans le Stade A de l'Exemple 2 (1,54 mmol) et le composé obtenu dans le Stade A de l'Exemple 3 (2,31 mmol). Le produit obtenu après traitement du milieu réactionnel est purifié sur gel de silice (SiO₂; Gradient AcOEt à AcOEt / MeOH, 9/1), puis transformé en chlorhydrate tel que décrit dans le Stade E de l'Exemple 1 pour conduire au produit du titre. Mélange d'isomères Z / E (99 / 1)
*Spectrométrie de masse* (ES +, m/z) : 437,1816 (M+H)⁺ et 437,1837 (M+H)⁺
*Point de fusion : 220 °C*
*Microanalyse élémentaire :*

| | *C* | *H* | *N* | *Cl⁻* |
|---|---|---|---|---|
| *% théorique* | *58,41* | *5,33* | *11,85* | *7,50* |
| *% expérimental* | *57,51* | *4,84* | *12, 07* | *8,51* |

### Exemple 5 : 3-{3-[4-((1R,5S)-3-Aza-bicyclo[3.1.0]hex-3-ylméthyl)-3,5-diméthyl-1H-pyrrol-2-ylméthylène]-2-oxo-2,3-dihydro-1H-indol-5-ylméthyl}-oxazolidine-2,4-dione, chlorhydrate

### Stade A : 3-[4-((1R,5S)-3-Aza-bicyclo[3.1.0]hex-3-ylméthyl)-3,5-diméthyl-1H-pyrrol-2-ylméthylène]-5-hydroxyméthyl-1,3-dihydro-indol-2-one

Le produit du titre est obtenu suivant le protocole décrit dans le Stade D de l'Exemple 1 par condensation du composé obtenu dans le Stade C de l'Exemple 1 (0,92 mmole) et le composé obtenu dans la Préparation 3 (1 mmole) en présence de pipéridine dans l'éthanol à 105°C sous activation micro-ondes. Après filtration, le solide obtenu est purifié sur gel de silice (SiO₂; Gradient AcOEt / MeOH) pour conduire au produit du titre. Mélange isomères Z/E(98/2)
*Spectrométrie de masse* (ES +, m/z) : 362,1875 (M-H)⁺ et 362,1883 (M-H)⁺

### Stade B : 3-{3-[4-((1R,5S)-3-Aza-bicyclo[3.1.0]hex-3-ylméthyl)-3,5-diméthyl-1H-pyrrol-2-ylméthylène]-2-oxo-2,3-dihydro-1H-indol-5-ylméthyl}-oxazolidine-2,4-dione, chlorhydrate

Le produit du titre est obtenu suivant le protocole décrit dans le Stade E de l'Exemple 1 par condensation du composé obtenu dans le Stade A (0,63 mmole) et l'oxazolidine-2,4-dione (0,95 mmole). Le produit obtenu après traitement du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (99 / 1)
*Spectrométrie de masse* (ES +, m/z) : 445,1865 (M-H)⁺ et 445,1846 (M-H)⁺
*Microanalyse élémentaire :*

| | *C* | *H* | *N* | *Cl⁻* |
|---|---|---|---|---|
| *% théorique* | *62,17* | *5, 63* | *11, 60* | *7,34* |
| *% expérimental* | *61,98* | *6, 28* | *11,55* | *7,44* |

### Exemple 6 : 1-[3-(4-Morpholin-4-ylméthyl-1H-pyrrol-2-ylméthylène)-2-oxo-2,3-dihydro-1H-indol-5-ylméthyl]-pyrrolidine-2,5-dione, chlorhydrate

Le produit du titre est obtenu suivant le protocole décrit dans le Stade E de l'Exemple 1 en remplaçant la thiazolidine-2,4-dione par la pyrrolidine-2,5-dione. Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (97 / 3)
*Spectrométrie de masse* (ES +, m/z): 421,1863 (M+H)⁺ et 421,1881 (M+H)⁺
*Point de fusion : 212 °C*
*Microanalyse élémentaire :*

| | *C* | *H* | *N* | *Cl⁻* |
|---|---|---|---|---|
| *% théorique* | *60,46* | *5,51* | *12, 26* | *7,76* |
| *% expérimental* | *60,75* | *5.37* | *12, 67* | *8, 20* |

### Exemple 7 : 5,5-Diméthyl-3-[3-(4-morpholin-4-ylméthyl-1H-pyrrol-2-ylméthylène)-2-oxo-2,3-dibydro-1H-indol-5-ylméthyl]-oxazolidine-2,4-dione, chlorhydrate

Le produit du titre est obtenu suivant le protocole décrit dans le Stade E de l'Exemple 1 en remplaçant la thiazolidine-2,4-dione par la 5,5-diméthyl-oxazolidine-2,4-dione. Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (96 / 4)
*Spectrométrie de masse* (ES +, m/z) : 451,1981 (M+H)⁺ et 451,1981 (M+H)⁺
*Point de fusion : 191 °C*
*Microanalyse élémentaire :*

| | *C* | *H* | *N* | *Cl⁻* |
|---|---|---|---|---|
| *% théorique* | *59, 20* | *5,59* | *11,51* | *7, 28* |
| *% expérimental* | *59,37* | *5,41* | *11,42* | *7,44* |

### Exemple 8 : 1-Méthyl-3-[3-(4-morpholin-4-ylméthyl-1H-pyrrol-2-ylméthylène)-2-oxo-2,3-dihydro-1H-indol-5-ylméthyl]-imidazolidine-2,4-dione, chlorhydrate

Le produit du titre est obtenu suivant le protocole décrit dans le Stade E de l'Exemple 1 en remplaçant la thiazolidine-2,4-dione par la 1-méthyl-imidazolidine-2,4-dione. Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (97,5 / 2,5)
*Spectrométrie de masse* (ES +, m/z) : 436,1981 (M+H)⁺ et 436,2001 (M+H)⁺
*Microanalyse élémentaire :*

| | *C* | *H* | *N* | *Cl⁻* |
|---|---|---|---|---|
| *% théorique* | *58,54* | *5,55* | *14,84* | *7,51* |
| *% expérimental* | *57,74* | *5, 28* | *14, 60* | *7,24* |

### Exemple 9 : 5-(4-Chloro-benzylidène)-3-[3-(4-morpholin-4-ylméthyl-1H-pyrrol-2-yl méthylène)-2-oxo-2,3-dihydro-1H-indol-5-ylméthyl]-thiazolidine-2,4-dione, chlorhydrate

### Stade A : 5-(4-Chloro-benzylidène)-thiazolidine-2,4-dione

Une solution de 4-chloro-benzaldéhyde (3 mmoles), de thiazolidine-2,4-dione (3 mmoles) et de pipéridine (3 mmoles) dans l'éthanol est portée au reflux pendant une nuit.
Le milieu réactionnel est refroidi dans un bain de glace. Les cristaux formés sont filtrés, lavés à l'éthanol froid et séchés sous vide (40°C) pour conduire au produit du titre.
*Spectrométrie de masse* (ES +, m/z) : 237,9732 (M-H)⁺

### Stade B : 5-(4-Chloro-benzylidène)-3-[3-(4-morpholin-4-ylméthyl-1H-pyrrol-2-yl méthylène)-2-oxo-2,3-dihydro-1H-indol-5-ylméthyl]-thiazolidine-2,4-dione, chlorhydrate

Le produit du titre est obtenu suivant le protocole décrit dans le Stade E de l'Exemple 1 en remplaçant la thiazolidine-2,4-dione par le composé obtenu dans le Stade A. Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (98,5 / 1,5) *Spectrométrie de masse* (ES +, m/z) : 561,1377 (M+H)⁺ et 561,1383 (M+H)⁺
*Microanalyse élémentaire :*

| | *C* | *H* | *N* | *S* | *Cl* |
|---|---|---|---|---|---|
| *% théorique* | *58, 29* | *4,39* | *9,38* | *5,37* | *7,51* |
| *% expérimental* | *57,79* | *4,30* | *9, 25* | *5,19* | *7, 24* |

### Exemple 10 : 3-{3-[4-((1R,5S)-3-Aza-bicyclo[3.1.0]hex-3-ytméthyl)-1H-pyrrol-2-ylméthylène]-2-oxo-2,3-dihydro-1H-indol-5-ylméthyl}-thiazolidine-2,4-dione, chlorhydrate

### Stade A : 3-[4-((1R,5S)-3-Aza-bicyclo[3.1.0]hex-3-ylméthyl)-1H-pyrrol-2-ylméthylène]-5-hydroxyméthyl-1,3-dihydro-indol-2-one

Une solution de 5-hydroxyméthyl-1,3-dihydro-indol-2-one (2,75 mmoles), du composé obtenu dans la Préparation 4 (3,30 mmoles) et de pipéridine (2,75 mmoles) dans l'éthanol est portée au reflux. Après 2 heures le milieu réactionnel est évaporé et le résidu trituré dans l'AcOEt. Le précipité formé est filtré pour conduire au produit du titre sous la forme d'un mélange d'isomères Z / E (96 / 4)
*Spectrométrie de masse* (ES +, m/z) : 336,1706 (M+H)⁺ et 336,1733 (M+H)⁺

### Stade B : 3-{3-[4-((1R,5S)-3-Aza-bicyclo[3.1.0]hex-3-ylméthyl)-1H-pyrrol-2-ylméthylène]-2-oxo-2,3-dihydro-1H-indol-5-ylméthyl}-thiazolidine-2,4-dione, chlorhydrate

Le produit du titre est obtenu suivant le protocole décrit dans le Stade E de l'Exemple 1 par condensation du composé obtenu dans le Stade B (2,02 mmoles) et la 5-(4-chloro-benzylidène)-thiazolidine-2,4-dione (3,04 mmoles). Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (97 / 3)
*Spectrométrie de masse* (ES +, m/z) : 435,1486 (M+H)⁺ et 435,1493 (M+H)⁺
*Microanalyse élémentaire :*

| | *C* | *H* | *N* | *S* | *Cl⁻* |
|---|---|---|---|---|---|
| *% théorique* | *58, 66* | *4,92* | *11,90* | *6,81* | *7,53* |
| *% expérimental* | *57,82* | *4,84* | *11,58* | *6, 60* | *7,59* |

### Exemple 11 : 3-(4-Morpholin-4-ylméthyl-1H-pyrrol-2-ylméthylène)-5-(2-oxo-[1,3,4]thiadiazol-3-ylméthyl)-1,3-dihydro-indol-2-one, chlorhydrate

Le produit du titre est obtenu suivant le protocole décrit dans le Stade E de l'Exemple 1 en remplaçant la thiazolidine-2,4-dione par la 3*H*-[1,3,4]thiadiazol-2-one. Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (97 / 3)
*Spectrométrie de masse* (ES +, m/z) : 424,1437 (M+H)⁺ et 424,1459 (M+H)⁺
*Microanalyse élémentaire :*

| | *C* | *H* | *N* | *S* | *Cl⁻* |
|---|---|---|---|---|---|
| *% théorique* | *54,84* | *4,82* | *15,23* | *6,97* | *7,71* |
| *% expérimental* | *54,60* | *4, 65* | *15, 07* | *6,89* | *7,72* |

### Exemple 12 : 5,5-Diméthyl-3-[3-(4-morpholin-4-ylméthyl-1H-pyrrol-2-ylméthylène)-2-oxo-2,3-dihydro-1H-indol-5-ylméthyl]-thiazolidine-2,4-dione, chlorhydrate

### Stade A : 5,5-Diméthyl-thiazolidine-2,4-dione

41,4 mmoles de 2-bromo-2-méthyl-propionate de méthyle et 4,56 mmole de thiourée sont mis en solution dans le n-butanol (90 mL) et agités à 110°C pendant 5 h. Le solvant est évaporé à sec et le résidu repris dans l'éthanol (120 ml) et HCl (2N, 120 mL) puis chauffé au reflux pendant 16 heures. L'éthanol est évaporé et le milieu dilué avec de l'eau (120 ml) puis extrait à l'AcOEt. La phase organique est lavée avec une solution saturée de NaCl, séchée sur sulphate de magnésium, filtré et évaporée à sec. Les cristaux obtenus sont lavés au pentane filtrés et séchés sous vide pour conduire au produit du titre.
*Point de fusion : 76,6°C*

### Stade B : 5,5-Diméthyl-3-[3-(4-morpholin-4-ylméthyl-1H-pyrrol-2-ylméthylène)-2-oxo-2,3-dihydro-1H-indol-5-ylméthyl]-thiazolidine-2,4-dione,chlorhydrate

Le produit titre est obtenu suivant le protocole décrit dans le Stade E de l'Exemple 1 en remplaçant la thiazolidine-2,4-dione par le composé obtenu dans le Stade A. Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (95 / 5)
*Spectrométrie de masse* (ES +, m/z) : 467,1754 (M+H)⁺ et 467,1750 (M+H)⁺
*Microanalyse élémentaire :*

| | *C* | *H* | *N* | *S* | *Cl*⁻ |
|---|---|---|---|---|---|
| *% théorique* | *57,31* | *5,41* | *11,14* | *6,37* | *7,05* |
| *% expérimental* | *56,99* | *5, 27* | *11,58* | *6,30* | *7,48* |

### Exemple 13 : 5-Isopropylidène-3-[3-(4-morpholin-4-ylméthyl-1H-pyrrol-2-yl méthylène)-2-oxo-2,3-dihydro-1H-indol-5-ylméthyl]-thiazolidine-2,4-dione, chlorhydrate

Le produit du titre est obtenu suivant le protocole décrit dans le Stade E de l'Exemple 1 en remplaçant la thiazolidine-2,4-dione par la 5-isopropylidene-thiazolidine-2,4-dione. Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (99 / 1)
*Spectrométrie de masse* (ES +, m/z) : 479,1739 (M+H)⁺ et 479,1744 (M+H)⁺
*Microanalyse élémentaire :*

| | *C* | *H* | *N* | *S* | *Cl⁻* |
|---|---|---|---|---|---|
| *% théorique* | *58,30* | *5, 28* | *10,88* | *6, 23* | *6,88* |
| *% expérimental* | *58,09* | *5,19* | *10,81* | *6,35* | *6,58* |

### Exemple 14 : 3-[3-(1-Méthyl-4-morpholin-4-ylméthyl-1H-pyrrol-2-ylméthylène)-2-oxo-2,3-dihydro-1H-indol-5-ylméthyl]-thiazolidine-2,4-dione, chlorhydrate

### Stade A : 5-Hydroxyméthyl-3-(1-méthyl-4-morpholin-4-ylméthyl-1H-pyrrol-2-ylméthylène)-1,3-dihydro-indol-2-one

Le produit du titre est obtenu suivant le protocole décrit dans le Stade D de l'Exemple 1 par condensation de la 5-hydroxyméthyl-1,3-dihydro-indol-2-one (1,31 mmole) et du composé obtenu dans la Préparation 6 (1,44 mmoles) en présence de pipéridine dans l'éthanol à 105°C sous activation micro ondes. Après évaporation à sec le résidu est purifié sur gel de silice (SiO₂; Gradient AcOEt / MeOH) pour conduire au produit du titre. Mélange d'isomères Z/E (65 / 35)
*Spectrométrie de masse* (ES +, m/z) : 354,1791 (M+H)⁺ et 354,1802 (M+H)⁺

### Stade B : 3-[3-(1-Méthyl-4-morpholin-4-ylméthyl-1H-pyrrol-2-ylméthylène)-2-oxo-2,3-dihydro-1H-indol-5-ylméthyl]-thiazolidine-2,4-dione, chlorhydrate

Le produit titre est obtenu suivant le protocole décrit dans le Stade E de l'Exemple 1 par condensation du composé obtenu dans le Stade A (0,88 mmole) et de la thiazolidine-2,4-dione (1,32 mmole). Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z/E(30/70)
*Spectrométrie de masse* (ES +, m/z) : 453,1607 (M+H)⁺ et 453,1585 (M+H)⁺
*Microanalyse élémentaire :*

| | *C* | *H* | *N* | *S* | *Cl⁻* |
|---|---|---|---|---|---|
| *% théorique* | *56,49* | *5,15* | *11,46* | *6,56* | *7,25* |
| *% expérimental* | *56, 29* | *4,96* | *11,01* | *6,68* | *7,23* |

### Exemple 15 : 3-{3-[1-(4-Morpholin4-ylméthyl-1H-pyrrol-2-yl)-propylidène]-2-oxo-2,3-dihydro-1H-indol-5-ylméthyl}-thiazolidine-2,4-dione, chlorhydrate

### Stade A : 5-Hydroxyméthyl-3-[1-(4-morpholin-4-ylméthyl-1H-pyrrol-2-yl)-propylidène]-1,3-dihydro-indol-2-one

Une solution de 5-hydroxyméthyl-1,3-dihydro-indol-2-one (0,22 mmoles), du composé obtenu dans la Préparation 5 (0,44 mmoles) et d'acétate d'ammonium (0 ,44 mmoles) dans du n-butanol (4 ml) est portée à 150°C sous activation micro-ondes pendant 1 heure. Le produit obtenu après évaporation à sec du milieu réactionnel est purifié sur gel de silice (SiO₂; Gradient AcOEt / MeOH) pour conduire au produit du titre. Mélange d'isomères Z / E (92 / 8)
*Spectrométrie de masse* (ES +, m/z) : 368,1941 (M+H)⁺ et 368,1970 (M+H)⁺

### Stade B : 3-{3-[1-(4-Morpholin-4-ylméthyl-1H-pyrrol-2-yl)-propylidène]-2-oxo-2,3-dihydro-1H-indol-5-ylméthyl}-thiazolidine-2,4-dione, chlorhydrate

Le produit du titre est obtenu suivant le protocole décrit dans le Stade E de l'Exemple 1 par condensation du composé obtenu dans le Stade A (0,96 mmole) et de la thiazolidine-2,4-dione (1,44 mmole). Le produit obtenu après filtration du milieu réactionnel est purifié sur gel de silice (SiO₂; Gradient AcOEt / MeOH), puis transformé en chlorhydrate tel que décrit dans le Stade E de l'Exemple 1 pour conduire au produit du titre. Mélange d'isomères Z / E (98,5/1,5)
*Spectrométrie de masse* (ES +, m/z) : 467,1762 (M+H)⁺ et 467,1782 (M+H)⁺

### Exemple 16 : 5-(4-Hydroxy-2-oxo-thiazolidin-3-ylméthyl)-3-(4-morpholin-4-ylméthyl-1H-pyrrol-2-ylméthylène)-1,3-dihydro-indol-2-one

A une solution du composé obtenu dans l'Exemple 1 (1,37 mmole) dans un mélange MeOH/dioxane (42 ml/55 ml) est ajouté NaBH₄ (2,75 mmoles) à 0°C. La réaction est agitée à température ambiante pendant 5 heures. Le milieu réactionnel est évaporé à sec et repris dans l'eau (20 ml) puis extrait à l'AcOEt (4x40 ml). Les phases organiques sont combinées. La phase organique est séchée sur sulfate de sodium, filtrée et évaporée à sec.
Le résidu et purifié sur gel de silice (SiO₂; Gradient DCM / MeOH) pour conduire au produit du titre. Mélange d'isomères Z / E (94,5 / 5,5)
*Spectrométrie de masse* (ES +, m/z) : 441,1608 (M+H)⁺ et 441,1621 (M+H)⁺
*Microanalyse élémentaire :*

| | *C* | *H* | *N* | *S* |
|---|---|---|---|---|
| *% théorique* | *59,98* | *5,49* | *12,72* | *7,28* |
| *% expérimental* | *59,86* | *5,58* | *12,56* | *6,93* |

### Exemple 17 : 3-[3-(1-Méthyl-5-morpholin-4-ylméthyl-1H-pyrrol-2-ylméthylène)-2-oxo-2,3-dihydro-1H-indol-5-ylméthyl]-thiazolidine-2,4-dione, chlorhydrate

### Stade A : 5-Hydroxyméthyl-3-(1-méthyl-5-morpholin-4-ylméthyl-1H-pyrrol-2-yl méthylène)-1,3-dihydro-indol-2-one

Le produit du titre est obtenu suivant le protocole décrit dans le Stade D de l'Exemple 1 par condensation de la 5-hydroxyméthyl-1,3-dihydro-indol-2-one (1,84 mmole) et du composé obtenu dans la Préparation 6 (2,02 mmoles) en présence de pipéridine dans l'éthanol au reflux pendant 4 heures. Le produit obtenu après évaporation à sec du milieu réactionnel est purifié sur gel de silice (SiO₂ ; Gradient AcOEt / MeOH) pour conduire au produit du titre.
Mélange d'isomères Z / E (22 / 78)
*Spectrométrie de masse* (ES +, m/z) : 354,1860 (M+H)⁺ et 376,1577 (M+H)⁺

### Stade B : 3-[3-(1-Méthyl-5-morpholin-4-ylméthyl-1H-pyrrol-2-ylméthylène)-2-oxo-2,3-dihydro-1H-indol-5-ylméthyl]-thiazolidine-2,4-dione, chlorhydrate

Le produit du titre est obtenu suivant le protocole décrit dans le Stade E de l'Exemple 1 par condensation du composé obtenu dans le Stade A (1,63 mmole) et de la thiazolidine-2,4-dione (2,69 mmoles). Le produit obtenu après traitement est purifié sur gel de silice (SiO₂; Gradient DCM / MeOH), puis transformé en chlorhydrate tel que décrit dans le Stade E de l'Exemple 1 pour conduire au produit du titre. Mélange d'isomères Z / E (30 / 70)
*Spectrométrie de masse* (ES +, m/z): 453,1600 (M+H)⁺ et 453,1559 (M+H)⁺

### Exemple 18 : 3-13-(5-Morpholin-4-ylméthyl-1H-pyrrol-2-ylméthylène)-2-oxo-2,3-dihydro-1H-indol-5-ylméthyl]-thiazolidine-2,4-dione, chlorhydrate

### Stade A : 5-Hydroxyméthyl-3-(5-morpholin-4-ylméthyl-1H-pyrrol-2-ylméthylène)-1,3-dihydro-indol-2-one

Le produit du titre est obtenu suivant le protocole décrit dans le Stade D de l'Exemple 1 par condensation de la 5-hydroxymethyl-1,3-dihydro-indol-2-one (1,53 mmole) et du composé obtenu dans la Préparation 7 (1,53 mmole) en présence de pipéridine dans l'éthanol au reflux pendant 3 heures. Le précipité formé est filtré, lavé à l'éthanol. Le produit obtenu est séché sous vide pour conduire au produit du titre. Mélange d'isomères Z / E (99,8/0,2)

### Stade B : 3-[3-(5-Morpholin-4-ylméthyl-1H-pyrrol-2-ylméthylène)-2-oxo-2,3-dihydro-1H-indol-5-ylméthyl]-thiazolidine-2,4-dione, chlorhydrate

Le produit du titre est obtenu suivant le protocole décrit dans le Stade E de l'Exemple 1 par condensation du composé obtenu dans le Stade A (1,21 mmole) et de la thiazolidine-2,4-dione (1,82 mmole). Le produit obtenu après traitement est purifié sur gel de silice (SiO₂; Gradient DCM / MeOH), puis transformé en chlorhydrate tel que décrit dans le Stade E de l'Exemple 1 pour conduire au produit du titre. Mélange d'isomères Z / E (99,5 / 0,5)
*Microanalyse élémentaire :*

| | *C* | *H* | *N* | *S* | *Cl⁻* |
|---|---|---|---|---|---|
| *% théorique* | *55,63* | *4,88* | *11,80* | *6,75* | *7,46* |
| *% expérimental* | *55,05* | *4,81* | *11,93* | *6, 65* | *7,65* |

### Exemple 19 : 3-(4-Morpholin-4-ylméthyl-1H-pyrrol-2-ylméthylène)-5-(2-oxo-oxazolidin-3-ylméthyl)-1,3-dihydro-indol-2-one, chlorhydrate

### Stade A : 3-(4-Nitro-benzyl)-oxazolidin-2-one

A une suspension de NaH (60% en dispersion dans huile, 0,34 mol) dans le THF anhydre (60 ml) à 0°C est ajoutée goutte à goutte une solution d'oxazolidin-2-one (0,31 mol) sous atmosphère inerte. Le milieu réactionnel et amené à température ambiante et agité pendant une heure. Une solution de 1-bromométhyl-4-nitro-benzène (0,35 mol) dans un mélange THF / DMF (10/1) (250 ml) est ajoutée goutte à goutte à 0°C. La réaction est alors agitée une nuit à température ambiante. La solution est alors refroidie à 0°C. Sont ajoutés successivement 20 ml de MeOH et 20 ml d'eau. La suspension obtenue est alors concentrée puis jetée dans 500 ml d'eau. La solution, est alors extraite au DCM, les phases organiques sont rassemblées. La phase organique obtenue est lavée à l'eau et avec une solution aqueuse saturée de NaCl, séchée sur sulfate de sodium puis filtrée. Le filtrat obtenu est concentré et le solide formé filtré, lavé à l'éther éthylique puis séché sous vide pour conduire au produit du titre qui est directement engagé dans l'étape suivante.

### Stade B :_3-(4-Amino-benzyl)-oxazolidin-2-one

Une solution du composé obtenu dans le Stade A (0,244 mol) et de SnCl₂·2H₂O (1,0 mol) dans l'éthanol (300 ml) et agitée 20 minutes au reflux. La solution est amenée à 0°C et alcalinisée à pH = 10-12 avec une solution aqueuse de soude puis 500 ml de DCM sont ajoutés et l'émulsion obtenue est filtrée sur célite. Le filtrat est alors extrait au DCM (2x300 ml), les phases organiques sont rassemblées. La phase organique obtenue est lavée à l'eau et avec une solution aqueuse saturée de NaCl, séchée sur sulfate de sodium puis filtrée. Le filtrat obtenu est évaporé à sec pour conduire au produit du titre qui est directement engagé dans l'étape suivante.

### Stade C :_3-Méthylsulfanyl-5-(2-oxo-oxazolidin-3-ylméthyl)-1,3-dihydro-indol-2-one

A une solution du composé obtenu dans le Stade B (0,113 mol) dans un mélange CH₃CN (200 ml) et THF (200 ml) à -60°C est ajouté goutte à goutte l'éthyl(méthylsulfanyl)acétate (0,136 mol) sous atmosphère d'azote. tBuOCl (0,136 mol) est ajouté goutte à goutte au milieu réactionnel en 20 minutes en gardant la température entre -60°C et -55°C. Après une heure d'agitation à -50°C de la triéthylamine (0,152mol) est ajoutée goutte à goutte. Le milieu réactionnel est amené à 0°C puis une solution aqueuse de HCl 3M (580 ml) est ajoutée goutte à goutte. Après 12 heures d'agitation à température ambiante les phases organiques sont évaporées sous vide puis la phase aqueuse est extraite au DCM et les phases organiques sont rassemblées. La phase organique obtenue est lavée à l'eau puis par une solution aqueuse de NaCl saturée. La phase organique est séchée sur sulfate de sodium, filtrée et évaporée à sec. Le produit obtenu est recristallisé dans un mélange éther éthylique / éthanol. Les cristaux obtenus sont filtrés lavés à l'éther et séché sous vide à 40°C pour conduire au produit du titre qui est directement engagé dans l'étape suivante.

### Stade D : 5-(2-Oxo-oxazolidin-3-ylméthyl)-1,3-dihydro-indol-2-one

A une solution du composé obtenu dans le Stade C dans l'acide acétique glacial (320 ml) est ajouté du Zn en poudre (1 mol). Le milieu réactionnel est agité une nuit à température ambiante puis filtré. Le filtrat est concentré puis 100 ml d'eau est ajouté. Le solide formé est filtré puis recristallisé dans l'éthanol pour conduire au produit du titre.
*Spectrométrie de masse* (ES +, m/z) : 233.0905 (M+H)⁺

### Stade E : 3-(4-Morpholin-4-ylméthyl-1H-pyrrol-2-ylméthylène)-5-(2-oxo-oxazolidin-3-ylméthyl)-1,3-dihydro-indol-2-one, chlorhydrate

Une solution du composé obtenu dans le Stade D (18,8 mmole), du composé obtenu dans la Préparation 1 (20,7 mmole) et de pipéridine (13,2 mmole) dans EtOH (100 mL) est agitée au reflux pendant 3 heures puis à température ambiante pendant douze heures. Le solide jaune formé est filtré à l'eau et à l'éthanol. Le produit obtenu est dissout à chaud dans 300 ml d'un mélange DCM / MeOH (1/1). La solution est amenée à température ambiante et 40 ml d'une solution de HCl (1,25 M) dans l'éthanol sont ajoutés. Le solide jaune formé est filtré sous azote, lavé à l'éther éthylique et séché à 40°C sous vide pendant douze heure pour conduire au produit du titre. Mélange d'isomères Z / E (99 /1).
*Spectrométrie de masse* (ES +, m/z) : 409,1863 (M+H)⁺ et 409,1886 (M+H)⁺
*Microanalyse élémentaire :*

| | *C* | *H* | *N* | *Cl⁻* |
|---|---|---|---|---|
| *% théorique* | *59,39* | *5, 66* | *12,59* | *7,97* |
| *% expérimental* | *60, 02* | *5,56* | *12,79* | *8,10* |

### Exemple 20 : 3-(5-Morpholin-4-ylméthyl-1H-pyrrol-2-ylméthylène)-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (1,5 mmole) et du composé obtenu dans la Préparation 7 (1,65 mmole) en présence de pipéridine dans l'éthanol au reflux. Après évaporation à sec du milieu réactionnel le résidu obtenu purifié par flash chromatographie sur gel de silice (SiO₂; Gradient c-Hexane / AcOEt). Le produit obtenu est transformé en chlorhydrate tel que décrit dans le Stade E de l'Exemple 19 pour conduire au produit du titre. Mélange d'isomères Z / E (98,3/1,7)
*Spectrométrie de masse* (ES +, m/z) : 409,1881 (M+H)⁺ et 409,1892 (M+H)⁺
*Point de fusion :* 195 °C
*Microanalyse élémentaire :*

| | *C* | *H* | *N* | *Cl⁻* |
|---|---|---|---|---|
| *% théorique* | *59,39* | *5, 66* | *12,59* | *7,97* |
| *% expérimental* | *60, 03* | *5,39* | *12, 65* | *8,14* |

### Exemple 21 : 3-(3-Morpholin-4-ylméthyl-1H-pyrrol-2-ylméthylène)-5-(2-oxo-oxazolidin-3-ylméthyl)-1,3-dihydro-indol-2-one

Le produit du titre est obtenu suivant le protocole décrit dans le Stade E de l'Exemple 19 par condensation de la 5-(2-oxo-oxazolidin-3-ylméthyl)-1,3-dihydro-indol-2-one (1,4 mmole) et du composé obtenu dans la Préparation 8 (1,7 mmole) en présence de pipéridine dans l'éthanol au reflux. Le produit obtenu après évaporation à sec est purifié par flash chromatographie sur gel de silice (SiO₂; Gradient DCM à DCM / MeOH 9/1) pour conduire au produit du titre. Mélange d'isomères Z / E (90 /10)
*Spectrométrie de masse* (ES +, m/z) : 409,1856 (M+H)⁺ et 409,1867 (M+H)⁺
*Point de fusion : 192 °C*
*Microanalyse élémentaire :*

| | *C* | *H* | *N* |
|---|---|---|---|
| *% théorique* | *64,69* | *5,92* | *13,72* |
| *% expérimental* | *64,37* | *5,73* | *13,73* |

### Exemple 22 : 3-[1-(4-Morpholin-4-ylméthyl-1H-pyrrol-2-yl)-éthylidène]-5-(2-oxo-oxazolidin-3-ylméthyl)-1,3-dihydro-indol-2-one, chlorhydrate

Un mélange du composé obtenu dans la Préparation 2 (3,22 mmoles), 5-(2-oxo-oxazolidin-3-ylméthyl)-1,3-dihydro-indol-2-one (2,15 mmoles), NH₄OAc (9,73 mmoles) dans l'éthanol est chauffé sous micro-ondes à 110°C pendant 45 minutes. Le précipité formé est filtré puis mis en suspension dans l'eau et de nouveau chauffé 10 minutes à 110°C sous micro-ondes. Le solide obtenu est filtré, mis en suspension dans l'éthanol et de nouveau chauffé 10 minutes à 110°C sous micro-ondes. Le produit obtenu est transformé en chlorhydrate tel que décrit dans le Stade E de l'Exemple 19 pour conduire au produit du titre. Mélange d'isomères Z / E (99,5 / 0,5)
*Spectrométrie de masse* (ES +, m/z) : 423,2012 (M+H)⁺ et 423,2056 (M+H)⁺
*Point de fusion : 212 °C*
*Microanalyse élémentaire :*

| | *C* | *H* | *N* | *Cl⁻* |
|---|---|---|---|---|
| *% théorique* | *60,19* | *5,93* | *12, 21* | *7,72* |
| *% expérimental* | *59,88* | *5,73* | *11,82* | *8,00* |

### Exemple 23 : 3-(5-Diéthylaminométhyl-1H-pyrrol-2-ylméthylène)-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (1,72 mmole) et du composé obtenu dans la Préparation 9 (1,9 mmole) en présence de pipéridine dans l'éthanol au reflux. Le produit obtenu après filtration du milieu réactionnel est purifié par flash chromatographie sur gel de silice (SiO₂; Gradient DCM 100% à DCM / MeOH 9/1). Le produit obtenu est transformé en chlorhydrate tel que décrit dans le Stade E de l'Exemple 19 pour conduire au produit du titre. Mélange d'isomères Z / E (99,5 / 0,5)
*Spectrométrie de masse* (ES +, m/z) : 393,1933 (M+H)⁺ et 393,1933 (M+H)⁺
*Point de fusion : 238 °C*
*Microanalyse élémentaire :*

| | *C* | *H* | *N* | *Cl⁻* |
|---|---|---|---|---|
| *% théorique* | *61,32* | *6,31* | *13, 00* | *8,23* |
| *% expérimental* | *61,44* | *6,31* | *13,37* | *8,33* |

### Exemple 24 : (1R,5S)-3-[3-(4-Morpholin-4-ylméthyl-1H-pyrrol-2-ylméthylène)-2-oxo-2,3-dihydro-1H-indol-5-ylméthyl]-3-aza-bicyclo[3.1.0]hexane-2,4-dione, chlorhydrate

### Stade A : 3-Méthylsulfanyl-2-oxo-2,3-dihydro-1H-indole-5-carbonitrile

A une solution de 4-amino-benzonitrile (84,64 mmoles) dans le DCM (200 ml) sous atmosphère d'azote à -78°C est ajouté goutte à goutte tBuOCl (84,64 mmoles)en solution dans 20 ml de DCM puis après 10 minutes l'éthyl(méthylsulfanyl)acétate (84,64 mmoles) en solution dans 20 ml de DCM est ajouté goutte à goutte au milieu réactionnel. Après une heure d'agitation à -78°C de la triéthylamine (0,152mol) est ajoutée goutte à goutte. Le milieu réactionnel est agité à température ambiante pendant une heure. De l'eau est ajoutée puis les phases sont séparées. La phase organique est lavée à l'eau puis par une solution aqueuse de NaCl saturée. La phase organique est séchée sur sulfate de sodium, filtrée et évaporée à sec. Le résidu est repris dans l'éther éthylique et une solution de HCl aq. 2M est ajoutée. Après une nuit d'agitation le précipité jaune est filtré est séché sous vide pour conduire au produit du titre qui est directement engagé dans la réaction suivante.

### Stade B : 2-Oxo-2,3-dihydro-1H-indole-5-carbonitrile

A une suspension de Ni de Raney dans le THF (100 ml) est ajouté le composé obtenu dans le Stade A (20 mmoles) en solution dans le THF (400 ml). La réaction est agitée à température ambiante jusqu'à disparition complète du produit de départ. Le milieu réactionnel est alors filtré sur un lit de célite puis évaporé à sec pour conduire au produit du titre qui est directement engagé dans la réaction suivante.

### Stade C : 5-Aminométhyl-1,3-dihydro-indol-2-one

Une solution du composé obtenu dans le Stade B (35 mmoles) dans le MeOH (500 ml) est ajouté à 6 ml d'HCl concentré. Le milieu réactionnel est agité 3 jours sous 10 bars d'hydrogène. Après filtration sur célite le filtrat est évaporé à sec pour conduire au produit du titre sous la forme d'une poudre blanche qui est directement engagée dans la réaction suivante.

### Stade D : (1R,5S)-3-(2-Oxo-2,3-dihydro-1H-indol-5-ylmethyl)-3-aza-bicyclo[3.1.0] hexane-2,4-dione

Une solution du composé obtenu dans le Stade C (15 mmoles) et de 3-oxa-bicyclo[3.1.0]hexane-2,4-dione (22,5 mmoles) dans l'acide acétique (60 ml) est portée au reflux pendant 48 heures. Le milieu est amené à température ambiante et le solide formé est filtré pour conduire au produit du titre qui est directement engagé dans la réaction suivante.
*Spectrométrie de masse* (ES +, m/z) : 257,0913 (M+H)⁺

### Stade E : (1R,5S)-3-[3-(4-Morpholin-4-ylméthyl-1H-pyrrol-2-ylméthylène)-2-oxo-2,3-dihydro-1H-indol-5-ylméthyl]-3-aza-bicyclo[3.1.0]hexane-2,4-dione, chlorhydrate

Le produit titre est obtenu suivant le protocole décrit dans le Stade E de l'Exemple 19 par condensation du composé obtenu dans le Stade D (1,54 mmole) et du composé obtenu dans la Préparation 1 (1,7 mmole) en présence de pipéridine dans l'éthanol au reflux. Le produit obtenu après filtration du milieu réactionnel est transformé en chlorhydrate tel que décrit dans le Stade E de l'Exemple 19 pour conduire au produit du titre. Mélange d'isomères Z / E (98,5 / 1,5)
*Spectrométrie de masse* (ES +, m/z) : 433,1857 (M+H)⁺ et 433,1861 (M+H)⁺
*Microanalyse élémentaire :*

| | *C* | *H* | *N* | *Cl⁻* |
|---|---|---|---|---|
| *% théorique* | *61,47* | *5,37* | *11,95* | *7,56* |
| *% expérimental* | *61,01* | *5,50* | *12,03* | *7,21* |

### Exemple 25 : 3-(3,5-Diméthyl-4-morpholin-4-ylméthyl-1H-pyrrol-2-ylméthylène)-5-(2-oxo-oxazolidin-3-ylméthyl)-1,3-dihydro-indol-2-one, chlorhydrate

### Stade A : 3-(3,5-Diméthyl-1H-pyrrol-2-ylméthylène)-5-(2-oxo-oxazolidin-3-ylméthyl)-1,3-dihydro-indol-2-one

Une solution de 5-(2-oxo-oxazolidin-3-ylméthyl)-1,3-dihydro-indol-2-one (4,3 mmoles), 3,5-diméthyl-1*H*-pyrrole-2-carbaldéhyde (5,17 mmoles) et de pipéridine (3 mmoles) dans l'éthanol (30 ml) est portée au reflux pendant 24 heures. Le solide formé est filtré, lavé à l'éthanol, à l'eau puis à l'éthanol et séché sous vide pour conduire au produit du titre.
*Spectrométrie de masse* (ES +, m/z) : 338,1485 (M+H)⁺

### Stade B : 3-(3,5-Diméthyl-4-morpholin-4-ylméthyl-1H-pyrrol-2-ylméthylène)-5-(2-oxo-oxazolidin-3-ylméthyl)-1,3-dihydro-indol-2-one, chlorhydrate

A une solution du composé obtenu dans le Stade A (1,3 mmole) dans l'acide acétique (20 ml) est ajouté à 0°C la morpholine (1,43 mmole) et du formaldéhyde (37% aq. 1,3 mmole). La réaction est agitée pendant une nuit à température ambiante. Le milieu est évaporé à sec et le résidu purifié sur gel de silice (SiO₂ Gradient DCM 100% à DCM / MeOH 9/1). Le produit obtenu est transformé en chlorhydrate tel que décrit dans le Stade E de l'Exemple 19 pour conduire au produit du titre. Mélange d'isomères Z / E (99,5 / 0,5)
*Spectrométrie de masse* (ES +, m/z) : 435,2036 (M+H)⁺ et 435,2035 (M+H)⁺
*Microanalyse élémentaire :*

| | *C* | *H* | *N* | *Cl⁻* |
|---|---|---|---|---|
| *% théorique* | *60,95* | *6,18* | *11,85* | *7,50* |
| *% expérimental* | *60,13* | *5,88* | *11,81* | *7,70* |

### Exemple 26 : 3-[3,5-Diméthyl-4-(4-méthyl-pipérazin-1-ylméthyl)-1H-pyrrol-2-ylméthylène]-5-(2-oxo-oxazolidin-3-ylméthyl)-1,3-dihydro-indol-2-one, dichlorhydrate

A une solution du composé obtenu dans le Stade A de l'Exemple 25 (1,18 mmole) dans l'acide acétique (20 ml) est ajouté à 0°C la 1-méthyl-pipérazine (1,18 mmole) et du formaldéhyde (37% aq. 1,18 mmole). La réaction est agitée pendant une nuit à température ambiante. Après extraction acide base, le produit obtenu est transformé en chlorhydrate tel que décrit dans le Stade E de l'Exemple 19 pour conduire au produit du titre. Isomère Z.
*Spectrométrie de masse* (ES +, m/z) : 448,2377 (M+H)⁺
*Point de fusion* 230°C (décomposition)

### Exemple 27: 3-(3,5-Diméthyl-4-pipéridin-1-ylméthyl-1H-pyrrol-2-ylméthylène)-5-(2-oxo-oxazolidin-3-ylméthyl)-1,3-dihydro-indol-2-one, chlorhydrate

A une solution du composé obtenu dans le Stade A de l'Exemple 25 (1,48 mmole) dans l'acide acétique (20 ml) est ajoutée à 0°C la pipéridine (1,48 mmole) et du formaldéhyde (37% aq. 1,48 mmole). La réaction est agitée pendant une nuit à température ambiante. Après extraction acide base, le produit obtenu est purifié sur gel de silice (SiO₂; Gradient DCM 100% à DCM / MeOH 9/1) puis transformé en chlorhydrate tel que décrit dans le Stade E de l'Exemple 19 pour conduire au produit du titre. Mélange d'isomères Z / E (98,5 / 1,5)
*Spectrométrie de masse* (ES +, m/z) : 435,2367 (M+H)⁺ et 435,2382 (M+H)⁺
*Microanalyse élémentaire :*

| | *C* | *H* | *N* | *Cl⁻* |
|---|---|---|---|---|
| *% théorique* | *63,75* | *6, 63* | *11,90* | *7,53* |
| *% expérimental* | *63,79* | *6,36* | *11,87* | *7,82* |

### Exemple 28 : 3-(4-Morpholin-4-ylméthyl-1H-pyrrol-2-ylméthylène)-5-(2-oxo-thiazolidin-3-ylméthyl)-1,3-dihydro-indol-2-one, chlorhydrate

### Stade A : 5-(2-Oxo-thiazolidin-3-ylméthyl)-1,3-dihydro-indol-2-one

Le produit du titre est obtenu comme dans les Stades A à D de l'Exemple 19 en remplaçant l'oxazolidin-2-one par la thiazolidin-2-one dans le Stade A.

### Stade B : 3-(4-Morpholin-4-ylméthyl-1H-pyrrol-2-ylméthylène)-5-(2-oxo-thiazolidin-3-ylméthyl)-1,3-dihydro-indol-2-one, chlorhydrate

Le produit du titre est obtenu suivant le protocole décrit dans le Stade E de l'Exemple 19 par condensation du composé obtenu dans le Stade A (1,54 mmole) et du composé obtenu dans la Préparation 1 (1,7 mmole) en présence de pipéridine dans l'éthanol au reflux. Le produit obtenu après filtration du milieu réactionnel est purifié sur gel de silice (SiO₂; Gradient DCM 100% à DCM / MeOH 9/1), puis transformé en chlorhydrate tel que décrit dans le Stade E de l'Exemple 19 pour conduire au produit du titre. Mélange d'isomères Z / E (99 / 1)
*Spectrométrie de masse* (ES +, m/z) : 425,1633 (M+H)⁺ et 425,1679 (M+H)⁺
*Point de fusion : 244 °C*
*Microanalyse élémentaire :*

| | *C* | *H* | *N* | S | *Cl⁻* |
|---|---|---|---|---|---|
| *% théorique* | *57,32* | *5,47* | *12,15* | *6,96* | *7,69* |
| *% expérimental* | *56,55* | *5,30* | *11,80* | *6,74* | *8,13* |

### Exemple 29 : 3-[4-(4-Méthyl-pipérazin-1-ylméthyl)-1H-pyrrol-2-ylméthylène]-5-(2-oxo-oxazolidin-3-ylméthyl)-1,3-dihydro-indol-2-one, dichlorhydrate

Le produit titre est obtenu suivant le protocole décrit dans le Stade E de l'Exemple 19 par condensation de la 5-(2-oxo-oxazolidin-3-ylmethyl)-1,3-dihydro-indol-2-one (1,66 mmole) et du composé obtenu dans la Préparation 10 (2,07 mmoles) en présence de pipéridine dans l'éthanol au reflux. Le produit obtenu après évaporation à sec du milieu réactionnel est purifié sur gel de silice (SiO₂ ; Gradient DCM 100% à DCM / MeOH 9/1), puis transformé en chlorhydrate tel que décrit dans le Stade E de l'Exemple 19 pour conduire au produit du titre. Mélange d'isomères Z / E (98 / 2)
*Spectrométrie de masse* (ES +, m/z) : 422,2183 (M+H)⁺ et 422,2198 (M+H)⁺
*Microanalyse élémentaire :*

| | *C* | *H* | *N* | *Cl⁻* |
|---|---|---|---|---|
| *% théorique* | *55,87* | *5,91* | *14,17* | *14,34* |
| *% expérimental* | *56,15* | *5, 65* | *14,30* | *14,22* |

### Exemple 30: 5-[5-(4-Chloro-phényl)-2-oxo-6H-[1,3,4]thiadiazin-3-ylméthyl]-3-(4-morpholin-4-ylméthyl-1H-pyrrol-2-ylméthylène)-1,3-dihydro-indol-2-one, chlorhydrate

### Stade A : 5-(4-Chloro-phényl)-3,6-dihydro-[1,3,4]thiadiazin-2-one

Un mélange de 2-bromo-1-(4-chloro-phényl)-éthanone (90,71 mmoles) et d'acide hydrazinecarbothioïque O-éthyl ester (90,71 mmoles) dans l'acétonitrile (84 ml) est agité à température ambiante pendant 3 heures. Le solide formé est filtré, lavé avec de l'acétonitrile et de l'éther éthylique puis séché sous vide pour conduire au produit du titre, qui est engagé directement dans l'étape suivante.

### Stade B : 5-(4-Chloro-phényl)-3-(4-nitro-benzyl)-3,6-dihydro-[1,3,4]thiadiazin-2-one

A une solution du composé obtenu dans le Stade A (57,83 mmoles) dans l'acétonitrile (260 ml) est ajouté du 1-bromométhyl-4-nitro-benzène (63,61 mmoles) et du carbonate de potassium (231,32 mmoles). Le milieu réactionnel est agité à 80°C pendant 1 heure sous azote. La réaction est amenée à température ambiante et 100 ml d'eau sont ajoutés. On extrait à l'éther éthylique (3x350 ml) et les phase organiques sont combinées. La phase organique obtenue est séchée sur sulfate de sodium, filtrée et évaporée à sec. Le solide est trituré dans l'éther éthylique, filtré et séché sous vide pour conduire au produit du titre, qui est engagé directement dans l'étape suivante.

### Stade C : 3-(4-Amino-benzyl)-5-(4-chloro-phényl)-3,6-dihydro-[1,3,4]thiadiazin-2-one

Le composé obtenu dans le Stade B est réduit dans les conditions déjà décrites précédemment dans le stade B de l'Exemple 19 pour conduire au produit du titre, qui est engagé directement dans l'étape suivante.

### Stade D : 5-[5-(4-Chloro-phényl)-2-oxo-6H-[1,3,4]thiadiazin-3-ylméthyl]-3-méthylsulfanyl-1,3-dihydro-indol-2-one

A une solution du composé obtenu dans le Stade C (38,27 mmoles) dans un mélange CH₃CN (115 ml) et THF (115 ml) à -60°C est ajouté goutte à goutte l'éthyl(méthylsulfanyl)acétate (45,92 mmoles) sous atmosphère d'azote. ^{t}BuOCl (45,92 mmoles) est ajouté goutte à goutte au milieu réactionnel en 20 minutes en gardant la température entre -60°c et -55°C. Après une heure d'agitation à -60°C de la triéthylamine (51,66 mmoles) est ajoutée goutte à goutte. Le milieu réactionnel est amené à température ambiante. 240 ml de DCM puis une solution aqueuse de HCL 3M (340 ml) sont ajoutées. Après 12 heures d'agitation à température ambiante le milieu réactionnel est alcalinisé avec une solution aqueuse de soude à 20% (pH 10-11). Après extraction au DCM la phase organique est lavée avec une solution aqueuse de NaCl saturée. La phase organique est séchée sur sulfate de sodium, filtrée et évaporée à sec. Le produit obtenu est trituré dans l'éther éthylique filtré et séché sous vide pour conduire au produit du titre qui est engagé directement dans l'étape suivante.

### Stade E : 5-[5-(4-Chloro-phényl)-2-oxo-6H-[1,3,4]thiadiazin-3-ylméthyl]-1,3-dihydro-indol-2-one

A une solution du composé obtenu dans le Stade D (27,68 mmoles) dans l'acide acétique glacial (350 ml) est ajouté du Zn en poudre (18,09 mmoles). Le milieu réactionnel est agité une nuit à température ambiante puis filtré. Le filtrat est concentré puis amené à pH 8-9 avec une solution aqueuse saturée de bicarbonate de sodium et soniqué pendant 5 minutes. Le solide formé est filtré puis recristallisé dans l'éthanol pour conduire au produit titre.
*Spectrométrie de masse* (ES +, m/z) : 372,0561 (M+H)⁺
*Microanalyse élémentaire :*

| | *C* | *H* | *N* | *S* |
|---|---|---|---|---|
| *% théorique* | *58,14* | *3,79* | *11,30* | *8,62* |
| *% expérimental* | *57,38* | *3,54* | *11,12* | *8,18* |

### Stade F : 5-[5-(4-Chloro-phényl)-2-oxo-6H-[1,3,4]thiadiazin-3-ylméthyl]-3-(4-morpholin-4-ylméthyl-1H-pyrrol-2-ylméthylène)-1,3-dihydro-indol-2-one, chlorhydrate

Le produit du titre est obtenu suivant le protocole décrit dans le Stade E de l'Exemple 19 par condensation du composé obtenu dans le Stade E (1,34 mmole) et du composé obtenu dans la Préparation 1 (1,5 mmole) en présence de pipéridine dans l'éthanol au reflux. Le produit obtenu après filtration du milieu réactionnel est transformé en chlorhydrate tel que décrit dans le Stade E de l'Exemple 19 pour conduire au produit du titre. Mélange d'isomères Z / E (98 / 2)
*Spectrométrie de masse* (ES +, m/z) : 548,1517 (M+H)⁺ et 548,1540 (M+H)⁺
*Microanalyse élémentaire :*

| | *C* | *H* | *N* | *S* | *Cl⁻* |
|---|---|---|---|---|---|
| *% théorique* | *57,54* | *4,66* | *11,98* | *5,49* | *6,07* |
| *% expérimental* | *56,56* | *4,76* | *11,61* | *5,25* | *5,73* |

### Exemple 31 : 5-(2-Oxo-oxazolidin-3-ylméthyl)-3-(4-pyrrolidin-1-ylméthyl-1H-pyrrol-2-ylméthylène)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (1,08 mmole) et du composé obtenu dans la Préparation 11 (1,08 mmole) en présence de pipéridine dans l'éthanol au reflux. Après filtration, le solide obtenu est purifié sur gel de silice (SiO₂ ; AcOEt / MeOH 7/3) puis transformé en chlorhydrate tel que décrit dans le Stade E de l'Exemple 19 pour conduire au produit du titre. Mélange d'isomères Z / E (97 / 3)
*Spectrométrie de masse* (ES +, m/z) : 393,1899 (M+H)⁺ et 393,1896 (M+H)⁺
*Point de fusion : 204 °C*
*Microanalyse élémentaire :*

| | *C* | *H* | *N* | *Cl⁻* |
|---|---|---|---|---|
| *% théorique* | *61,61* | *5,87* | *13,06* | *8,27* |
| *% expérimental* | *61,33* | *5,83* | *13,01* | *8,61* |

### Exemple 32: 3-[4-((3aR,6aS)-Hexahydro-cyclopenta[c]pyrrol-2-(1H)-ylméthyl)-1H-pyrrol-2-ylméthylène]-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (1,08 mmole) et du composé obtenu dans la Préparation 12 (1,13 mmole) en présence de pipéridine dans l'éthanol au reflux. Après filtration, le solide obtenu est transformé en chlorhydrate tel que décrit dans le Stade E de l'Exemple 19 pour conduire au produit du titre. Mélange d'isomères Z / E (96 / 4)
*Spectrométrie de masse* (ES +, m/z) : 433,2212 (M+H)⁺ et 433,2217 (M+H)⁺
*Point de fusion :* 237 °C
*Microanalyse élémentaire :*

| | *C* | *H* | *N* | *Cl⁻* |
|---|---|---|---|---|
| *% théorique* | *64,03* | *6,23* | *11,95* | *7,56* |
| *% expérimental* | *63,57* | *6,11* | *11,92* | *7,42* |

### Exemple 33: 5-(2-Oxo-oxazolidin-3-ylméthyl)-3-(4-pipéridin-1-ylméthyl-1H-pyrro1-2-ylméthylène)-1,3-dihydro-indol-2-one, chlorhydrate

Le produit du titre est obtenu suivant le protocole dans le Stade E de l'Exemple 19 par condensation de la 5-(2-oxo-oxazolidin-3-ylméthyl)-1,3-dihydro-indol-2-one (1,08 mmole) et du composé obtenu dans la Préparation 13 (1,13 mmole) en présence de pipéridine dans l'éthanol au reflux. Après filtration, le solide obtenu est transformé en chlorhydrate tel que décrit dans le Stade E de l'Exemple 19 pour conduire au produit du titre. Mélange d'isomères Z / E (97 / 3)
*Spectrométrie de masse* (ES +, m/z) : 407,2047 (M+H)⁺ et 407,2053 (M+H)⁺
*Point de fusion :* 256 *°C*
*Microanalyse élémentaire :*

| | *C* | *H* | *N* | *Cl⁻* |
|---|---|---|---|---|
| *% théorique* | *62,37* | *6,14* | *12,65* | *8,00* |
| *% expérimental* | *62,36* | *6,04* | *12,78* | *7,99* |

### Exemple 34 : 3-(4-Diéthylaminométhyl-1H-pyrrol-2-ylméthylène)-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (1,08 mmole) et du composé obtenu dans la Préparation 14 (1,08 mmole) en présence de pipéridine dans l'éthanol au reflux. Après filtration, le solide obtenu est purifié sur gel de silice (SiO₂; Gradient AcOEt à AcOEt / MeOH 7/3) puis transformé en chlorhydrate tel que décrit dans le Stade E de l'Exemple 19 pour conduire au produit du titre. Mélange d'isomères Z / E (93 / 7)
*Spectrométrie de masse* (ES +, m/z) : 395,2066 (M+H)⁺ et 395,2082 (M+H)⁺
*Point de fusion : 192 °C*
*Microanalyse élémentaire :*

| | *C* | *H* | *N* | *Cl⁻* |
|---|---|---|---|---|
| *% théorique* | *61,61* | *5,87* | *13,06* | *8,27* |
| *% expérimental* | *61,33* | *5,83* | *13,01* | *8,61* |

### Exemple 35 : 5-[5-(4-Chloro-phényl)-2-oxo-6H-[1,3,4]thiadiazin-3-ylméthyl]-3-[1-(4-morpholin-4-ylméthyl-1H-pyrrol-2-yl)-éthylidène]-1,3-dihydro-indol-2-one, chlorhydrate

Un mélange du composé obtenu dans le Stade E de l'Exemple 30 (1 mmole), du composé obtenu dans la Préparation 2 (1,5 mmole) et NH₄OAc (4,5 mmoles) dans l'éthanol est chauffé sous micro-ondes à 110°C pendant 20 minutes. Après filtration, le solide obtenu est purifié sur gel de silice (SiO₂ ; Gradient AcOEt à AcOEt / MeOH 9/1) puis transformé en chlorhydrate tel que décrit dans le Stade E de l'Exemple 19 pour conduire au produit du titre. Mélange d'isomères Z / E (98 / 2)
*Spectrométrie de masse* (ES +, m/z) : 562,1647 (M+H)⁺ et 562,1666 (M+H)⁺
*Microanalyse élémentaire :*

| | *C* | *H* | *N* | *S* |
|---|---|---|---|---|
| *% théorique* | *57,76* | *5,47* | *10,86* | *4,97* |
| *% expérimental* | *57,65* | *5,56* | *10,86* | *4,88* |

### Exemple 36 : 3-[4-((1R,5S)-3-Aza-bicyclo[3.1.0]hex-3-ylméthyl)-3,5-diméthyl-1H-pyrrol-2-ylméthylène]-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (0,63 mmole) et du composé obtenu dans la Préparation 3 (0,69 mmole) en présence de pipéridine dans l'éthanol à 110°C sous activation micro-ondes. Après filtration, le solide obtenu est purifié sur gel de silice (SiO₂ ; Gradient AcOEt / MeOH) puis transformé en chlorhydrate tel que décrit dans le Stade E de l'Exemple 19 pour conduire au produit du titre. Mélange d'isomères Z / E (93 / 7)
*Spectrométrie de masse* (ES +, m/z) : 431,2070 (M+H)⁺ et 431,2074 (M+H)⁺
*Microanalyse élémentaire :*

| | *C* | *H* | *N* | *Cl⁻* |
|---|---|---|---|---|
| *% théorique* | *64,03* | *6,23* | *11,95* | *7,56* |
| *% expérimental* | *64, 21* | *6, 27* | *12,18* | *7,82* |

### Exemple 37 : (1R,5S)-3-(2-Oxo-3-(4-pipéridin-1-ylméthyl-1H-pyrrol-2-ylméthylène)-2,3-dihydro-1H-indol-5-ylméthyl]-3-aza-bicyclo[3.1.0]hexane-2,4-dione, chlorhydrate

Le produit du titre est obtenu suivant le protocole décrit dans le Stade 5 de l'Exemple 19 par condensation de la (1R,5S)-3-(2-oxo-2,3-dihydro-1*H*-indol-5-ylméthyl)-3-aza-bicyclo[3.1.0]hexane-2,4-dione (0,62 mmole) et du composé obtenu dans la Préparation 13 (0,65 mmole) en présence de pipéridine dans l'éthanol au reflux. Le produit obtenu après filtration du milieu réactionnel est transformé en chlorhydrate tel que décrit dans le Stade E de l'Exemple 19 pour conduire au produit du titre. Mélange d'isomères Z / E (98,5 / 1,5)
*Spectrométrie de masse* (ES +, m/z) : 431,2056 (M+H)⁺ et 431,2077 (M+H)⁺
*Point de fusion : 198°C*
*Microanalyse élémentaire :*

| | *C* | *H* | *N* | *Cl⁻* |
|---|---|---|---|---|
| *% théorique* | *64,30* | *5,83* | *12,00* | *7,59* |
| *% expérimental* | *63,52* | *5,80* | *12,16* | *8,18* |

### Exemple 38 : 3-[4-((1R,5S)-3-Aza-bicyclo[3.1.0]hex-3-ylméthyl)-1H-pyrrol-2-yl méthylène]-5-(2-oxo-oxazolidin-3-ylméthyl)-1,3-dihydro-indol-2-one, chlorhydrate

Le produit titre est obtenu suivant le protocole décrit dans le Stade E de l'Exemple 19 par condensation de la 3-(5-(2-oxo-oxazolidin-3-ylméthyl)-1,3-dihydro-indol-2-one (1,5 mmole) et du composé obtenu dans la Préparation 4 (1,9 mmole) en présence de pipéridine au reflux de l'éthanol pendant une nuit. Le produit obtenu après filtration du milieu réactionnel est transformé en chlorhydrate tel que décrit dans le Stade E de l'Exemple 19 pour conduire au produit du titre. Mélange d'isomères Z / E (94,5 / 5,5)
*Spectrométrie de masse* (ES +, m/z) : 405,1923 (M+H)⁺ et 405,1926 (M+H)⁺
*Point de fusion : 179°C*
*Microanalyse élémentaire :*

| | *C* | *H* | *N* | *Cl⁻* |
|---|---|---|---|---|
| *% théorique* | *62,65* | *5,71* | *12,71* | *8,04* |
| *% expérimental* | *62,45* | *5,73* | *12,88* | *8,75* |

### Exemple 39 : 3-[4-((3aR,6aS)-Hexahydro-cyclopenta[c]pyrrot-2(1H)-ytméthyl)-3,5-diméthyl-1H-pyrrol-2-ylméthylène]-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (1,72 mmole) et du composé obtenu dans la Préparation 15 (2,06 mmoles) en présence de pipéridine dans l'éthanol au reflux. Après filtration, le solide obtenu est transformé en chlorhydrate tel que décrit dans le Stade E de l'Exemple 19 pour conduire au produit du titre. Mélange d'isomères Z / E (96,5 / 3,5)
*Spectrométrie de masse* (ES +, m/z) : 461,2549 (M+H)⁺ et 461,2566 (M+H)⁺
*Point de fusion* 204°C
*Microanalyse élémentaire :*

| | *C* | *H* | *N* | *Cl⁻* |
|---|---|---|---|---|
| *% théorique* | *65,25* | *6,69* | *11,27* | *7,13* |
| *% expérimental* | *64,82* | *6,32* | *11,21* | *7, 24* |

### Exemple 40 : 3-(4-Morpholin-4-ylméthyl-1H-pyrrol-2-ylméthylène)-5-(2-oxo-pipéridin-1-ylméthyl)-1,3-dihydro-indol-2-one, chlorhydrate

### Stade A : 5-(2-Oxo-pipéridin-1-ylméthyl)-1,3-dihydro-indol-2-one

Le produit du titre est obtenu selon les Stades A à D de l'Exemple 19 en utilisant la pipéridin-2-one dans le stade A.
*Spectrométrie de masse* (ES +, m/z) : 245,1275 (M+H)⁺

### Stade B : 3-(4-Morpholin-4-ylméthyl-1H-pyrrol-2-ylméthylène)-5-(2-oxo-pipéridin-1-ylméthyl)-1,3-dihydro-indol-2-one, chlorhydrate

Le produit du titre est obtenu suivant le protocole décrit dans le Stade E de l'Exemple 19 par condensation du composé obtenu dans le Stade A (1,3 mmole) et du composé obtenu dans la Préparation 1 (1,3 mmoles) en présence de pipéridine dans l'éthanol au reflux. Après filtration, le solide obtenu est transformé en chlorhydrate tel que décrit dans le Stade E de l'Exemple 19 pour conduire au produit du titre. Mélange d'isomères Z / E (98,5 / 1,5)
*Spectrométrie de masse* (ES +, m/z) : 421,2222 (M+H)⁺ et 421,2245 (M+H)⁺
*Microanalyse élémentaire :*

| | *C* | *H* | *N* | *Cl⁻* |
|---|---|---|---|---|
| *% théorique* | *63, 08* | *6,40* | *12,26* | *7,76* |
| *% expérimental* | *63,15* | *6,25* | *12,12* | *7,87* |

### Exemple 41 : 3-{[(4-Morpholinylméthyl)-1H-pyrrol-2-yl]méthylène}-5-[(1,1-dioxido-2-isothiazolidinyl)methyl]-1,3-dihydro-2H-indol-2-one, chlorhydrate

### Stade A : 5-[(1,1-Dioxido-2-isothiazolidinyl)méthyl]-1,3-dihydro-2H-indol-2-one

Le produit du titre est obtenu suivant le protocole décrit dans les Stades A à D de l'Exemple 19 en utilisant l'isothiazolidine-l,l-dioxide dans le Stade A.
*Spectrométrie de masse* (ES +, m/z) : 267.0796 (M+H)⁺

### Stade B : 3-{[(4-Morpholinylméthyl)-1H-pyrrol-2-yl]méthylène}-5-[(1,1-dioxido-2-isothiazolidinyl)methyl]-1,3-dihydro-2H-indol-2-one, chlorhydrate

Le produit titre est obtenu suivant le protocole décrit dans le Stade E de l'Exemple 19 par condensation du composé obtenu dans le Stade A (1,32 mmole) et du composé obtenu dans la Préparation 1 (1,65 mmoles) en présence de pipéridine dans l'éthanol au reflux. Le produit obtenu après évaporation à sec du milieu réactionnel est purifié sur gel de silice (SiO₂; Gradient AcOEt / MeOH), puis transformé en chlorhydrate tel que décrit dans le Stade E de l'Exemple 19 pour conduire au produit du titre. Mélange d'isomères Z / E (99,5 / 0,5)
*Spectrométrie de masse* (ES +, m/z) : 443,1746 (M+H)⁺ et 443,1758 (M+H)⁺
*Microanalyse élémentaire :*

| | *C* | *H* | *N* | S | *Cl⁻* |
|---|---|---|---|---|---|
| *% théorique* | *55,17* | *5,68* | *11,70* | *6,69* | *7,40* |
| *% expérimental* | *54,42* | *5,64* | *11,58* | *6,55* | *7,50* |

### Exemple 42: 3-(4-Morpholin-4-ylméthyl-1H-pyrrol-2-ylméthylène)-5-(2-oxo-pyrrolidin-1-ylméthyl)-1,3-dihydro-indol-2-one, chlorhydrate

### Stade A : 5-(2-Oxo-pyrrolidin-1-ylméthyl)-1,3-dihydro-indol-2-one

Le produit du titre est obtenu suivant le protocole décrit dans les Stades A à D de l'Exemple 19 en utilisant la pyrrolidin-2-one dans le Stade A.
*Spectrométrie de masse* (ES +, m/z) : 231,1120 (M+H)⁺

### Stade B : 3-(4-Morpholin-4-ylméthyl-1H-pyrrol-2-ylméthylène)-5-(2-oxo-pyrrolidin-1-ylméthyl)-1,3-dihydro-indol-2-one, chlorhydrate

Le produit du titre est obtenu suivant le protocole décrit dans le Stade E de l'Exemple 19 par condensation du composé obtenu dans le Stade A (1,63 mmole) et du composé obtenu dans la Préparation 1 (1,95 mmoles) en présence de pipéridine dans l'éthanol au reflux. Le produit obtenu après filtration est purifié sur gel de silice (SiO₂ ; Gradient DCM / MeOH), puis transformé en chlorhydrate tel que décrit dans le Stade E de l'Exemple 19 pour conduire au produit du titre. Mélange d'isomères Z / E (99,5 / 0,5)
*Spectrométrie de masse* (ES +, m/z) : 407,2056 (M+H)⁺ et 407,2065 (M+H)⁺

### Exemple 43 : 3-(1-Méthyl-4-morpholin-4-ylméthyl-1H-pyrrol-2-ylméthylène)-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (1,31 mmole) et du composé obtenu dans la Préparation 6 (1,44 mmoles) en présence de pipéridine dans l'éthanol au reflux. Le produit obtenu après évaporation à sec du milieu réactionnel est purifié sur gel de silice (SiO₂ ; Gradient AcOEt / MeOH), puis transformé en chlorhydrate tel que décrit dans le Stade E de l'Exemple 19 pour conduire au produit du titre. Mélange d'isomères Z / E (33 / 66)
*Spectrométrie de masse* (ES +, m/z) : 423,2034 (M+H)⁺
*Microanalyse élémentaire :*

| | *C* | *H* | *N* | *Cl⁻* |
|---|---|---|---|---|
| *% théorique* | *60,19* | *5,93* | *12,21* | *7,72* |
| *% expérimental* | *59,54* | *5,73* | *12,09* | *8,10* |

### Exemple 44 : 3-[4-((1R,5S)-3-Aza-bicyclo[3.1.0]hex-3-ylméthyl)-1H-pyrrol-2-yl méthylène]-5-[5-(4-chloro-phényl)-2-oxo-6H-[1,3,4]oxadiazin-3-ylméthyl]-1,3-dihydro-indol-2-one, chlorhydrate

### Stade A : 1-(4-Chloro-phényl)-2-hydroxy-éthanone

Un mélange de 2-bromo-1-(4-chloro-phényl)-éthanone (100 mmoles) et de formate de potassium (130 mmoles) dans le MeOH (100 ml) est agité reflux pendant 3 heures. Le milieu réactionnel est jeté dans de la glace pilée. Le solide formé est filtré, lavé au MeOH froid puis séché sous vide pour conduire au produit du titre, qui est engagé directement dans l'étape suivante.

### Stade B : N'-[1-(4-Chloro-phényl)-2-hydroxy-éthylidène]-hydrazinecarboxylate d'éthyle

Un mélange du composé obtenu dans le Stade A (195 mmole) et d'hydrazinecarboxylate d'éthyle (224 mmole) dans l'éthanol est agité au reflux pendant 18 heures. Le milieu réactionnel est refroidi à 0°C et le précipité formé est filtré, lavé à l'éther éthylique et séché sous vide pour conduire au produit du titre, qui est engagé directement dans l'étape suivante.

### Stade C : 5-(4-Chloro-phényl)-3,6-dihydro-[1,3,4]oxadiazin-2-one

A une solution du composé obtenu dans le Stade B (100,5 mmoles) dans l'éthanol (800 ml) est ajouté de l'hydrure de Sodium (60% dans l'huile, 16 mmoles) en petites portions et à température ambiante. Après 2 heures de réaction, le milieu réactionnel est refroidi à 0°C et l'agitation est poursuivie pendant 30 minutes. Le précipité formé est filtré, lavé à l'éthanol froid puis séché sous vide pour conduire au produit du titre, qui est engagé directement dans l'étape suivante.
*Spectrométrie de masse* (ES +, m/z) : 211,0277 (M+H)⁺

### Stade D : 5-(4-Chloro-phényl)-3-(4-nitro-benzyl)-3,6-dihydro-[1,3,4]oxadiazin-2-one

A une solution du composé obtenu dans le Stade C (47,48 mmoles) dans de l'acétonitrile (260 ml) est ajouté du 1-bromométhyl-4-nitro-benzène (52,23 mmoles) et du carbonate de potassium (189,92 mmoles). Le milieu réactionnel est agité au reflux pendant 4 heures sous azote. La réaction est amenée à température ambiante puis diluée dans 500 ml de DCM et 100 ml d'eau. On extrait au DCM (2x200 ml), puis les phases organiques sont combinées. La phase organique obtenue est lavée avec une solution saturée de NaCl, puis séchée sur sulfate de sodium, filtrée et concentrée à environ 75 ml. Après formation d'un précipité en suspension, on ajoute de l'éther éthylique (150 ml) que l'on laisse sous agitation une nuit à température ambiante. Le précipité est filtré, lavé à l'éther éthylique et séché sous vide pour conduire au produit du titre, qui est engagé directement dans l'étape suivante.

### Stade E : 3-(4-Amino-benzyl)-5-(4-chloro-phényl)-3,6-dihydro-[1,3,4]oxadiazin-2-one

Une suspension du composé obtenu dans le Stade D (42,02 mmoles) dans un mélange d'EtOH (350 ml) et d'acide acétique (26 ml) est portée au reflux pendant 5 minutes. A cette solution est ajouté le Fer en poudre (309 mmoles) et du FeCl₃.6H₂O (2,1 mmoles). Le milieu réactionnel est agité au reflux pendant une nuit. Après évaporation, au résidu est ajouté de l'eau (250 ml) puis soniqué pendant 2 minutes. Le milieu est alcalinisé (pH 12) avec une solution aqueuse de soude 2M puis dilué à l'AcOEt (500 ml) puis filtré sur célite. Les phases sont séparées et la phase aqueuse extraite à AcOEt. Les phases organiques sont rassemblées. La phase organique obtenue est lavée à l'eau, puis une solution saturée de NaCl, séchée sur sulfate de sodium filtrée et évaporée à sec. Le résidu est trituré dans l'éther éthylique filtré et séché sous vide pour conduire au produit du titre sous la forme d'une poudre blanche qui est engagée directement dans l'étape suivante.

### Stade F : 5-[5-(4-Chloro-phényl)-2-oxo-6H-[1,3,4]oxadiazin-3-ylméthyl]-3-méthyl sulfanyl-1,3-dihydro-indol-2-one

A une solution du composé obtenu dans le Stade E (23,75 mmoles) dans le THF (200 ml) à -60°C est ajouté goutte à goutte l'éthyl(méthylsulfanyl)acétate (27,31 mmoles) sous atmosphère d'azote. ^{t}BuOCl (27,31 mmoles) est ajouté goutte à goutte au milieu réactionnel en 20 minutes en gardant la température entre -60°C et -55°C. Après 3 heures d'agitation à - 60°C de la triéthylamine (29,68 mmoles) est ajouté goutte à goutte. Le milieu réactionnel est amené à température ambiante, puis une solution aqueuse de HCl 3M (340 ml) est ajoutée et l'agitation est maintenue encore 2 heures. La phase organique est évaporée et la suspension aqueuse est extraite au DCM. Les phases organiques sont rassemblées puis lavées avec une solution HCl 2M puis avec une solution saturée de NaCl, séchée sur sulfate de sodium filtrée et évaporée à sec. Le résidu est trituré dans l'éther éthylique filtré et séché sous vide pour conduire au produit du titre, qui est engagé directement dans l'étape suivante.

### Stade G : 5-[5-(4-Chloro-phényl)-2-oxo-6H-[1,3,4]oxadiazin-3-ylméthyl]-1,3-dihydro-indol-2-one

A une solution du composé obtenu dans le Stade F (15,73 mmoles) dans l'acide acétique glacial (150 ml) est ajouté du Zn en poudre (157,3 mmoles). Le milieu réactionnel est agité pendant 6 heures puis filtré. Le filtrat est évaporé à sec puis repris dans l'eau (200 ml) et du DCM (400 ml). Les phases sont séparées et la phase aqueuse extraite au DCM, les phases organiques sont combinées. La phase organique obtenue est lavée à l'eau puis avec une solution saturée de NaCl, séchée sur sulfate de sodium filtrée et évaporée à sec. Le résidu obtenu est recristallisé dans l'éthanol pour conduire au produit du titre.
*Spectrométrie de masse* (ES +, m/z) : 356,0796 (M+H)⁺

### Stade H : 3-[4-((1R,5S)-3-Aza-bicyclo[3.1.0]hex-3-ylméthyl)-1H-pyrrol-2-yl méthylène]-5-[5-(4-chloro-phényl)-2-oxo-6H-[1,3,4]oxadiazin-3-ylméthyl]-1,3-dihydro-indol-2-one, chlorhydrate

Le produit titre est obtenu suivant le protocole décrit dans le Stade E de l'Exemple 19 par condensation du composé obtenu dans le Stade G (1,2 mmole) et du composé obtenu dans la Préparation 4 (1,38 mmoles) en présence de pipéridine dans l'éthanol au reflux. Le produit obtenu après évaporation à sec du milieu réactionnel est purifié sur gel de silice (SiO₂ ; Gradient DCM / MeOH), puis transformé en chlorhydrate tel que décrit dans le Stade E de l'Exemple 19 pour conduire au produit du titre. Mélange d'isomères Z / E (94,5 / 5,5)
*Spectrométrie de masse* (ES +, m/z) : 528,1808 (M+H)⁺ et 528,1803 (M+H)⁺
*Microanalyse élémentaire :*

| | *C* | *H* | *N* | *Cl⁻* |
|---|---|---|---|---|
| *% théorique* | *61,71* | *4,82* | *12,41* | *6,28* |
| *% expérimental* | *61,91* | *4,43* | *12,51* | *7,52* |

### Exemple 45 : 5-[5-(4-Chloro-phényl)-2-oxo-6H-[1,3,4]oxadiazin-3-ylméthyl]-3-(4-morpholin-4-ylméthyl-1H-pyrrol-2-ylméthylène)-1,3-dihydro-indol-2-one, chlorhydrate

Le produit du titre est obtenu suivant le protocole décrit dans le Stade E de l'Exemple 19 par condensation de la 5-[5-(4-chloro-phényl)-2-oxo-6*H*-[1,3,4]oxadiazin-3-ylméthyl]-1,3-dihydro-indol-2-one (1,2 mmole) et du composé obtenu dans la Préparation 1 (1,38 mmoles) en présence de pipéridine dans l'éthanol au reflux. Le produit obtenu après évaporation à sec du milieu réactionnel est purifié sur gel de silice (SiO₂ ; Gradient DCM / MeOH), puis transformé en chlorhydrate tel que décrit dans le Stade E de l'Exemple 19 pour conduire au produit du titre. Mélange d'isomères Z / E (98 / 2)
*Spectrométrie de masse* (ES +, m/z) : 532,1721 (M+H)⁺ et 532,1749 (M+H)⁺
*Microanalyse élémentaire :*

| | *C* | *H* | *N* | *Cl⁻* |
|---|---|---|---|---|
| *% théorique* | *59,16* | *4,79* | *12,32* | *6,24* |
| *% expérimental* | *58,71* | *4,73* | *12,33* | *6,37* |

### Exemple 46: 3-(5-Morpholin-4-ylméthyl-1H-pyrrol-3-ylméthylène)-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (0,86 mmole) et du composé obtenu dans la Préparation 16 (0,91 mmole) en présence de pipéridine dans l'éthanol au reflux. Le produit obtenu après évaporation à sec du milieu réactionnel est purifié sur gel de silice (SiO₂ ; Gradient DCM / MeOH), puis transformé en chlorhydrate tel que décrit dans le Stade E de l'Exemple 19 pour conduire au produit du titre. Mélange d'isomères Z / E (78 / 22)
*Spectrométrie de masse* (ES +, m/z) : 409,1883 (M+H)⁺ et 409,1865 (M+H)⁺

### Exemple 47: 3-[5-((1R,5S)-3-Aza-bicyclo[3.1.0]hex-3-ylméthyl)-1H-pyrrol-3-yl méthylène]-5-(2-oxo-oxazolidin-3-ylméthyl)-1,3-dihydro-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one (0,95 mmole) et du composé obtenu dans la Préparation 17 (0,95 mmole) en présence de pipéridine dans l'éthanol au reflux. Le produit obtenu après évaporation à sec du milieu réactionnel est purifié sur gel de silice (SiO₂ ;Gradient DCM / MeOH), puis transformé en chlorhydrate tel que décrit dans le Stade E de l'Exemple 19 pour conduire au produit du titre. Mélange d'isomères Z / E (82 / 18)
*Spectrométrie de masse* (ES +, m/z) : 405,1931 (M+H)⁺ et 405,1931 (M+H)⁺

### Exemple 48: 3-(4-Morpholin-4-ylméthyl-1H-pyrrol-2-ylméthylène)-5-[2-(2-oxo-oxa zolidin-3-yl)-éthyl]-1,3-dihydro-indol-2-one, chlorhydrate

### Stade A : 2-[2-(4-Nitro-phényl)-éthylamino]-éthanol

Un mélange de 1-(2-bromo-éthyl)-4-nitro-benzène (86,9 mmoles) est de 2-amino-éthanol (869 mmoles) est chauffé à 150°C sous activation micro-ondes pendant 10 minutes. Le milieu réactionnel est évaporé à sec et le résidu obtenu purifié sur gel de silice (SiO₂ ; Gradient DCM / MeOH) pour conduire au produit du titre, qui est engagé directement dans l'étape suivante.

### Stade B : 3-[2-(4-Nitro-phényl)-éthyl]-oxazolidin-2-one

A une solution du composé obtenu dans le Stade A (44,5 mmoles) et de DMAP (44,5 mmoles) dans le DCM (250 ml) est ajouté du CDI (89 mmoles). Après 4 heures d'agitation à température ambiante le milieu réactionnel est dilué avec 300 ml de DCM puis lavé avec une solution de HCl 2M (5x200 ml). La phase organique est séchée sur sulfate de magnésium, filtrée et évaporée à sec pour conduire au produit du titre sous la forme d'un solide orange, qui est engagé directement dans l'étape suivante.

### Stade C : 3-[2-(4-Amino-phényl)-éthyl]-oxazolidin-2-one

A une solution du composé obtenu dans le Stade B (16,6 mmoles) dans le MeOH (100 ml) est ajouté du Pd/C à 10 % (500 mg). Le milieu réactionnel et agité à 50°C sous 4,5 bars d'hydrogène pendant 4 heures. Le catalyseur est filtré puis lavé au MeOH. Le filtrat est évaporé à sec et le produit obtenu trituré dans 10 ml de MeOH. Le solide obtenu est filtré et séché sous vide à 40°C pendant une nuit pour conduire au produit du titre sous la forme d'un solide blanc, qui est engagé directement dans l'étape suivante.

### Stade D : 2-Hydroxyimino-N-{4-[2-(2-oxo-oxazolidin-3-yl)éthyl]phényl}acétamide

Une solution d'hydrate de trichloroacétaldéhyde (32,5 mmoles) dans l'eau (100 ml) est ajouté du sulfate de sodium (78,3 g). A cette suspension est ajoutée une solution de 3-[2-(4-amino-phényl)éthyl]oxazolidin-2-one dans l'eau (20 ml) et une solution de HCl concentré (2,83 ml). Puis une solution dans l'eau (50 ml) de chlorhydrate d'hydroxylamine (95 mmoles) est ajoutée. Le milieu réactionnel est porté au reflux pendant deux heures. Le milieu est alors refroidi à température ambiante. Le solide formé est alors filtré lavé à l'eau et séché sous vide en présence de P₂O₅ et à 40°C pendant une nuit pour conduire au produit du titre, qui est engagé directement dans l'étape suivante.

### Stade E : 5-[2-(2-Oxo-oxazolidin-3-yl)-éthyl]-1H-indole-2,3-dione

Du H₂SO₄ concentré (20 ml) est chauffé à 50°C. Le composé obtenu dans le Stade D (25,24 mmoles) est ajouté lentement sous forme solide tout en maintenant la température entre 60 et 70°C. Puis le milieu réactionnel est agité pendant une heure à 80°C. Le milieu est amené à température ambiante puis jeté sur 200 g de glace pilée. Après 30 minutes le solide rouge formé est filtré et lavé intensivement à l'eau glacée puis séché sous vide en présence de P₂O₅ et à 40°C pendant une nuit pour conduire au produit du titre, qui est engagé directement dans l'étape suivante.

### Stade F : 5-[2-(2-Oxo-oxazolidin-3-yl)éthyl]-1,3-dihydro-indol-2-one

A une solution du composé obtenu dans le Stade E (7,68 mmoles) dans l'acide acétique (15 ml) est ajouté du TFA (7 ml) et du Pd/C à 10 % (400 mg). Le milieu réactionnel et agité à 50°C sous 4 bars d'hydrogène pendant une nuit. Le catalyseur est filtré sur un lit de célite puis lavé avec de l'acide acétique (20 ml). Le filtrat est évaporé à sec et l'acide acétique résiduel éliminé par entrainement azéotropique au toluène. Le solide obtenu est recristallisé dans un mélange EtOH / éther éthylique pour conduire au produit du titre après séchage sous vide.
*Spectrométrie de masse* (ES +, m/z) : 247,1078 (M+H)⁺

### Stade G : 3-(4-Morpholin-4-ylméthyl-1H-pyrrol-2-ylméthylène)-5-[2-(2-oxo-oxa zolidin-3-yl)-éthyl]-1,3-dihydro-indol-2-one, chlorhydrate

Le produit du titre est obtenu suivant le protocole décrit dans le Stade E de l'Exemple 19 par condensation du composé obtenu dans le Stade F (1,62 mmole) et du composé obtenu dans la Préparation 1 (1,86 mmole) en présence de pipéridine dans l'éthanol au reflux. Le produit obtenu après filtration est transformé en chlorhydrate tel que décrit dans le Stade E de l'Exemple 19 pour conduire au produit du titre. Isomère Z.
*Spectrométrie de masse* (ES +, m/z) : 423,2031 (M+H)⁺
*Microanalyse élémentaire :*

| | *C* | *H* | *N* | *Cl⁻* |
|---|---|---|---|---|
| *% théorique* | *60,19* | *5,93* | *12,21* | *7,72* |
| *% expérimental* | *60,21* | *5,77* | *12,09* | *8,17* |

### Exemple 49 : 3-[4-((1R,5S)-3-Aza-bicyclo[3.1.0]hex-3-ylméthyl)-1H-pyrrol-2-yl méthylène]-5-[2-(2-oxo-oxazolidin-3-yl)éthyl]-1,3-dihydro-indol-2-one, chlorhydrate

Le produit du titre est obtenu suivant le protocole décrit dans le Stade E de l'Exemple 19 par condensation de la 5-[2-(2-oxo-oxazolidin-3-yl)éthyl]-1,3-dihydro-indol-2-one (1 mmole) et du composé obtenu dans la Préparation 4 (1,15 mmole) en présence de pipéridine dans l'éthanol au reflux. Le produit obtenu après filtration est transformé en chlorhydrate tel que décrit dans le Stade E de l'Exemple 19 pour conduire au produit du titre. Isomère Z.
*Spectrométrie de masse* (ES +, m/z) : 419,2088 (M+H)⁺
*Microanalyse élémentaire :*

| | *C* | *H* | *N* | *Cl⁻* |
|---|---|---|---|---|
| *% théorique* | *63,36* | *5,98* | *12,32* | *7,79* |
| *% expérimental* | *63,40* | *5,78* | *12,22* | *8,16* |

### Exemple 50 : 3-[(6-Fluoro-3-{[4-(4-morpholinylméthyl)-1H-pyrrol-2-yl]méthylène}-2-oxo-2,3-dihydro-1H-indol-5-yl)méthyl]-1,3-thiazolidine-2,4-dione

### Stade A : 5-(2-Chloroacétyl)-6-fluoroindolin-2-one

20 mL de DCM sont ajoutés goutte à goutte sous azote à du chlorure d'aluminium (31,1 mmol) à 0-5°C. La 6-fluoroindolin-2-one (5,18 mmol) et le chlorure d'acétyle (6,73 mmol) sont ajoutés au milieu. La réaction est chauffée à 50°C pendant une nuit. Le milieu réactionnel est refroidi à température ambiante, puis jeté dans un bain de glace, et la suspension obtenue est filtrée puis lavée à l'eau froide pour conduire au produit du titre.

### Stade B : 6-Fluoro-2-oxoindoline-5-carboxylate de méthyle

Une solution du composé obtenu dans le Stade A (3,24 mmoles) dans la pyridine (99 mmol) est agitée à 90°C pendant 2 heures. Le milieu réactionnel est refroidi à température ambiante puis filtré. Le solide obtenu est lavé à l'éthanol et séché sous vide à 50°C. Le composé est dissout dans du MeOH (80 mL) et 0,434 mmol de carbonate de potassium sont ajoutées. Le milieu est agité à 80°C pendant 5 heures. Après refroidissement à température ambiante, une solution HCl/dioxane 4M est ajoutée (pH 3) et le milieu est évaporé jusqu'à mise à sec. Le résidu obtenu est trituré avec de l'eau, filtré, lavé à l'eau puis séché sous vide à 50°C pour conduire au produit du titre.

### Stade C : 6-Fluoro-5-(hydroxyméthyl)indolin-2-one

Une solution du composé obtenu dans le Stade B (2,68 mmoles) dans du THF anhydre (20 mL) est maintenue sous azote en présence de 0,3 mL d'EtOH. La solution est refroidie à 0°C, puis 5,28 mmol de LiBH₄ sont ajoutées. Le milieu réactionnel est agité pendant 2 heures à température ambiante puis une seconde portion de LiBH₄ est ajoutée (6,20 mmol) et l'agitation est maintenue 3 heures supplémentaires. De l'EtOH (0,3 mL) et une troisième portion de LiBH₄ (9,14 mmol) sont ajoutés et la réaction est agitée pendant une nuit. Le milieu réactionnel est quenché avec 10 mL d'eau puis une solution aqueuse saturée en NH₄Cl (30 mL) est ajoutée. On extrait avec EtOAc, et les phases organiques sont séchées sur Na₂SO₄, filtrées et évaporées sous vide. Le résidu obtenu est purifié par chromatographie flash (DCM/MeOH 10:1) pour conduire au produit du titre.

### Stade D : 6-Fluoro-5-(hydroxyméthyl)-3-{[4-(4-morpholinylméthyl)-1H-pyrrol-2-yl]méthylène}-1,3-dihydro-2H-indol-2-one

La morpholine (0,662 mmol) et du composé obtenu dans la Préparation 1 (0,696 mmol) sont ajoutés au composé obtenu dans le Stade C (10,662 mmol), dissout dans EtOH (3 ml), et le milieu est porté au reflux pendant 2 heures. Le milieu est refroidi avec un bain de glace et le précipité solide formé est filtré, lavé avec EtOH froid, puis remis en suspension dans du toluene et évaporé sous vide (3 fois). Le résidu obtenu est séché sous vide à 40°C pendant 4 heures pour conduire au produit du titre.

### Stade E : 3-[(6-Fluoro-3-{[4-(4-morpholinylméthyl)-1H-pyrrol-2-yl]méthylène}-2-oxo-2,3-dihydro-1H-indol-5-yl)méthyl]-1,3-thiazolidine-2,4-dione

La thiazolidine-2,4-dione (0,350 mmol) et de la triphénylphosphine sur résine (1,66 mmol/g, 0,415 mmol) sont agitées 10 min. à température ambiante dans du THF anhydre (9 ml) sous azote. Le mélange est refroidi dans un bain de glace et l'azodicarboxylate de di-*tert-*butyle (0,369 mmol) est ajouté en une fois. Le milieu est agité à 0°C pendant 10 min. puis le composé obtenu dans le Stade D (0,238 mmol) est ajouté en une fois. Le milieu est agité à 0°C pendant 1,5 heure, puis de la thiazolidine-2,4-dione (0,350 mmol), trois portions de triphénylphosphine sur résine (1,66 mmol/g, 0,166 mmol ; 0,415 mmol et 0,166 mmol) et trois portions d' azodicarboxylate de *tert-*butyle (0,369 mmol chaque portion) sont ajoutées sur une période de 48h. Du gel de Silice est ajouté et le milieu est évaporé sous vide. Le résidu obtenu est purifié par chromatographie flash (DCM/MeOH 100:0 à 95:5) pour conduire à un solide jaune foncé qui, après trituration avec CH₃CN, conduit au produit du titre sous la forme d'un solide jaune.
*Point de fusion : 224-226°C*

### Exemple 51 : 3-[(7-Fluoro-3-{[4-(4-morpholinylméthyl)-1H-pyrrol-2-yl]méthylène}-2-oxo-2,3-dihydro-1H-indol-5-yl)méthyl]-1,3-thiazolidine-2,4-dione

On procède comme dans les Stades A à E de l'Exemple 50 en remplaçant dans le Stade A la 6-fluoroindolin-2-one par la 7-fluoroindolin-2-one. Le produit du titre est obtenu sous la forme d'un solide jaune.
*Point de fusion : 246-248°C*

### Exemple 52 : 3-[(4-Fluoro-3-{[4-(4-morpholinylméthyl)-1H-pyrrol-2-yl]méthylène}-2-oxo-2,3-dihydro-1H-indol-5-yl)méthyl]-1,3-thiazolidine-2,4-dione

On procède comme dans les Stades A à E de l'Exemple 50 en remplaçant dans le Stade A la 6-fluoroindolin-2-one par la 4-fluoroindolin-2-one. Le produit du titre est obtenu sous la forme d'un solide jaune.
*Point de fusion : 248-250°C*

### Exemple 53 : 3-({4-[2-(4-Morpholinyl)éthyl]-1H-pyrrol-2-yl}méthylène)-5-[(2-oxo-1,3-oxazolidin-3-yl)méthyl]-1,3-dihydro-2H-indol-2-one, chlorhydrate

Le produit du titre est obtenu suivant le protocole décrit dans le Stade E de l'Exemple 19 par condensation de la 5-(2-oxo-oxazolidin-3-ylméthyl)-1,3-dihydro-indol-2-one et du composé obtenu dans la Préparation 19.
*Point de fusion : 174 °C*
*Microanalyse élémentaire :*

| | *C* | *H* | *N* | *Cl⁻* |
|---|---|---|---|---|
| *% théorique* | *60,19* | *5,93* | *12,21* | *7,72* |
| *% expérimental* | *59,47* | *5,98* | *11,62* | *7,16* |

### Exemple 54 : 3-[(3-{[5-(Morpholin-4-ylméthyl)-1H-pyrrol-3-yl]méthylène}-2-oxo-2,3-dihydro-1H-indol-5-yl)méthyl]-1,3-thiazolidine-2,4-dione, chlorhydrate

### Stade A: 5-(Hydroxyméthyl)-3-{[5-(morpholin-4-ylméthyl)-1H-pyrrol-3-yl]méthylène}-1,3-dihydro-2H-indol-2-one

Le composé du titre est obtenu par condensation du composé obtenu dans le stade C de l'exemple 1 et du produit de la préparation 16 dans les conditions décrites dans le stade D exemple 1. Mélange d'isomères Z / E (46/54).
*Spectrométrie de masse* (ES +, m/z) : 340,1645 (M+H) et 340,1665 (M+H)

### Stade B: 3-[(3-{[5-(Morpholin-4-ylméthyl)-1H-pyrrol-3-yl]méthylène}-2-oxo-2,3-dihydro-1H-indol-5-yl)méthyl]-1,3-thiazolidine-2,4-dione, chlorhydrate

Le produit du titre est obtenu par condensation du produit obtenu dans le stade A et de la thiazolidine-2,4-dione dans les conditions décrites dans le stade E exemple 1. Mélange d'isomères Z/E (47 / 53)
*Spectrométrie de masse* (ES +, m/z) : 439,1447 (M+H)⁺ et 439,1425 (M+H)⁺
*Microanalyse élémentaire :*

| | *C* | *H* | *N* | *S* | *Cl* |
|---|---|---|---|---|---|
| *% théorique* | *55,63* | *4,88* | *11,80* | *6,75* | *7,46* |
| *% expérimental* | *56,17* | *4,89* | *12,62* | *5,92* | *7,84* |

### Exemple 55: 3-{[3-({5-[(1R,5S)-3-Azabicyclo[3.1.0]hex-3-ylméthyl]-1H-pyrrol-3-yl} méthylène)-2-oxo-2,3-dihydro-1H-indol-5-yl]méthyl}-1,3-thiazolidine-2,4-dione, chlorhydrate

### Stade A: 3-({5-[(1R,5S)-3-Azabicyclo[3.1.0]hex-3-ylméthyl]-1H-pyrrol-3-yl} méthylène)-5-(hydroxy-méthyl)-1,3-dihydro-2H-indol-2-one

Le composé du titre est obtenu par condensation du composé obtenu dans le stade C de l'Exemple 1 et du composé obtenu dans la préparation 17 dans les conditions décrites dans le stade D de l'Exemple 1. Mélange d'isomères Z / E (46/54)
*Spectrométrie de masse* (ES +, m/z) : 340,1645 (M+H) et 340,1665 (M+H)

### Stade B: 3-{[3-({5-[(1R,5S)-3-Azabicyclo[3.1.0]hex-3-ylméthyl]-1H-pyrrol-3-yl} méthylène)-2-oxo-2,3-dihydro-1H-indol-5-yl]méthyl}-1,3-thiazolidine-2,4-dione, chlorhydrate

Le produit du titre est obtenu par condensation du produit obtenu dans le stade A et de la thiazolidine-2,4-dione dans les conditions décrites dans le stade E de l'Exemple 1. Mélange d'isomères Z/E (60 / 40)
*Spectrométrie de masse* (ES +, m/z) : 435,1487 (M+H)⁺ et 435,1504 (M+H)⁺

### Exemple 56: 3-({4-[(1R,5S)-3-Azabicyclo[3.1.0]hex-3-ylméthyl]-1H-pyrrol-2-yl} méthylène)-6-fluoro-5-[(2-oxo-1,3-oxazolidin-3-yl)méthyl]-1,3-dihydro-2H-indol-2-one, chlorhydrate

### Stade A : 1-(Bromométhyl)-2-fluoro-4-nitrobenzène

A une solution de 2-fluoro-1-méthyl-4-nitrobenzène (0,152 mole) dans le toluène au reflux sont ajoutés goutte à goutte pendant 5 heures 5,1 ml de di-brome. Le mélange réactionnel est soumis à une distillation fractionnée. Le résidu obtenu est trituré dans le cyclohexane pour conduire au produit titre sous forme de cristaux. Le produit du titre est engagé directement dans l'étape suivante.

### Stade B : 3-(2-Fluoro-4-nitrobenzyl)-1,3-oxazolidin-2-one

A une suspension d'hydrure de sodium à 60% dans l'huile (4,48 mmoles) dans un mélange THF / DMF (2,7 ml / 0,45 ml) à 0°C sous atmosphère inerte est ajoutée goutte à goutte une solution de 1,3-oxazolidin-2-one (24,1 mmoles) dans un mélange THF / DMF (71 ml / 5,6 ml) en 30 minutes. Après 1 heure d'agitation est ajoutée une solution du composé obtenu dans le Stade A (26,5 mmoles) dans un mélange THF / DMF (56 ml / 11,2 ml) en 1,5 heure en maintenant la température inférieure à 10°C. L'agitation est maintenue pendant 1,5 heure. De l'eau (12 ml) est ajoutée lentement au milieu réactionnel puis le THF est éliminé sous pression réduite. Le milieu réactionnel est dilué avec une solution aqueuse saturée de NaCl puis extraite 3 fois avec 100 ml d'AcOEt. Les phases organiques sont combinées, lavées avec une solution aqueuse saturée de NaCl puis séchées avec du MgSO₄. Après filtration la phase organique est évaporée à sec. Le résidu est purifié sur gel de silice (SiO₂, éluant CH₂Cl₂) pour conduire au produit du titre qui est engagé directement dans l'étape suivante.

### Stade C : 3-(4-Amino-2-fluorobenzyl)-1,3-oxazolidin-2-one

A une solution du composé obtenu dans le Stade B (10,37 mmoles) dans l'éthanol (25 ml) sont ajoutés 122 microlitres d'acide acétique. La solution est portée au reflux et agitée pendant 10 minutes. Est ajouté du Fer en poudre (2,08 g) et 75 mg de FeCl₃.6H₂O. Après 2 heures d'agitation au reflux le milieu est amené à température ambiante puis concentré à sec. Au résidu obtenu, sont ajoutés 50 ml d'eau et le pH est alcalinisé avec de la soude. La solution est extraite 4 fois à l'AcOEt. Les phases organiques sont combinées et lavées avec une solution aqueuse saturée de NaCl puis séchées avec du MgSO₄. Après filtration la phase organique est évaporée à sec pour conduire au produit du titre qui est engagé directement dans l'étape suivante.

### Stade D : 6-Fluoro-3-(méthylsulfanyl)-5-[(2-oxo-1,3-oxazolidin-3-yl)méthyl]-1,3-dihydro-2H-indol-2-one et 4-fluoro-3-(méthylsulfanyl)-5-[(2-oxo-1,3-oxazolidin-3-yl)méthyl]-1,3-dihydro-2H-indol-2-one

Les produits du titre sont obtenus suivant le protocole décrit dans le stade C de l'Exemple 19 en utilisant comme produit de départ le 3-(4-amino-2-fluorobenzyl)-1,3-oxazolidin-2-one et séparés sur gel de silice (SiO₂ gradient heptane / AcOEt). Les produits du titre sont directement engagés dans l'étape suivante.

### Stade E : 6-Fluoro-5-[(2-oxo-1,3-oxazolidin-3-yl)méthyl]-1,3-dihydro-2H-indol-2-one

Le 6-fluoro-3-(méthylsulfanyl)-5-[(2-oxo-1,3-oxazolidin-3-yl)méthyl]-1,3-dihydro-2*H-*indol-2-one obtenu au stade D est traité comme décrit dans le stade D de l'Exemple 19 pour conduire au produit du titre.
*Spectrométrie de masse* (ES +, m/z) : 251,0834 (M+H)⁺

### Stade F : 3-({4-[(1R,5S)-3-Azabicyclo[3.1.0]hex-3-ylméthyl]-1H-pyrrol-2-yl} méthylène)-6-fluoro-5-[(2-oxo-1,3-oxazolidin-3-yl)méthyl]-1,3-dihydro-2H-indol-2-one, chlorhydrate

Le produit du titre est obtenu comme dans le stade E de l'exemple 19 en utilisant le composé obtenu au Stade E et le composé obtenu dans la Préparation 4. Mélange d'isomères Z / E (93/7)
*Spectrométrie de masse* (ES +, m/z) : 423,1817 (M+H)⁺ et 423,1838 (M+H)⁺
*Microanalyse élémentaire :*

| | *C* | *H* | *N* | *Cl⁻* |
|---|---|---|---|---|
| *% théorique* | *60,20* | *5,27* | *12,21* | *7,73* |
| *% expérimental* | *59,84* | *4,86* | *11,76* | *7,20* |

### Exemple 57: 6-Fluoro-3-{[4-(morpholin-4-ylméthyl)-1H-pyrrol-2-yl]méthylène}-5-[(2-oxo-1,3-oxazolidin-3-yl)méthyl]-1,3-dihydro-2H-indol-2-one, chlorhydrate

Le produit du titre est obtenu comme dans le stade E de l'Exemple 19 en utilisant le composé obtenu au Stade D de l'exemple 56 et le composé obtenu dans la Préparation 1. Mélange d'isomères Z / E (95/5)
*Spectrométrie de masse* (ES +, m/z) : 427,1784 (M+H)⁺ et 427,1795 (M+H)⁺
*Microanalyse élémentaire :*

| | *C* | *H* | *N* | *Cl⁻* |
|---|---|---|---|---|
| *% théorique* | *57,08* | *5,23* | *12,10* | *7,66* |
| *% expérimental* | *57,08* | *4,72* | *11,77* | *8,13* |

### Exemple 58: 4-Fluoro-3-{[4-(morpholin-4-ylméthyl)-1H-pyrrol-2-yl]méthylène}-5-[(2-oxo-1,3-oxazolidin-3-yl)méthyl]-1,3-dihydro-2H-indol-2-one, chlorhydrate

### Stade A : 4-Fluoro-5-[(2-oxo-1,3-oxazolidin-3-yl)méthyl]-1,3-dihydro-2H-indol-2-one

Le 4-fluoro-3-(méthylsulfanyl)-5-[(2-oxo-1,3-oxazolidin-3-yl)méthyl]-1,3-dihydro-2*H-*indol-2-one obtenu au stade D de l'exemple 56 est traité comme décrit dans le stade D de l'Exemple 19 pour conduire au produit du titre.
*Spectrométrie de masse* (ES +, m/z) : 251,0831 (M+H)⁺

### Stade B : 4-Fluoro-3-{[4-(morpholin-4-ylméthyl)-1H-pyrrol-2-yl]méthylène}-5-[(2-oxo-1,3-oxazolidin-3-yl)méthyl]-1,3-dihydro-2H-indol-2-one, chlorhydrate

Le produit du titre est obtenu comme dans le stade E de l'Exemple 19 en utilisant le composé obtenu au Stade A et le composé obtenu dans la Préparation 1. Mélange d'isomères Z / E (97,4/2,6)
*Spectrométrie de masse* (ES +, m/z) : 427,1781 (M+H)⁺ et 427,1785 (M+H)⁺
*Microanalyse élémentaire :*

| | *C* | *H* | *N* | *Cl⁻* |
|---|---|---|---|---|
| *% théorique* | *57,08* | *5,23* | *12,10* | *7,66* |
| *% expérimental* | *57,36* | *4,48* | *11,88* | *8,03* |

### Exemple 59: 3-({4-[(1R,5S)-3-Azabicyclo[3.1.0]hex-3-ylméthyl]-1H-pyrrol-2-yl} méthylène)-4-fluoro-5-[(2-oxo-1,3-oxazolidin-3-yl)méthyl]-1,3-dihydro-2H-indol-2-one, chlorhydrate

Le produit du titre est obtenu comme dans le stade E de l'Exemple 19 en utilisant le composé obtenu au Stade A et le composé obtenu dans la Préparation 4. Mélange d'isomères Z / E (98,5/1,5)
*Spectrométrie de masse* (ES +, m/z) : 423,1827 (M+H)⁺ et 423,1840 (M+H)⁺
*Microanalyse élémentaire :*

| | *C* | *H* | *N* | *Cl⁻* |
|---|---|---|---|---|
| *% théorique* | *60,20* | *5,27* | *12,21* | *7,73* |
| *% expérimental* | *59,61* | *5,05* | *11,73* | *7,32* |

### Exemple 60: 3-[(3-{[3-(Morpholin-4-ylméthyl)-1H-pyrrol-2-yl]méthylène}-2-oxo-2,3-dihydro-1H-indol-5-yl)méthyl]-1,3-thiazolidine-2,4-dione, chlorhydrate

### Stade A : 5-(Hydroxyméthyl)-3-{[3-(morpholin-4-ylméthyl)-1H-pyrrol-2-yl] méthylène}-1,3-dihydro-2H-indol-2-one

Le produit du titre est obtenu par condensation du produit obtenu dans le Stade C de l'Exemple 1 et du produit obtenu dans la Préparation 8 en utilisant les conditions décrites dans le Stade D de l'Exemple 1. Mélange d'isomères Z / E (96/4)
*Spectrométrie de masse* (ES +, m/z) : 340,1652 (M+H)⁺ et 340,1638 (M+H)⁺

### Stade B : 3-[(3-{[3-(Morpholin-4-ylméthyl)-1H-pyrrol-2-yl]méthylène}-2-oxo-2,3-dihydro-1H-indol-5-yl)méthyl]-1,3-thiazolidine-2,4-dione, chlorhydrate

Le produit du titre est obtenu comme décrit dans le stade E de l'Exemple 1 en partant du produit obtenu dans le Stade A. Mélange d'isomères Z / E (96/4)
*Spectrométrie de masse* (ES +, m/z) : 423,1827 (M-FH)⁺ et 423,1840 (M+H)⁺
*Microanalyse élémentaire :*

| | *C* | *H* | *N* | *Cl⁻* |
|---|---|---|---|---|
| *% théorique* | *55,63* | *4,88* | *11,80* | *7,46* |
| *% expérimental* | *56,06* | *4,59* | *12,03* | *7,05* |

### Exemple 61: 3-({5-[(3aR,6aS)-Hexahydrocyclopenta[c]pyrrot-2(1H)-ylméthyl]-1H-pyrrol-2-yl}méthylène)-5-[(2-oxo-1,3-oxazolidin-3-yl)méthyl]-1,3-dihydro-2H-indol-2-one, chlorhydrate

On procède comme dans le stade E de l'Exemple 19 en remplaçant le produit de la Préparation 1 par le produit de la Préparation 20. Mélange d'isomères Z / E (98/2)
*Spectrométrie de masse* (ES +, m/z) : 433,2227 (M+H)⁺ et 433,2234 (M+H)⁺

### Exemple 62: 3-{[3-({4-[(4-Méthoxypipéridin-1-yl)méthyl]-1H-pyrrol-2-yl}méthylène)-2-oxo-2,3-dihydro-1H-indol-5-yl]méthyl}-1,3-thiazolidine-2,4-dione

### Stade A : 5-(Hydroxyméthyl)-3-({4-[(4-méthoxypipéridin-1-yl)méthyl]-1H-pyrrol-2-yl} méthylène)-1,3-dihydro-2H-indol-2-one

On procède comme dans le Stade D de l'Exemple 1 en remplaçant le produit de la Préparation 1 par le produit de la Préparation 21. Le produit du titre est utilisé directement dans l'étape suivante.

### Stade B : 3-{[3-({4-[(4-Méthoxypipéridin-1-yl)méthyl]-1H-pyrrol-2-yl}méthylidène)-2-oxo-2,3-dihydro-1H-indol-5-yl]méthyl}-1,3-thiazolidine-2,4-dione

On procède comme dans le Stade E de l'Exemple 1 en remplaçant le produit du Stade D de l'Exemple 1 par le produit du Stade A. Dans ce cas le produit obtenu n'est pas transformé en chlorhydrate. Mélange d'isomères Z / E (92,5/7,5)
*Spectrométrie de masse* (ES +, m/z) : 467,1741 (M+H)⁺ et 467,1742 (M+H)⁺
*Microanalyse élémentaire :*

| | *C* | *H* | *N* | *S⁻* |
|---|---|---|---|---|
| *% théorique* | *61,79* | *5,62* | *12,01* | *6,87* |
| *% expérimental* | *61,05* | *5,60* | *11,64* | *6,69* |

### Exemple 63: 5-[(2-Oxo-1,3-oxazolidin-3-yl)méthyl]-3-({4-[2-(pyrrolidin-1-yl)éthyl]-1H-pyrrol-2-yl} méthylène)-1,3-dihydro-2H-indol-2-one

Le produit du titre est obtenu suivant le protocole décrit dans le Stade E de l'Exemple 19 par condensation du composé obtenu dans le stade D de l'Exemple 19 et du composé obtenu dans la Préparation 18.
*Spectrométrie de masse* (ES +, m/z) : 407,2088 (M+H)⁺

### Exemple 64: 3-{[4-(Morpholin-4-ylméthyl)-1H-pyrrol-3-yl]méthylène}-5-[(2-oxo-1,3-oxazolidin-3-yl)méthyl]-1,3-dihydro-2H-indol-2-one, chlorhydrate

Le produit du titre est obtenu suivant le protocole décrit dans le Stade E de l'Exemple 19 par condensation du composé obtenu dans le Stade D de l'Exemple 19 et du composé obtenu dans la Préparation 22. Mélange d'isomères Z / E (96,7/5,3)
*Spectrométrie de masse* (ES +, m/z) : 409,1871 (M+H)⁺
*Point de fusion : 210°C*
*Microanalyse élémentaire :*

| | *C* | *H* | *N* | *Cl⁻* |
|---|---|---|---|---|
| *% théorique* | *59,39* | *5,66* | *12,59* | *7,97* |
| *% expérimental* | *58,89* | *5,45* | *12,48* | *7,79* |

### Exemple 65: 3-[2-(3-{[4-(Morpholin-4-ylméthyl)-1H-pyrrol-2-yl]méthylène}-2-oxo-2,3-dihydro-1H-indol-5-yl)éthyl]-1,3-thiazolidine-2,4-dione, chlorhydrate

### Stade A : [3-(Méthylsulfanyl)-2-oxo-2,3-dihydro-1H-indol-5-yl]acétate d'éthyle

Le produit du titre est obtenu comme décrit dans le Stade A de l'Exemple 1 en partant du (4-aminophényl)acétate d'éthyle et le produit obtenu est utilisé directement dans l'étape suivante.

### Stade B : (2-Oxo-2,3-dihydro-1H-indol-5-yl)acétate d'éthyle

Le produit du titre est obtenu comme décrit dans le Stade B de l'Exemple 1. Le produit obtenu est utilisé directement dans l'étape suivante.

### Stade C : 5-(2-Hydroxyéthyl)-1,3-dihydro-2H-indol-2-one

A une solution de NaBH₄ (0,45 g) dans le THF (15 ml) est ajouté LiBr (1,03 g). Le milieu réactionnel est porté au reflux pendant 7 heures et le composé obtenu dans le Stade B est ajouté (1,3 g) suivi de B(OMe)₃ (0,067 ml). Après 18 heures de reflux le milieu est concentré sous pression réduite et le résidu obtenu est repris dans 20 ml de H₂SO₄ 3N. La phase aqueuse est saturée au NaCl et extraite à l'acétate d'éthyle. La phase organique est séchée sur sulfate de magnésium, filtrée et évaporée à sec. Le résidu est purifié sur gel de silice (SiO₂, éluant : AcOEt) pour conduire au produit du titre sous la forme d'un solide beige.
Le composé obtenu est directement utilisé dans la réaction suivante.

### Stade D : 5-(2-Hydroxyéthyl)-3-{[4-(morpholin-4-ylméthyl)-1H-pyrrol-2-yl] méthylène}-1,3-dihydro-2H-indol-2-one

Le produit du titre est obtenu comme dans le Stade D de l'Exemple 1 en utilisant le produit obtenu dans le Stade C et le composé de la Préparation 1. Le produit obtenu est directement utilisé dans la réaction suivante.

### Stade E : 3-[2-(3-{[4-(Morpholin-4-ylméthyl)-1H-pyrrol-2-yl]méthylène}-2-oxo-2,3-dihydro-1H-indol-5-yl)éthyl]-1,3-thiazolidine-2,4-dione, chlorhydrate

Le produit du titre est obtenu comme décrit dans le Stade E de l'Exemple 1 en partant du composé obtenu dans le Stade D.
*Spectrométrie de masse* (ES +, m/z) : 453,1612 (M+H)⁺
*Point de fusion : 289°C décomposition.*
*Microanalyse élémentaire :*

| | *C* | *H* | *N* | *S* | *Cl⁻* |
|---|---|---|---|---|---|
| *% théorique* | *56,49* | *5,15* | *11,46* | *6,56* | *7,25* |
| *% expérimental* | *55,74* | *5,02* | *11,04* | *6,13* | *6,96* |

### Exemple 66: 3-{[3-({4-[(2-Méthyl-4-morpholinyl)méthyl]-1H-pyrrol-2-yl}méthylène)-2-oxo-2,3-dihydro-1H-indol-5-yl]méthyl}-1,3-thiazolidine-2,4-dione, chlorhydrate

### Stade A : 5-(Hydroxyméthyl)-3-({4-[(2-méthyl-4-morpholinyl)méthyl]-1H-pyrrol-2-yl}méthylène)-1,3-dihydro-2H-indol-2-one

Le produit du titre est obtenu par condensation du composé obtenu dans le Stade C de l'Exemple 1 et du produit obtenu dans la Préparation 23 dans les conditions décrites dans le Stade D de l'Exemple 1.

### Stade B: 3-{[3-({4-[(2-Méthyl-4-morpholinyl)méthyl]-1H-pyrrol-2-yl}méthylène)-2-oxo-2,3-dihydro-1H-indol-5-yl]méthyl}-1,3-thiazolidine-2,4-dione, chlorhydrate

Le produit du titre est obtenu par condensation du produit obtenu dans le Stade A et de la thiazolidine-2,4-dione dans les conditions décrites dans le Stade E de l'Exemple 1. Mélange d'isomères Z/E (95/5).
*Spectrométrie de masse* (ES +, m/z) : 354,1807 (M+H)⁺ et 354,1803 (M+H)⁺
*Microanalyse élémentaire :*

| | *C* | *H* | *N* | *S* |
|---|---|---|---|---|
| *% théorique* | *61,05* | *5,35* | *12,38* | *7,09* |
| *% expérimental* | *60,54* | *5,37* | *12,13* | *7,20* |

### ETUDE PHARMACOLOGIQUE

Les composés de l'invention sont de puissants inhibiteurs de la migration de cellules cancéreuses, sans toxicité non-spécifique. A titre d'exemple sont reportés ci-dessous les résultats de l'étude sur les cellules A549, carcinome de poumon non à petites cellules.

### EXEMPLE A: Inhibition de la migration des cellules A549 : « scratch assay »

Des cellules de la lignée A549, carcinome humain du poumon non à petites cellules, sont cultivées dans un incubateur à 37°C en présence de 5% de CO₂, dans du milieu RPMI 1640 contenant 10% de sérum de veau foetal, 40 ng/ml de HGF (hepatocyte growth factor), 2 mM de glutamine, 50 unités/ml de pénicilline, 50 µg/ml de streptomycine et 10 mM de tampon Hepes, pH = 7,4.

Pour le test de migration, les cellules A549 sont réparties dans des plaques 96 puits (50.000 cellules par puits) et cultivées 24 heures pour obtenir des cellules à confluence. Une rayure d'un millimètre d'épaisseur est effectuée dans le tapis cellulaire, et les cellules sont exposées aux composés à tester pendant 38 heures. Les cultures cellulaires sont ensuite photographiées, et le remplissage de la rayure par la migration des cellules est estimé.

Les résultats montrent que les composés de l'invention sont de puissants inhibiteurs de la migration cellulaire. A titre d'exemple, le composé de l'Exemple 1 montre une inhibition totale de la migration à 30 nM.

### EXEMPLE B: Absence de toxicité non-spécifique

Pour montrer l'absence de toxicité non-spécifique, les cellules A549 sont réparties dans des plaques 96 puits et exposées aux composés à tester pendant 96 heures. La viabilité cellulaire est ensuite quantifiée par un essai colorimétrique, le Microculture Tetrazolium Assay (Cancer Res., 1987, 47, 939-942).

Les résultats montrent que les composés de l'invention ne sont pas toxiques sur les cellules A549 à des concentrations allant jusqu'a 100 µM.

Les composés de l'invention présentent donc une excellente activité anti-migratrice sans toxicité non spécifique.

### Exemple C : Inhibition de la croissance de la tumeur U87-MG

La tumeur U87-MG, glioblastome humain, a été greffée en localisation sous-cutanée sur la souris Nude BalbC femelle a raison de 10⁶ cellules. Les tumeurs ont été randomisées en groupes de huit souris lorsque le volume tumoral a atteint 200 mm³. Le traitement quotidien (6,25 , 12,5 , 25 et 50 mg/kg) a été administré par voie orale (véhicule = eau) sur une période de 17 jours à l'exception des week-ends. Les volumes tumoraux ont été mesurés à raison de deux fois par semaine au pied à coulisse. L'inhibition de la croissance constatée après administration des composés selon l'invention est statistiquement significative (ANOVA à deux facteurs, avec mesures répétées au cours du temps suivi d'un test de DUNETT) à toutes les doses testées entre le jour 21 et le jour 35 (date du sacrifice éthique des souris du groupe contrôle en conformité avec les règles éthiques). Ainsi, à titre d'exemple, le composé de l'Exemple 1 à jour 35, permet d'obtenir une inhibition de la croissance de 102%, 84% et 98% pour les doses de 6,25 mg/kg, 12,5 mg/kg et 25 mg/kg respectivement. Une régression de l'ordre de 70% est observée à la dose de 50 mg/kg.

### EXEMPLE D : Composition pharmaceutique : Comprimés

| | |
|---|---|
| 1000 comprimés dosés à 5 mg de 3-[3-{[4-(4-morpholinylméthyl)-1*H*-pyrrol-2-yl]méthylène}-2-oxo-2,3-dihydro-1*H*-indol-5-yl)méthyl]-1,3-thiazolidine-2,4-dione,chlorhydrate (Exemple 1) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 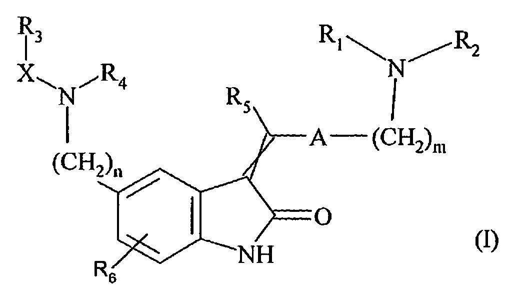 dans laquelle :
◆ m représente 1 ou 2,
◆ n représente 1 ou 2,
◆ A représente un groupement pyrrolyle non substitué ou substitué par 1 à 3 groupements alkyle (C₁-C₆) linéaire ou ramifié,
◆ X représente le groupement C(O), S(O) ou SO₂,
◆ R₁ et R₂, identiques ou différents, représentent chacun un groupement alkyle (C₁-C₆) linéaire ou ramifié,
ou R₁ et R₂ forment ensemble avec l'atome d'azote qui les porte un groupement hétérocyclique,
◆ R₃ et R₄ forment ensemble avec les atomes qui les portent un groupement hétérocyclique,
◆ R₅ représente un atome d'hydrogène ou un groupement alkyle (C₁-C₆) linéaire ou ramifié,
◆ R₆ représente un atome d'hydrogène ou un atome d'halogène,
étant entendu que :
- par "groupement hétérocyclique", on entend un groupement mono ou bicyclique pouvant contenir de 5 à 8 sommets, pouvant contenir de un à trois hétéroatomes choisis parmi azote, oxygène et soufre, et pouvant contenir une ou plusieurs insaturations, le groupement hétérocyclique ainsi défini pouvant être non substitué ou substitué par un ou plusieurs groupements choisis parmi alkyle (C₁-C₆) linéaire ou ramifié, alkényle (C₂-C₆) linéaire ou ramifié, oxo, hydroxy, alkoxy (C₁-C₆) linéaire ou ramifié, aryle, arylalkyle, et arylalkényle,
- par "aryle", on entend le groupement phényle non substitué ou substitué par un ou plusieurs groupements choisis parmi les atomes d'halogène et le groupement alkyle (C₁-C₆) linéaire ou ramifié,
- la notation 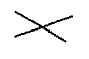 signifie que la double liaison est de configuration Z ou E,
leurs isomères optiques et géométriques, ainsi que leurs sels d'addition à un acide ou à une base pharmaceutiquement acceptable.

2. Composés de formule (I) selon la revendication 1 pour lesquels n représente 1, leurs isomères optiques et géométriques ainsi que leurs sels d'addition à un acide ou à une base pharmaceutiquement acceptable.

3. Composés de formule (I) selon la revendication 1 pour lesquels m représente 1, leurs isomères optiques et géométriques ainsi que leurs sels d'addition à un acide ou à une base pharmaceutiquement acceptable.

4. Composés de formule (I) selon la revendication 1 pour lesquels R₁ et R₂ représentent un groupement alkyle, leurs isomères optiques et géométriques ainsi que leurs sels d'addition à un acide ou à une base pharmaceutiquement acceptable.

5. Composés de formule (I) selon la revendication 1 pour lesquels R₁ et R₂ forment ensemble avec l'atome d'azote qui les porte un groupement monocyclique à 5 ou 6 chaînons, leurs isomères optiques et géométriques ainsi que leurs sels d'addition à un acide ou à une base pharmaceutiquement acceptable.

6. Composés de formule (I) selon la revendication 1 pour lesquels R₁ et R₂ forment ensemble avec l'atome d'azote qui les porte un groupement morpholinyle, leurs isomères optiques et géométriques ainsi que leurs sels d'addition à un acide ou à une base pharmaceutiquement acceptable.

7. Composés de formule (I) selon la revendication 1 pour lesquels R₃ et R₄ forment ensemble avec le groupement X et l'atome d'azote qui les porte un groupement thiazolidinedionyle, leurs isomères optiques et géométriques ainsi que leurs sels d'addition à un acide ou à une base pharmaceutiquement acceptable.

8. Composés de formule (I) selon la revendication 1 pour lesquels R₅ représente un atome d'hydrogène, leurs isomères optiques et géométriques ainsi que leurs sels d'addition à un acide ou à une base pharmaceutiquement acceptable.

9. Composés de formule (I) selon la revendication 1 pour lesquels R₆ représente un atome d'hydrogène, leurs isomères optiques et géométriques ainsi que leurs sels d'addition à un acide ou à une base pharmaceutiquement acceptable.

10. Composés de formule (I) selon la revendication 1 pour lesquels X représente un groupement C=O, leurs isomères optiques et géométriques ainsi que leurs sels d'addition à un acide ou à une base pharmaceutiquement acceptable.

11. Composés de formule (I) selon la revendication 1 pour lesquels A représente le groupement 1*H*-pyrrol-2,4-yle, leurs isomères optiques et géométriques ainsi que leurs sels d'addition à un acide ou à une base pharmaceutiquement acceptable.

12. Composés de formule (I) selon la revendication 1 pour lesquels R₁ et R₂ forment ensemble avec l'atome d'azote qui les porte le groupement morpholinyle, m et n représentent la valeur 1, R₅ et R₆ représentent un atome d'hydrogène et A représente le groupement 1*H*-pyrrol-2,4-yle, leurs isomères optiques et géométriques ainsi que leurs sels d'addition à un acide ou à une base pharmaceutiquement acceptable.

13. Composé de formule (I) selon la revendication 1 qui est le 3-[(3-{[4-(4-morpholinylméthyl)-1*H*-pyrrol-2-yl]méthylène}-2-oxo-2,3-dihydro-1*H*-indol-5-yl)méthyl]-1,3-thiazolidine-2,4-dione, ses isomères géométriques ainsi que ses sels d'addition à un acide ou à une base pharmaceutiquement acceptable.

14. Composé de formule (I) selon la revendication 1 qui est le 3-[((3Z)-3-{[4-(4-morpholinylméthyl)-1*H*-pyrrol-2-yl]méthylène}-2-oxo-2,3-dihydro-1*H*-indol-5-yl)méthyl]-1,3-thiazolidine-2,4-dione, ainsi que ses sels d'addition à un acide ou à une base pharmaceutiquement acceptable.

15. Procédé de préparation des composés de formule (I) selon la revendication 1 **caractérisé en ce que** l'on utilise comme produit de départ le composé de formule (II) : 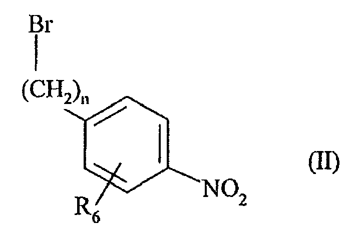 dans laquelle n et R₆ sont tels que défini dans la formule (I),
sur lequel on condense, en présence d'une base, le composé de formule (III) : 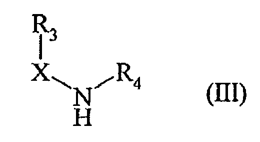 dans laquelle X, R₃ et R₄ sont tels que définis dans la formule (I),
pour conduire au composé de formule (IV) : 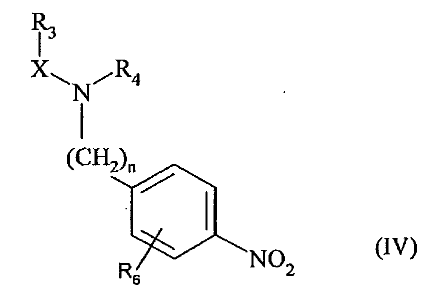 dans laquelle n, X, R₃, R₄ et R₆ sont tels que définis précédemment,
que l'on soumet à une hydrogénation chimique ou catalytique, pour conduire au composé de formule (V) : 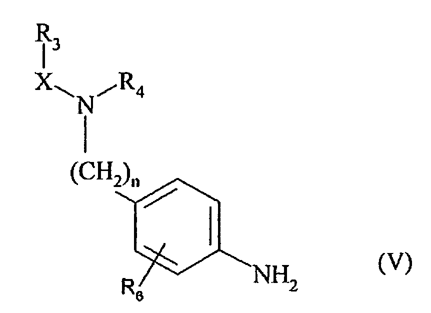 dans laquelle n, X, R₃, R₄ et R₆ sont tels que définis précédemment,
que l'on soumet à l'action de tBuOCl en présence de (méthylsulfanyl)acétate d'éthyle, suivi de l'action successive de triéthylamine et d'acide chlorhydrique pour conduire au composé de formule (VI) : 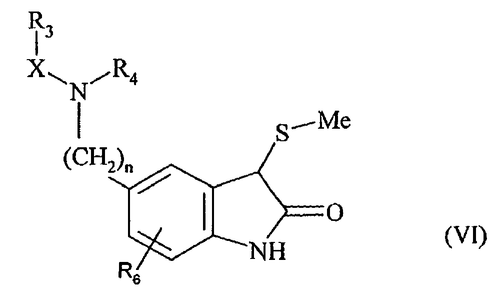 dans laquelle n, X, R₃, R₄ et R₆ sont tels que définis précédemment,
que l'on soumet à l'action de Zinc en poudre pour conduire au composé de formule (VII) : 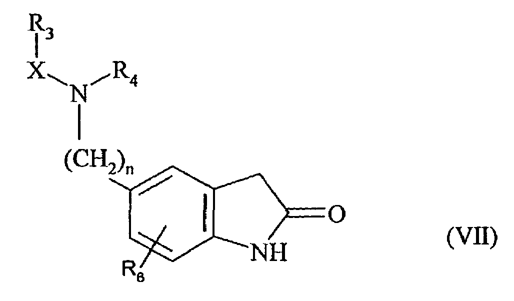 dans laquelle n, X, R₃, R₄ et R₆ sont tels que définis précédemment,
sur lequel on condense en présence de pipéridine le composé de formule (VIII) : 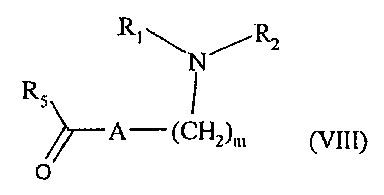 dans laquelle m, A, R₁, R₂, et R₅ sont tels que définis dans la formule (I),
pour conduire après traitement acide au composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

16. Procédé de préparation des composés de formule (I) selon la revendication 1, **caractérisé en ce que** l'on utilise comme produit de départ le composé de formule (IX) : 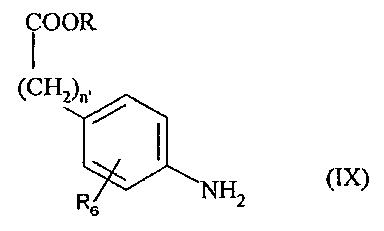 dans laquelle n' représente 0 ou 1, et R représente un groupement alkyle (C₁-C₆)linéaire ou ramifié, et R₆ est tel que défini dans la formule (I),
que l'on soumet à l'action de tBuOCl en présence de (méthylsulfanyl)acétate d'éthyle, suivi de l'action successive de triéthylamine et d'acide chlorhydrique, pour conduire au composé de formule (X) : 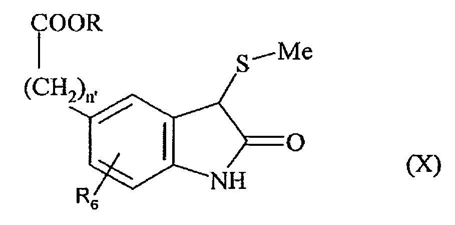 dans laquelle n', R et R₆ sont tels que définis précédemment,
que l'on soumet à l'action de Zinc en poudre pour conduire au composé de formule (XI) : 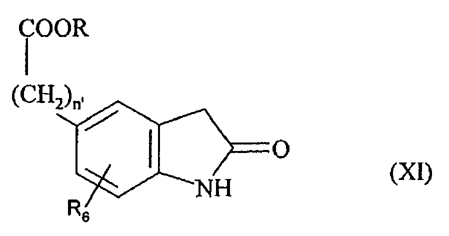 dans laquelle n', R et R₆ sont tels que définis précédemment,
que l'on place dans un milieu réducteur pour conduire au composé de formule (XII) : 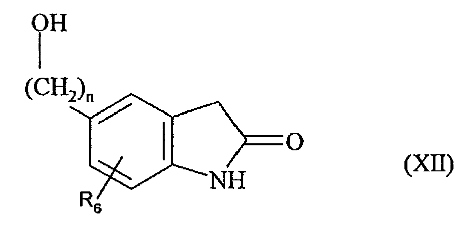 dans laquelle n et R₆ sont tels que définis précédemment,
sur lequel on condense en présence de pipéridine le composé de formule (VIII) : 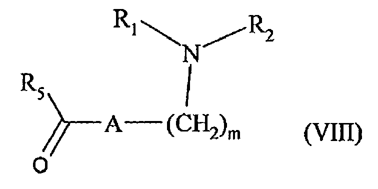 dans laquelle m, A, R₁, R₂, et R₅ sont tels que définis dans la formule (I), pour conduire après traitement acide au composé de formule (XIII) : 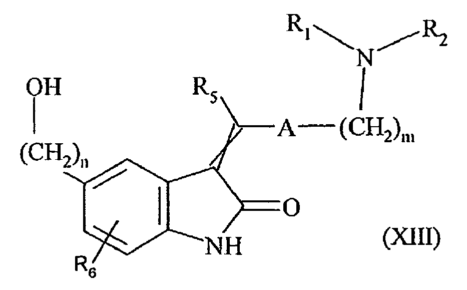 dans laquelle m, n, A, R₁, R₂ , R₅ et R₆ sont tels que définis précédemment,
sur lequel on condense directement en présence de triphénylphosphine et d'azodicarboxylate de diéthyle par exemple le composé de formule (III) : 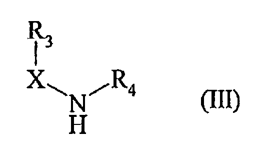 dans laquelle X, R₃ et R₄ sont tels que définis dans la formule (I),
pour conduire au composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

17. Compositions pharmaceutiques contenant au moins un composé de formule (I) selon l'une quelconque des revendications 1 à 14 ou un de leurs sels d'addition avec un acide ou une base pharmaceutiquement acceptable en combinaison avec un ou plusieurs excipients pharmaceutiquement acceptables.

18. Compositions pharmaceutiques selon la revendication 17 utiles pour la fabrication de médicaments en tant qu'agents inhibiteurs de la migration des cellules cancéreuses.

19. Compositions pharmaceutiques selon la revendication 17 utiles pour la fabrication de médicaments utiles dans le traitement des cancers métastatiques.

20. Compositions pharmaceutiques selon la revendication 17 utiles pour la fabrication de médicaments utiles dans le traitement des cancers du colon, du sein, du foie, des reins, du cerveau, de l'oesophage, les mélanomes, les myélomes, les cancers de l'ovaire, les cancers du poumon non à petites cellules, les cancers du poumon à petites cellules, les cancers de la prostate et du pancréas, les sarcomes.

21. Association d'un composé de formule (I) selon l'une quelconque des revendications 1 à 14 avec un agent anticancéreux choisi parmi les agents génotoxiques, les poisons mitotiques, les anti-métabolites, les inhibiteurs du protéasome, ou les inhibiteurs de kinases.

22. Utilisation d'une association selon la revendication 21 pour la fabrication de médicaments utiles dans le traitement des cancers.

23. Utilisation d'un composé de formule (I) selon l'une quelconque des revendications 1 à 14 pour l'utilisation en association avec une radiothérapie dans le traitement des cancers.

## Claims

1. Compounds of formula (I): 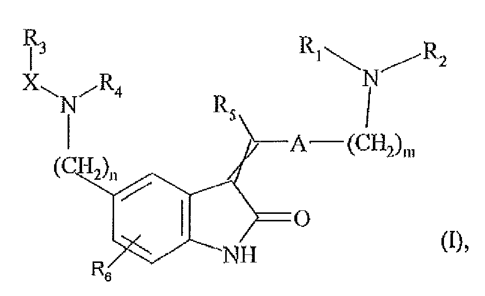 wherein:
◆ m represents 1 or 2,
◆ n represents 1 or 2,
◆ A represents a pyrrolyl group which is unsubstituted or substituted by 1 to 3 linear or branched (C₁-C₆)alkyl groups,
◆ X represents a C(O), S(O) or SO₂ group,
◆ R₁ and R₂, which are the same or different, each represent a linear or branched (C₁-C₆)alkyl group,
or R₁ and R₂, together with the nitrogen atom carrying them, form a heterocyclic group,
◆ R₃ and R₄, together with the atoms carrying them, form a heterocyclic group,
◆ R₅ represents a hydrogen atom or a linear or branched (C₁-C₆)alkyl group,
◆ R₆ represents a hydrogen atom or a halogen atom,
it being understood that:
- a "heterocyclic group" means a mono- or bi-cyclic group which may contain from 5 to 8 apices, which may contain from one to three hetero atoms selected from nitrogen, oxygen and sulphur, and which may contain one or more unsaturated bonds, it being possible for the heterocyclic group so defined to be unsubstituted or substituted by one or more groups selected from linear or branched (C₁-C₆)alkyl, linear or branched (C₂-C₆)alkenyl, oxo, hydroxy, linear or branched (C₁-C₆)alkoxy, aryl, arylalkyl and arylalkenyl,
- "aryl" means a phenol group which is unsubstituted or substituted by one or more groups selected from halogen atoms and linear or branched (C₁-C₆)alkyl groups,
- the notation 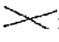 means that the double bond is of Z or E configuration,
their optical and geometric isomers, and also addition salts thereof with a pharmaceutically acceptable acid or base,

2. Compounds of formula (I) according to claim 1, wherein n represents 1, their optical and geometric isomers, and also addition salts thereof with a pharmaceutically acceptable acid or base.

3. Compounds of formula (I) according to claim 1, wherein in represents 1, their optical and geometric isomers, and also addition salts thereof with a pharmaceutically acceptable acid or base.

4. Compounds of formula (I) according to claim 1, wherein R₁ and R₂ represent an alkyl group, their optical and geometric isomers, and also addition salts thereof with a pharmaceutically acceptable acid or base.

5. Compounds of formula (I) according to claim 1, wherein R₁ and R₂, together with the nitrogen atom carrying them, form a 5- or 6-membered monocyclic group, their optical and geometric isomers, and also addition salts thereof with a pharmaceutically acceptable acid or base,

6. Compounds of formula (I) according to claim 1, wherein R₁ and R₂, together with the nitrogen atom carrying them, form a morpholinyl group, their optical and geometric isomers, and also addition salts thereof with a pharmaceutically acceptable acid or base.

7. Compounds of formula (I) according to claim 1, wherein R₃ and R₄, together with the X group and the nitrogen atom carrying them, form a thiazolidinedionyl group, their optical and geometric isomers, and also addition salts thereof with a pharmaceutically acceptable acid or base.

8. Compounds of formula (I) according to claim 1, wherein R₅ represents a hydrogen atom, their optical and geometric isomers, and also addition salts thereof with a pharmaceutically acceptable acid or base.

9. Compounds of formula (I) according to claim 1, wherein R₆ represents a hydrogen atom, their optical and geometric isomers, and also addition salts thereof with a pharmaceutically acceptable acid or base.

10. Compounds of formula (I) according to claim 1, wherein X represents a C=O group, their optical and geometric isomers, and also addition salts thereof with a pharmaceutically acceptable acid or base.

11. Compounds of formula (I) according to claim 1, wherein A represents a 1*H*-pyrrol-2,4-yl group, their optical and geometric isomers, and also addition salts thereof with a pharmaceutically acceptable acid or base.

12. Compounds of formula (I) according to claim 1, wherein R₁ and R₂, together with the nitrogen atom carrying them, form a morpholinyl group, m and n have the value 1, R₅ and R₆ represent a hydrogen atom and A represents a 1*H*-pyrrol-2,4-yl group, their optical and geometric isomers, and also addition salts thereof with a pharmaceutically acceptable acid or base.

13. Compound of formula (I) according to claim 1, which is 3-[(3-{[4-(4-morpholinylmethyl)-1*H*-pyrrol-2-yl]methylene}-2-oxo-2,3-dihydro-1*H*-indol-5-yl)methyl]-1,3-thiazolidine-2,4-dione, its geometric isomers, and also addition salts thereof with a pharmaceutically acceptable acid or base.

14. Compound of formula (I) according to claim 1, which is 3-[((3Z)-3-{[4-(4-morpholinylmethyl)-1*H*-pyrrol-2-yl]methylene}-2-oxo-2,3-dihydro-1*H*-indol-5-yl)methyl]-1,3-thiazolidine-2,4-dione, and also its addition salts with a pharmaceutically acceptable acid or base.

15. Process for the preparation of compounds of formula (I) according to claim 1, **characterised in that** there is used as starting material the compound of formula. (II): 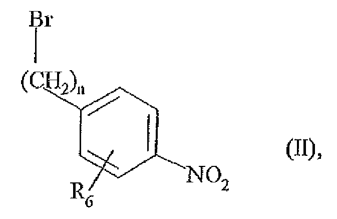 wherein n and R₆ are as defined for formula (I),
with which there is condensed, in the presence of a base, the compound of formula (III): 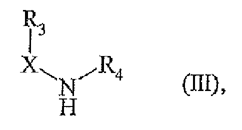 wherein X, R₃ and R₄ are as defined for formula (I),
to yield the compound of formula (IV): 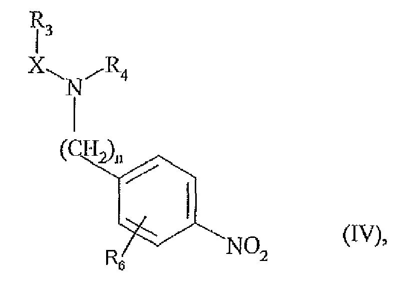 wherein n, X, R₃, R₄ and R₆ are as defined hereinbefore,
which is subjected to chemical or catalytic hydrogenation to yield the compound of formula (V): 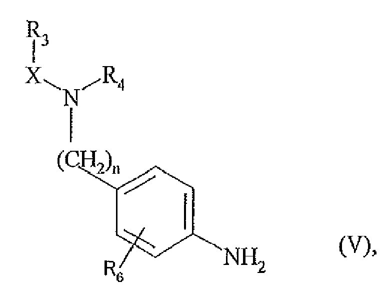 wherein n, X, R₃, R₄ and R₆ are as defined hereinbefore,
which is subjected to the action of tBuOCl in the presence of ethyl (methylsulphanyl)acetate, followed by the successive action of triethylamine and of hydrochloric acid to yield the compound of formula (VI): 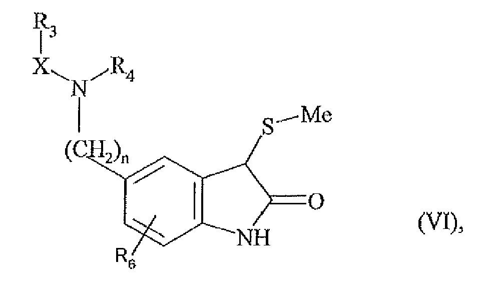 wherein n, X, R₃, R₄ and R₆ are as defined hereinbefore,
which is subjected to the action of powdered zinc to yield the compound of formula (VII): 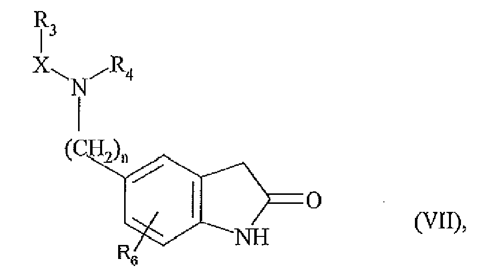 wherein n, X, R₃, R₄ and R₆ are as defined hereinbefore,
with which there is condensed, in the presence of piperidine, the compound of formula (VIII): 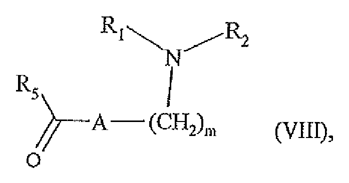 wherein m, A, R₁, R₂ and R₅ are as defined for formula (I),
to yield, after acid treatment, the compound of formula (I), which may be purified according to a customary separation technique, converted, if desired, intro its addition salts with a pharmaceutically acceptable acid or base and separated, where appropriate, into its isomers according to a customary separation technique.

16. Process for the preparation of compounds of formula (I) according to claim 1, **characterised in that** there is used as starting material the compound of formula (IX): 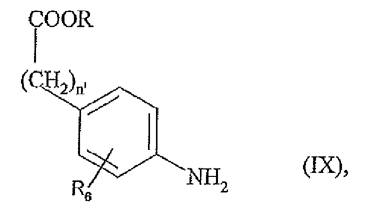 wherein n' represents 0 or 1, and R represents a linear or branched (C₁-C₆)alkyl group, and R₆ is as defined for formula (I),
which is subjected to the action of tBuOCl in the presence of ethyl (methylsulphanyl)acetate, followed by the successive action of triethylamine and of hydrochloric acid, to yield the compound of formula (X): 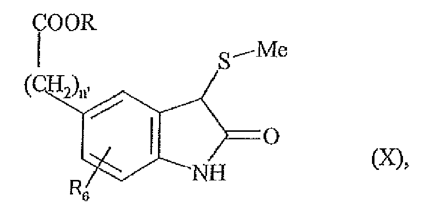 wherein n', R and R₆ are as defined hereinbefore,
which is subjected to the action of powdered zinc to yield the compound of formula (XI): 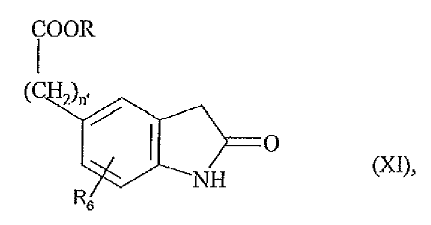 wherein n', R and R₆ are as defined hereinbefore,
which is placed in a reducing medium to yield the compound of formula (XII): 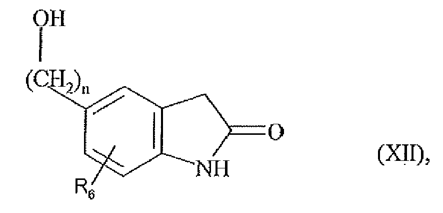 wherein n and R₆ are as defined hereinbefore,
with which there is condensed, in the presence of pipéridine, the compound of formula (VIII): 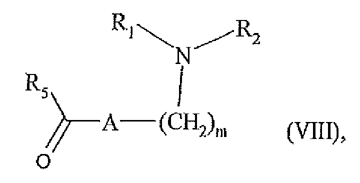 wherein m, A, R₁, R₂ and R₅ are as defined for formula (I),
to yield, after acid treatment, the compound of formula (XIII): 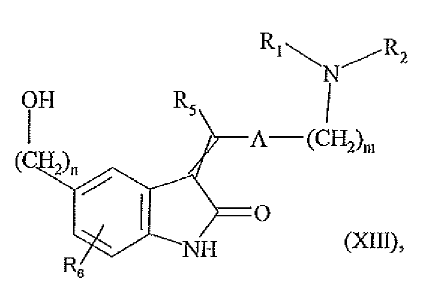 wherein m, n, A, R₁, R₂, R₅ and R₆ are as defined hereinbefore,
with which there is condensed directly in the presence of triphenylphosphine and diethyl azodicarboxylate for example the compound of formula (III): 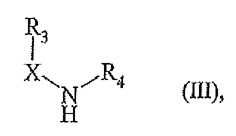 wherein X, R₃ and R₄ are as defined for formula (I),
to yield the compound of formula (I), which may be purified according to a customary separation technique, converted, if desired, into its addition salts with a pharmaceutically acceptable acid or base and separated, where appropriate, into its isomers according to a customary separation technique.

17. Pharmaceutical compositions comprising at least one compound of formula (I) according to any one of claims 1 to 14 or an addition salt thereof with a pharmaceutically acceptable acid or base in Kombination with one or more pharmaceutically acceptable excipients.

18. Pharmaceutical compositions according to claim 17 for use in the manufacture of medicaments as agents inhibiting the migration of cancerous cells,

19. Pharmaceutical compositions according to claim 17 for use in the manufacture of medicaments for use in the treatment of metastatic cancers.

20. Pharmaceutical compositions according to claim 17 for use in the manufacture of medicaments for use in the treatment of cancers of the colon, breast, liver, kidneys, brain, oesophagus, melanomas, myelomas, cancers of the ovary, non-small cell lung cancers, small cell lung cancers, cancers of the prostate and pancreas, and sarcomas.

21. Association of a compound of formula (I) according to any one of claims 1 to 14 with an anticancer agent selected from genotoxic agents, mitotic poisons, antimetabolites, proteasome inhibitors and kinase inhibitors.

22. Use of an association according to claim 21 in the manufacture of medicaments for use in the treatment of cancers.

23. Compound of formula (I) according to any one of claims 1 to 14 for use in association with radiotherapy in the treatment of cancers.

## Patentansprüche

1. Verbindungen der Formel (I): 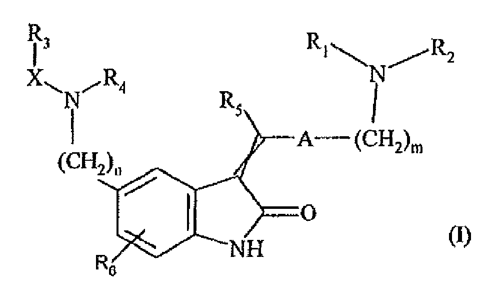 in der:
◆ m 1 oder 2 bedeutet,
◆ n 1 oder 2 bedeutet,
◆ A eine nichtsubstituierte oder durch 1 bis 3 geradkettige oder verzweigte (C₁-C₆)-Alkylgruppen substituierte Pyrrolylgruppe bedeutet,
◆ X die Gruppe C(O), S(O) oder SO₂ bedeutet,
◆ R₁ und R₂, die identisch oder verschieden sind, jeweils eine geradkettige oder verzweigte (C₁-C₆)-Alkylgruppe bedeuten,
oder R₁ und R₂ gemeinsam mit dem sie tragenden Stickstoffatom eine heterocyclische Gruppe bilden,
◆ R₃ und R₄ gemeinsam mit den sie tragenden Atomen eine heterocyclische Gruppe bilden,
◆ R₅ ein Wasserstoffatom oder eine geradkettige oder verzweigte (C₁-C₆)-Alkylgruppe bedeutet,
◆ R₆ ein Wasserstoffatom oder ein Halogenatom bedeutet,
mit der Maßgabe, dass:
- man unter "heterocyclischer Gruppe" eine mono- oder bicyclische Gruppe versteht, die 5 bis 8 Ringglieder aufweisen kann und die ein bis drei Heteroatome ausgewählt aus Stickstoff, Sauerstoff und Schwefel enthalten kann und die eine oder mehrere Unsättigungen aufweisen kann, wobei die in dieser Weise definierte heterocyclische Gruppe nichtsubstituiert oder durch eine oder mehrere Gruppen ausgewählt aus geradkettigen oder verzweigtem (C₁-C₆)-Alkyl, geradkettigem oder verzweigtem (C₂-C₆)-Alkenyl, Oxo, Hydroxy, geradkettigen oder verzweigtem (C₁-C₆)-Alkoxy, Aryl, Arylalkyl und Arylalkenyl substituiert sein kann,
- man unter "Aryl" die Phenylgruppe versteht, die nichtsubstituiert oder durch eine oder mehrere Gruppen ausgewählt aus Halogenatomen und geradkettigen oder verzweigten (C₁-C₆)-Alkylgruppen substituiert sein kann,
- das Symbol 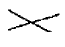 bedeutet, dass die Doppelbindung in der Konfiguration Z oder E vorliegt,
ihre optischen und geometrischen Isomeren und ihre Additionssalze mit einer pharmazeutisch annehmbaren Säure oder Base.

2. Verbindungen der Formel (I) nach Anspruch 1, worin n 1 bedeutet, ihre optischen und geometrischen Isomeren sowie ihre Additionssalze mit einer pharmazeutisch annehmbaren Säure oder Base.

3. Verbindungen der Formel (I) nach Anspruch 1, worin m 1 bedeutet, ihre optischen und geometrischen Isomeren sowie ihre Additionssalze mit einer pharmazeutisch annehmbaren Säure oder Base.

4. Verbindungen der Formel (I) nach Anspruch 1, worin R₁ und R₂ eine Alkylgruppe bedeuten, ihre optischen und geometrischen Isomeren sowie ihre Additionssalze mit einer pharmazeutisch annehmbaren Säure oder Base.

5. Verbindungen der Formel (I) nach Anspruch 1, worin R₁ und R₂ gemeinsam mit dem sie tragenden Stickstoffatom eine monocyclische Gruppe mit 5 oder 6 Kettengliedern bilden, ihre optischen und geometrischen Isomeren sowie ihre Additionssalze mit einer pharmazeutisch annehmbaren Säure oder Base.

6. Verbindungen der Formel (I) nach Anspruch 1, worin R₁ und R₂ gemeinsam mit dem sie tragenden Stickstoffatom eine Morpholinylgruppe bilden, ihre optischen und geometrischen Isomeren sowie ihre Additionssalze mit einer pharmazeutisch annehmbarren Säure oder Base.

7. Verbindungen der Formel (I) nach Anspruch 1, worin R₃ und R₄ gemeinsam mit der Gruppe X und dem sie tragenden Stiekstoffatom eine Thiazolidindionylgruppe bilden, ihre optischen und geometrischen Isomeren sowie ihre Additionssalze mit einer pharmazeutisch annehmbaren Säure oder Base.

8. Verbindungen der Formel (I) nach Anspruch 1, worin R₅ ein Wasserstoffatom bedeutet, ihre optischen und geometrischen Isomeren sowie ihre Additionssalze mit einer pharmazeutisch annehmbaren Säure oder Base.

9. Verbindungen der Formel (I) nach Anspruch 1, worin R₆ ein Wasserstoffatom bedeutet, ihre optischen und geometrischen Isomeren sowie ihre Additionssalze mit einer pharmazeutisch annehmbaren Säure oder Base.

10. Verbindungen der Formel (I) nach Anspruch 1, worin X eine Gruppe C=O bedeutet, ihre optischen und geometrischen Isomeren sowie ihre Additionssalze mit einer pharmazeutisch annehmbaren Säure oder Base.

11. Verbindungen der Formel (I) nach Anspruch 1, worin A die 1*H*-Pyrrol-2,4-yl-gruppe bedeutet, ihre optischen und geometrischen Isomeren sowie ihre Additionssalze mit einer pharmazeutisch annehmbaren Säure oder Base.

12. Verbindungen der Formel (I) nach Anspruch 1, worin R₁ und R₇ gemeinsam mit dem sie tragenden Stickstoffatom die Morpholinylgruppe bilden, m und n den Wert 1 besitzen, R₅ und R₆ ein Wasserstoffatom bedeuten und A die 1*H*-Pyrrol-2,4-yl-gruppe bedeutet, ihre optischen und geometrischen Isomeren sowie ihre Additionssalze mit einer pharmazeutisch annehmbaren Säure oder Base.

13. Verbindung der Formel (I) nach Anspruch 1, nämlich 3-[(3-{[4-(4-Morplxolinylmethyl)-1*H*-pyrrol-2-yl]-methylen}-2-oxo-2,3-dihydro-1*H*-indol-5-yl)-methyl]-1,3-thiazolidin-2,4-dion, seine geometrischen Isomeren sowie seine Additionssalze mit einer pharmazeutisch annehmbaren Säure oder Base.

14. Verbindung der Formel (I) nach Anspruch 1, nämlich 3-[((3Z)-3-{[4-(4-Morpholinylmethyl)-1*H*-pyrrol-2-yl]-methylen}-2-oxo-2,3-dihydro-1*H*-indol-5-yl}-methyl]-1,3-thiazolidin-2,4-dion, sowie seine Additionssalze mit einer pharmazeutisch annehmbaren Säure oder Base.

15. Verfahren zur Herstellung der Verbindungen der Formel (I) nach Anspruch 1, **dadurch gekennzeichnet, dass** man als Ausgangsprodukt die Verbindung der Formen (II) verwendet: 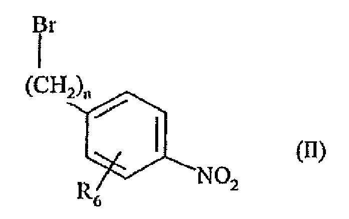 in der n und R₆ die bezüglich der Formel (I) angegebenen Bedeutungen besitzen,
welche man in Gegenwart einer Base mit der Verbindung der Formel (III) kondensiert: 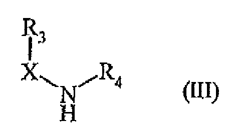 in der X, R₃ und R₄ die bezüglich der Formel (I) angegebenen Bedeutungen besitzen,
zur Bildung der Verbindung der Formel (IV): 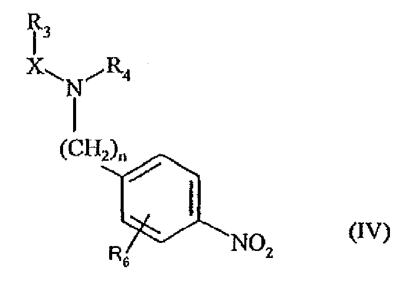 in der n, X, R₃, R₄ und R₆ die oben angegebenen Bedeutungen besitzen, welche man einer chemischen oder katalytischen Hydrierung unterzieht zur Bildung der Verbindung der Formel (V): 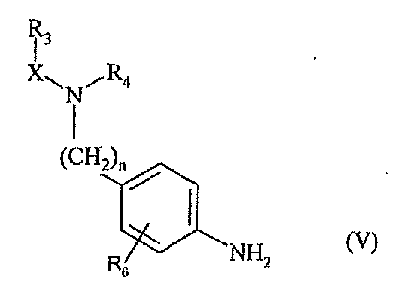 in der n, X, R₃, R₄ und R₆ die oben angegebenen Bedeutungen besitzen,
welche man der Einwirkung von tBuOCl in Gegenwart von (Methylsulfanyl)-essigsäureethylester unterzieht, gefolgt von der nacheinander erfolgenden Einwirkung von Triethylamin und Chlorwasserstoffsäure zur Bildung der Verbindung der Formel (VI): 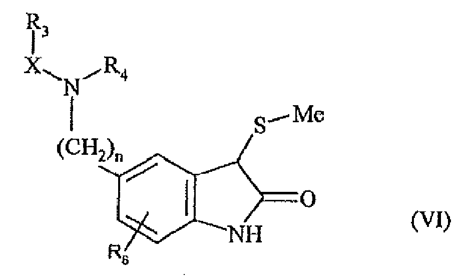 in der n, X, R₃, R₄ und R₆ die oben angegebenen Bedeutungen besitzen,
welche man der Einwirkung von pulverförmigem Zink unterzieht zur Bildung der Verbindung der Formel (VII): 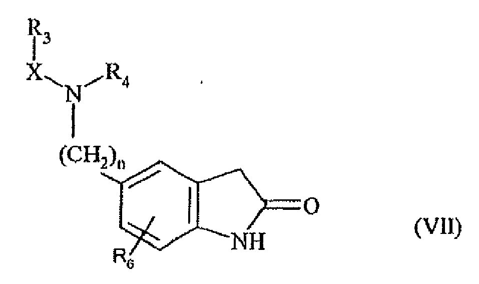 in der n, X, R₃, R₄ und R₆ die oben angegebenen Bedeutungen besitzen,
welche man in Gegenwart von Piperidin mit der Verbindung der Formel (VIII) kondensiert: 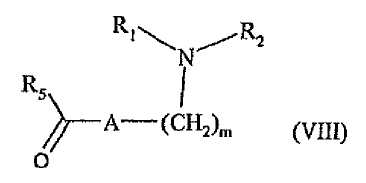 in der m, A, R₁, R₂ und R₅ die bezüglich der Formel (I) angegebenen Bedeutungen
besitzen,
sodass man nach der Behandlung mit Säure die Verbindung der Formen (I) verhält, welche mit Hilfe einer klassischen Trennungsmethode gereinigt werden kann, welche gewünschtenfalls in ihre Additionssalze mit einer pharmazeutisch annehmbaren Säure oder Base überführt werden kann und welche man gegebenenfalls mit Hilfe einer klassischen Trennmethode in ihre Isomeren aufspaltet.

16. Verfahren zur Herstellung der Verbindungen der Formel (I) nach Anspruch 1, **dadurch gekennzeichnet, dass** man als Ausgangsprodukt die Verbindung der Formel (IX) verwendet: 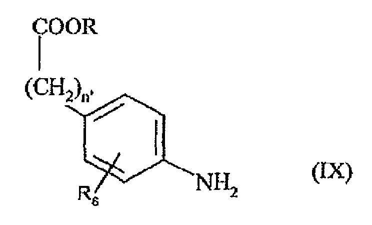 in der n' 0 oder 1 bedeutet und R eine geradkettige oder verzweigte (C₁-C₆)-Alkylgruppe darstellt und R₆ die bezüglich der Formel (I) angegebenen Bedeutungen besitzt,
welche man der Einwirkung von tBuOCl in Gegenwart von (Methylsulfanyl)-essigsäureethylester unterwirft gefolgt von der nacheinander erfolgenden Einwirkung von Triethylamin und Chlorwasserstoffsäure zur Bildung der Verbindung der Formel (X): 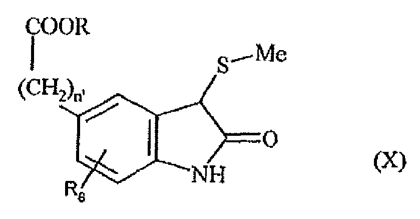 in der n', R und R₆ die oben angegebenen Bedeutungen besitzen,
welche man der Einwirkung von pulverförmigem Zink unterwirft zur Bildung der Verbindung der Formel (XI): 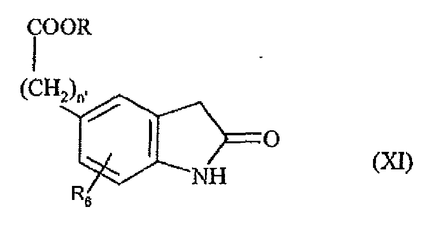 in der n', R und R₆ die oben angegebenen Bedeutungen besitzen,
welche man in ein Reduktionsmedium einbringt zur Bildung der Verbindung der Formel (XII): 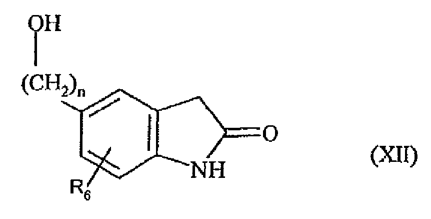 in der n und R₆ die oben angegebenen Bedeutungen besitzen,
welche man in Gegenwart von Piperidin mit der Verbindung der Formel (VIII) kondensiert: 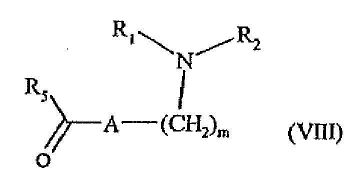 in der in, A, R₁, R₂ und R₅ die bezüglich der Formel (I) angegebenen Bedeutungen besitzen,
sodass man nach der Behandlung mit Säure die Verbindung der Formel (XIII) erhält: 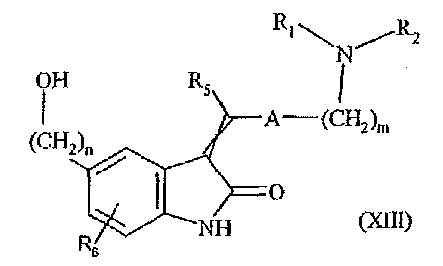 in der m, n, A, R₁, R₂, R₅ und R₆ die oben angegebenen Bedeutungen besitzen,
welche man direkt beispielsweise in Gegenwart von Triphenylphosphin und Azodicarbonsäurediethylester mit der Verbindung der Formel (III) kondensiert: 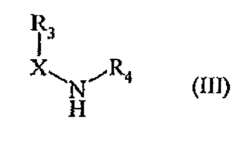 in der X, R₃ und R₄ die bezüglich der Formel (I) angegebenen Bedeutungen besitzen, zur Bildung der Verbindung der Formel (I), welche mit Hilfe einer klassischen Trennungsmethode gereinigt werden kann, welche man gewünschtenfalls in ihre Additionssalze mit einer pharmazeutisch annehmbaren Säure oder Base überfährt und die man gegebenenfalls mit Hilfe einer klassischen Trennungsmethode in ihre Isomeren aufspaltet.

17. Pharmazeutische Zubereitungen enthaltend mindestens eine Verbindung der Formel (I) nach einem der Ansprüche 1 bis 14 oder eines ihrer Additionssalze mit einer pharmazeutisch annehmbaren Säure oder Base in Kombination mit einem oder mehreren pharmazeutisch annehmbaren Hilfsstoffen.

18. Pharmazeutische Zubereitung nach Anspruch 17, nützlich für die Herstellung von Arzneimitteln, die als Inhibitoren, der Migration von Krebszellen wirken.

19. Pharmazeutische Zubereitung nach Anspruch 17, nützlich für die Herstellung von Arzneimitteln, die zur Behandlung von metastatischen Krebsen nützlich sind.

20. Pharmazeutische Zubereitung nach Anspruch 17 für die Herstellung von Arzneimitteln, die nützlich sind bei der Behandlung von Krebsen des Darms, der Brust, der Leber, der Nieren, des Gehirns, der Speiseröhre, von Melanomen, Myelomen, Eierstockkrebsen, nichtkleinzelligen Lungenkrebsen, kleinzelligen Lungenkrebsen, Prostata- und Pankreaskrebsen, Sarkomen.

21. Kombination einer Verbindung der Formel (I) nach einem der Ansprüche 1 bis 14 mit einem Antikrebsmittel ausgewählt aus genotoxischen Mitteln, mitotischen Giften, Antimetaboliten, Proteasominhibitoren oder Kinaseinhibitoren.

22. Verwendung einer Kombination nach Anspruch 21 zur Herstellung von Arzneimitteln, die nützlich sind bei der Behandlung von Krebs.

23. Verbindung der Formel (I) nach einem der Ansprüche 1 bis 14 zur Verwendung in Kombination mit einer Strahlentherapie bei der Behandlung von Krebs.
